(19) Europäisches Patentamt
European Patent Office
Office européen des brevets

(11) **EP 2 191 011 B1**

(12) **EUROPEAN PATENT SPECIFICATION**

(45) Date of publication and mention of the grant of the patent:
**29.03.2017 Bulletin 2017/13**

(21) Application number: **08829345.1**

(22) Date of filing: **28.08.2008**

(51) Int Cl.:
***C12Q 1/68*** *(2006.01)* ***C12N 15/10*** *(2006.01)*

(86) International application number:
**PCT/US2008/010206**

(87) International publication number:
**WO 2009/032167 (12.03.2009 Gazette 2009/11)**

# (54) METHOD FOR SEQUENCING A POLYNUCLEOTIDE TEMPLATE

VERFAHREN ZUR SEQUENZIERUNG EINER POLYNUKLEOTIDMATRIZE

PROCÉDÉ DE SÉQUENÇAGE D’UNE MATRICE DE POLYNUCLÉOTIDES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.08.2007 US 968770 P**
**26.11.2007 US 990104 P**
**18.04.2008 US 46203 P**

(43) Date of publication of application:
**02.06.2010 Bulletin 2010/22**

(73) Proprietor: **Illumina Cambridge Limited**
**Little Chesterford**
**Saffron Walden**
**Essex CB10 1XL (GB)**

(72) Inventors:
- **BIGNELL, Helen**
  **Walden, Essex CB10 1XL (GB)**
- **GORMLEY, Niall, Anthony**
  **Walden, Essex CB10 1XL (GB)**
- **HIMS, Matthew**
  **Walden, Essex CB10 1XL (GB)**
- **SMITH, Geoffery**
  **Walden, Essex CB10 1XL (GB)**
- **WEST, John, Stephen**
  **San Diego, CA 92121 (US)**

(74) Representative: **Leissler-Gerstl, Gabriele et al**
**Hoefer & Partner**
**Patentanwälte**
**Pilgersheimer Strasse 20**
**81543 München (DE)**

(56) References cited:

| | |
|---|---|
| **EP-A- 0 438 292** | **EP-A- 1 647 602** |
| **WO-A-03/074734** | **WO-A-2005/093094** |
| **WO-A-2007/010251** | **WO-A-2007/076726** |
| **WO-A-2007/091077** | **WO-A1-2007/061284** |
| **US-A- 5 118 604** | **US-A- 5 935 788** |
| **US-A1- 2006 024 681** | **US-A1- 2006 292 611** |

- **SAN LUIS BORIS B ET AL: "Analysis of a gene (vch) encoding hemolysin isolated and sequenced from Vibrio campbellii" JOURNAL OF GENERAL AND APPLIED MICROBIOLOGY, vol. 52, no. 6, December 2006 (2006-12), pages 303-313, XP002512992 ISSN: 0022-1260**
- **MARDIS ELAINE R: "Next-generation DNA sequencing methods" ANNUAL REVIEW OF GENOMICS AND HUMAN GENETICS, vol. 9, 2008, pages 387-402, XP002512993 ISSN: 1527-8204**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 191 011 B1

## Description

### Cross reference to related applications

This application claims priority under 35 USC §119(e) from U.S Provisional Application Serial Nos. 60/968,770, filed August 29, 2007; 60/990,104, filed November 26, 2007; and 61/046,203, filed April 18, 2008.

### Field of the invention

The invention relates to methods for pairwise sequencing of a double-stranded polynucleotide template, which methods result in the sequential determination of nucleotide sequences in two distinct and separate regions of the polynucleotide template.

### Background to the invention

Advances in the study of biological molecules have been led, in part, by improvement in technologies used to characterise the molecules or their biological reactions. In particular, the study of the nucleic acids DNA and RNA has benefited from developing technologies used for sequence analysis.

Methods for sequencing a polynucleotide template can involve performing multiple extension reactions using a DNA polymerase or DNA ligase, respectively, to successively incorporate labelled nucleotides or polynucleotides complementary to a template strand. In such "sequencing by synthesis" reactions a new nucleotide strand base-paired to the template strand is built up by successive incorporation of nucleotides complementary to the template strand. The substrate nucleoside triphosphates or oligonucleotide cassettes used in the sequencing reaction are blocked to prevent over-incorporation. The substrate nucleoside triphosphates or oligonucleotide cassettes can also be labelled, permitting determination of the identity of the incorporated nucleotide(s) as successive nucleotides are added.

In order to carry out accurate sequencing using nucleoside triphosphates, a reversible chain-terminating structural modification or "blocking group" may be added to the substrate nucleotides to ensure that nucleotides are incorporated one at a time in a controlled manner. As each single nucleotide is incorporated, the blocking group prevents any further nucleotide incorporation into the polynucleotide chain. Once the identity of the last-incorporated labelled nucleotide has been determined the label moiety and blocking group are removed, allowing the next blocked, labelled nucleotide to be incorporated in a subsequent round of sequencing.

In certain circumstances the amount of sequence data that can be reliably obtained with the use of sequencing-by-synthesis techniques, particularly when using blocked, labelled nucleotides, may be limited. In some circumstances the sequencing "run" may be limited to a number of bases that permits sequence realignment with the human genome, for example around 25-30 cycles of incorporation. Whilst sequencing runs of this length are extremely useful, particularly in applications such as, for example, SNP analysis and genotyping, it would be advantageous in many circumstances to be able to reliably obtain further sequence data for the same template molecule.

The technique of "paired-end" or "pairwise" sequencing is generally known in the art of molecular biology, particularly in the context of whole-genomic shotgun sequencing. Paired-end sequencing allows the determination of two "reads" of sequence from two places on a single polynucleotide duplex. The advantage of the paired-end approach is that there is significantly more information to be gained from sequencing two stretches each of "n" bases from a single template than from sequencing "n" bases from each of two independent templates in a random fashion. With the use of appropriate software tools for the assembly of sequence information it is possible to make use of the knowledge that the "paired-end" sequences are not completely random, but are known to occur on a single duplex, and are therefore linked or paired in the genome. This information has been shown to greatly aid the assembly of whole genome sequences into a consensus sequence.

Paired-end sequencing has typically been performed by making use of specialized circular shotgun cloning vectors known in the art. After cutting the vector at a specific single site, the template DNA to be sequenced (typically genomic DNA) is inserted into the vector and the ends resealed to form a new construct. The vector sequences flanking the insert DNA include binding sites for sequencing primers which permit sequencing of the insert DNA on opposite strands.

US2006/292611A1 discloses a method for obtaining a DNA construct comprising two end regions of a target nucleic acid comprising the steps of fragmenting a large nucleic acid molecule to produce a target nucleic acid, ligating a capture element to said target nucleic acid to form a first circular nucleic acid molecule, digesting said first circular nucleic acid with a restriction endonuclease which cuts said target nucleic acid but which does not cut said capture element to produce a linear nucleic acid which comprises two ends of said target nucleic acid separated by said capture element, ligating said linear nucleic acid with a separator element to form a second circular nucleic acid, converting said second circular nucleic acid to a circular single strand nucleic acid, annealing a first oligonucleotide to said circular single

stranded nucleic acid and amplifying said circular single stranded nucleic acid by rolling circle amplification to produce a single stranded rolling circle amplification product, annealing a second oligonucleotide to said single stranded rolling circle amplification product to form multiple double stranded regions in said single stranded rolling amplification product; and digesting said single stranded rolling circle amplification product into small fragments with a restriction endonuclease which cleaves said multiple double stranded regions to produce said DNA construct comprising two end regions of a target nucleic acid. The disclosed method can be performed simultaneously on a plurality of target DNA fragments to produce a library of DNA constructs which contain the ends from a large fragment of DNA.

A disadvantage of this approach is that it requires time-consuming cloning of the DNA sequencing templates into an appropriate sequencing vector. Furthermore, because of the need to clone the DNA template into a vector in order to position binding sites for sequencing primers at both ends of the template fragment it can be difficult to make use of array-based sequencing techniques. With array-based techniques a sequence is generally read from one end of a nucleotide template, this often being the end proximal to the point of attachment to the array. However, a variety of methods for double-ended sequencing of a polynucleotide template are known.

Also known are methods of nucleic acid amplification which generate amplification products immobilised on a solid support in order to form arrays comprised of clusters or "colonies" formed from a plurality of identical immobilised polynucleotide strands and a plurality of identical immobilised complementary strands. The nucleic acid molecules present in DNA colonies on the clustered arrays prepared according to these methods can provide templates for sequencing reactions but only a single sequencing read is typically obtained from one type of immobilised strand in each colony.

An exemplary method that is useful for paired end sequencing on clusters uses three grafted primers. This method is applicable to templates that can be amplified using bridge amplification, and the length of the templates used may be up to, for example, 1000 base pairs or so, however for many DNA sequencing applications, it may be necessary to determine a sequence read from either end of a target fragment of greater length. The sample preparation methods described in the present invention allow the analysis of a pair of reads for the ends of a fragment of any length.

## Summary of the invention

The present inventors have developed a method as defined in the claims for preparing a nucleic acid sample of any length such that it is suitable for paired-end, or pairwise sequencing. The term pairwise sequencing refers to a pair of reads obtained by sequencing two distinct regions, either on the same strand or the complementary strand of a particular target polynucleotide duplex molecule. Using the method of the invention it is possible to prepare a nucleic acid sample to obtain two linked or paired reads of sequence information from each double-stranded template.

In accordance with the methods described herein the ends of linear fragments of any desired length are directly connected together to form circular constructs. Fragmentation of the circular constructs gives rise to a collection of linear fragments, at least a portion of which contain the two ends of the original fragment. Sequencing the resultant fragment library gives rise to a collection of nucleic acid sequences, some of which comprise paired sequence fragments from both ends of the original linear fragments.

According to the invention there is provided a method as defined in the claims for producing a library of 3’ and 5’ modified nucleic acids suitable for paired end sequencing comprising;

a. Fragmenting a primary double stranded nucleic acid target sequence to produce a first population of linear nucleic acid target fragments;

b. end repairing the linear nucleic acid target fragments with nucleotide triphosphates and a nucleic acid polymerase to obtain blunt ends on each fragment;

c. self-ligating the blunt ends of the fragments to form circular constructs;

d. treating the circular constructs to reduce the ratio of linear fragments to circular fragments;

e. fragmenting the circular constructs to produce a second population of linear fragments, a percentage of which will span the two ends of the first population of linear target fragments;

f. ligating adapter sequences to the 3’ and 5’ ends of the second population of linear fragments to produce a library of 3’ and 5’ modified nucleic acids suitable for paired end sequencing.

According to the invention, there is provided a nucleic acid construct for paired end sequencing comprising a circular nucleic acid molecule containing no adapter regions of known sequence.

The invention covers use of the library of fragments for sequencing, or in the formation of arrays.

## Brief description of the figures

**Figure 1** shows a summary of the steps involved in one implementation of the method.

**Figures 2a and 2b** show a schematic of the steps involved in one implementation of the protocol.

**Figure 2c** shows a possible alternative implementation of the protocol.

**Figure 3** shows a schematic of a paired-end read using the first method of the invention enabled with a nicking enzyme.

**Figure 4** shows a schematic of a paired read enabled with a uracil primer attached to the surface.

**Figure 5** shows a schematic of a paired read using an alternative method of the invention.

**Figure 6** shows a schematic of a paired read wherein three grafting primers are used.

**Figure 7** shows sequencing data obtained from a selection of clones prepared from the library prepared using biotin selection of the end repaired fragments.

**Figure 8** shows cluster/SBS sequencing data from clusters prepared using a library prepared using biotin selection of the end repaired fragments.

**Figure 9** shows an exemplary method using random priming with biotinylated primers to generate fragments for circularisation.

**Figure 10** shows data where the end repair reaction was carried out in two stages in order to reduce the level of biotin nucleotides incorporated at internal sites. If the DNA fragments are not first blunted using the pre-end repair step, then biotin nucleotides can be incorporated at sites some distance away from the terminal ends of the DNA fragments, leading to subsequent selection of biotinylated DNA that does not represent the junction of the DNA fragment's two ligated ends. This leads to a higher proportion of final paired end sequences being inward facing fragments rather than the desired outward facing fragments.

**Figure 11** shows the effect of varying Biotin concentration used in the exchange reaction on the final library diversity. The percentage of duplicated sequences found in 100,000 paired sequences (100K duplicates) is used as a measure of the library's diversity. The lower the duplicates the more diverse the library population. The lowest levels of duplicates and thus the most diverse libraries were made using Biotin nucleotide concentrations in the range of 20 - 2.5%, with the most diverse library generated at 4% biotin nucleotide concentration. Below 2.5% and above 20% biotin the levels of duplicates in the library preparations begin to rise, indicating this optimisation is a balance between incorporating enough biotin to pull out the label junctions, but not too much biotin, as this inhibits the self-ligation event.

**Figure 12** shows a gel based assay which shows that high levels of biotin at the ends of fragments prevents self-ligation.

**Figure 13** shows a comparison of T3 and T4 ligase in the self-ligation reaction, and indicates that T3 lowers the level of duplicates and intramolecular chimeras compared to T4 under the same ligation conditions using the identical size selected DNA in both ligations. Sequencing data showed that the diversity is 10 fold less and the chimeras 5 fold higher using T4, and that therefore the use of T3 ligase is optimal for this protocol.

**Figure 14** shows a comparison of the two protocols outlined in figure 2, where the protocol 2c with the streptavidin pull out earlier in the process gives an improvement in the sequencing quality.

## Detailed description of the invention

The invention provides methods as defined in the claims for preparing a nucleic acid sample for paired end sequencing. In one implementation of the method, each nucleic acid fragment in the sample is prepared such that sequencing two regions of the target double-stranded polynucleotide template is possible, referred to herein as the first and second regions. In another implementation of the method, only a proportion of the nucleic acid sample is a paired end construct that gives reads from opposite ends of a linear fragment. The first and second regions are either on the same strand, or on complementary strands, of the double-stranded polynucleotide template, which are referred to herein

respectively as first and second template strands. The fragments can be described as a library of 3' and 5' modified nucleic acids suitable for paired end sequencing as the 5' and 3' ends of each fragment contain known sequences, whereas the internal sequences between the known ends are unknown. Typically, the known sequence on one or both ends of each fragment in a population of fragments is the same, although the 3' ends and 5' ends might be different from each other, whereas the internal unknown sequences in each of these fragments is different. It will be understood that a population of fragments can include several subpopulations, wherein the fragments in each subpopulation have the same known sequence ends but the known sequences at the ends of the fragments in the different subpopulations differ from each other.

The following passages describe different aspects of the invention in greater detail. Each aspect of the invention may be combined with any other aspect or aspects of the invention unless clearly indicated to the contrary. In particular, any feature indicated as being particular, preferred or advantageous may be combined with any other feature or features indicated as being particular, preferred or advantageous.

The terms 'target nucleic acid sequence', 'target nucleic acid molecule', 'target nucleic acid' and 'target nucleic acid fragment' may be used interchangeably to refer to nucleic acid molecules that it is desired to sequence. Such sequencing may be performed on an array of amplified fragments according to the invention. The target nucleic acid may be essentially any nucleic acid of known or unknown sequence. It may be, for example, a fragment of genomic DNA or cDNA. Sequencing may result in determination of the sequence of the whole, or a part of the target molecule. The targets can be derived from a primary nucleic acid sample that has been randomly fragmented. The targets can be processed into templates suitable for amplification by the placement of universal amplification sequences at the ends of each target fragment. The targets can also be obtained from a primary RNA sample by reverse transcription into cDNA.

As used herein, the term 'polynucleotide' refers to deoxyribonucleic acid (DNA), ribonucleic acid (RNA) or analogs of either DNA or RNA made, for example, from nucleotide analogs. The term is applicable to single stranded (such as sense or antisense) and double stranded polynucleotides. The term as used herein also encompasses cDNA, that is complementary or copy DNA produced from an RNA template, for example by the action of reverse transcriptase.

The primary polynucleotide molecules may originate in double-stranded DNA (dsDNA) form (e.g. genomic DNA fragments, PCR and amplification products and the like) or may have originated in single-stranded form, as DNA or RNA, and been converted to dsDNA form. By way of example, mRNA molecules may be copied into double-stranded cDNAs suitable for use in the method of the invention using standard techniques well known in the art. The precise sequence of the primary polynucleotide molecules useful in the invention may be known or unknown.

In a particular embodiment, the primary polynucleotide molecules are DNA molecules. A population of the primary polynucleotide molecules can represent the entire genetic complement of an organism or a portion thereof. For example, the population can include at least 50%, 75%, 90%, 95%, 98%, or 99% of the genetic complement of an organism. The population can include genomic DNA molecules which include both intron and exon sequences (coding sequence), as well as non-coding regulatory sequences such as promoter and enhancer sequences. In an embodiment wherein genomic DNA molecules are used, genome-wide analysis or analysis of the entire genome may be achieved. It is, however, envisaged that particular sub-sets of polynucleotide sequences or genomic DNA could also be used, such as, for example, particular chromosomes obtained by known chromosome isolation methods or coding sequences derived from a cDNA sample. The subsets of the genome may be selected, for example using hybridisation with pools of oligonucleotides, for example as described in co-pending applications WO07057652 and US 20070141604. The sequence of the primary polynucleotide molecules can be known but need not be known in order to function as a target polynucleotide in the methods set forth herein. The primary polynucleotide molecules can be human genomic DNA molecules. The DNA target molecules may be treated chemically or enzymatically, either prior to, or subsequent to any random fragmentation processes, and prior to or subsequent to the ligation of the adaptor sequences.

Random fragmentation refers to the fragmentation of a polynucleotide molecule in a non-ordered fashion, for example, by enzymatic, chemical or mechanical means. Such fragmentation methods are known in the art and utilise standard methods (Sambrook and Russell, Molecular Cloning, A Laboratory Manual, third edition,). In some embodiments, the random fragmentation methods do not include methods in which smaller fragments are amplified from a larger piece of nucleic acid sequence that remains in intact. However, in other embodiments smaller fragments can be obtained by amplifying smaller regions of a larger template nucleic acid sequence. Moreover, random fragmentation is typically designed to produce fragments irrespective of the sequence identity or position of nucleotides comprising and/or surrounding the break. In the application methods described herein, fragmentation may be in the range 50-100000 bases. The fragment range may be controlled, or the fragment may be size selected prior to circularisation. More particularly, random fragmentation is achieved by mechanical means such as nebulisation or sonication, which can be controlled to produce fragment length of different sizes. The method describes two fragmentation steps, one of the primary sample, and one of the circular constructs, and these fragment lengths may be different sizes. For example, the fragmentation of the primary sample may give rise to fragments of, on average about 1000-100000 bases, more particularly 1000-5000 bases, and the fragmentation of the circularised constructs may give rise to fragments of, on average about 50-1500 base pairs in length, still more particularly 50-700 base pairs in length, yet more particularly 50-400 base

pairs in length. Most particularly, the method is used to generate smaller fragments of from, on average about 50-150 base pairs in length. The primary target fragments may be purified on a gel to obtain fragments of a narrow size distribution, for example, on average about 3000-4000 base pairs. If desired, the fragments of both steps can be about the same size on average.

Fragmentation of polynucleotide molecules by mechanical means (nebulization, sonication and Hydroshear for example) results in fragments with a heterogeneous mix of blunt and 3'- and 5'-overhanging ends. It is therefore desirable to repair the fragment ends using methods or kits (such as the Lucigen DNA terminator End Repair Kit) known in the art to generate ends that are optimal for insertion, for example, into blunt sites of cloning vectors. In a particular embodiment, the fragment ends of the population of nucleic acids are blunt ended. One method of the invention involves repairing the fragment ends with nucleotide triphosphates and a nucleic acid polymerase. The nucleotide triphosphates may contain a labelling modification, for example biotin or similar protein binding ligand, that allows selection of the end repaired fragments. The polymerase may be Klenow DNA polymerase or similar nucleic acid polymerase, that may have exonuclease activity in order to remove any 3' overhanging ends. The reaction may be carried out with all four nucleotides, of which 0-4 may carry labelling modifications. The reaction may be carried out with a single labelled nucleoside triphosphate, and three unlabelled triphosphates, or may be carried out with two, three or four labelled nucleotides. The nucleotides may carry the labels on the nucleobases, for example the four biotinylated nucleoside triphosphates as sold by Perkin Elmer.

In particular embodiments of the invention, a population of fragments is provided in which the ends of the fragments in the population are biotinylated. The population can include varying levels of biotinylation such that all or a portion of the member fragments in the population are biotinylated at their ends. Preferably the level of biotinylation in a population of fragments is selected to allow substantially all of the member fragments to self ligate to form circles having biotin moieties for subsequent selection. However, the level of biotinylation in a population of fragments can be selected to allow a desired fraction of the member fragments to self ligate to form circles having biotin moieties for subsequent selection. The desired fraction can be for example at least 50%, 60%, 70%, 80%, 90%, 95% or 99% of the population. Thus, in accordance with the methods of the invention conditions can be selected to avoid too high a level of biotin at the ends of the fragments which can hinder self-ligation of the fragments under some conditions, thereby reducing the yield of circles formed. Alternatively or additionally, conditions can be selected to avoid too low a level of biotinylation in a population of fragments which can, under some conditions, yield a substantial fraction of circles that contain no biotin moieties for subsequent selection.

The invention further provides a population of fragments in which the ends of the fragments selectively carry the biotin modification. For example, the fragments in the population may only carry the biotin modification at the ends of the fragments. The level of biotin and the ratio of circular to linear fragments obtained under particular conditions tested is shown in figure 12. If only a small number of the template strands become circularised, then some of the sequence coverage may be lost from the library. This may be measured in terms of diversity, which is a measure of how many times a certain sequence appears more than once in a given number of sequence reads. A library of a single template will have a duplication rate of 100%, whereas for a nucleic acid sample with a large number of bases, it may be desirable in certain embodiments that very few fragments be exact duplicates upon fragmentation. Typically it is advantageous that the protocol used to prepare the sample gives rise to a low level of duplication, as duplication is a measure of bias in the method. It can be seen in figure 11 that under the conditions tested a higher level of bias is seen with a higher level of biotinylation, which is indicative of the low efficiency of self ligation from highly biotinylated templates. The level of biotinylation in the self-ligated product can be controlled using the ratio of biotinylated to unbiotinylated dNTP's in the end repair mix. The biotinylated dNTP may be between 1-20% of the concentration of the unbiotinylated dNTP of the same nucleotide. The biotinylated dNTP may be between 2-5% of the concentration of the unbiotinylated dNTP of the same nucleotide.

Fragmentation of the primary nucleic acid sample may also be achieved using randomised amplification, for example with randomised primers. Randomised primers are members of a population of primers, the population being degenerate with respect to having 2 or more different nucleotide species at one or more positions along a given length of primer sequence. For example, a population of primers can include individual primers having one of four different nucleotide species at every position along a given length such that the population as a whole includes every possible sequence of that length. In some embodiments randomized primers can be members of a population in which a first region of the primers is randomized and a second region is common to all of the primers in the population. Random amplification may also be obtained by using low stringency conditions that result in mis-priming of a particular sequence, or by using primers containing universal bases that can hybridise against more that one of the nucleobases in a template strand.

Whilst the primary sample remains intact in randomised amplification, this process is described as 'fragmentation' for the purposes of this application, as a collection of shorter pieces of the original sample are obtained. The primers used in randomized amplification may carry a ligand allowing for their later selection. Such a ligand may be biotin, dinitrophenol, fluorescein or any other molecule that can be attached to a short oligonucleotide sequence, and

has a known receptor that can be used to bind to the ligand. Each of the primers may carry a ligand, or a subset of the primers may carry a ligand, with the remainder being unlabelled. Examples will be described using biotin/streptavidin, but it is anticipated that other similar moieties could be substituted. The use of the selection step allows for generation of libraries that carry a high population of fragments where the original ends were spaced apart in the sample. As only the ends of the fragments carry the biotin moiety, the only regions of the sample that will be selected after fragmentation of the circles will be those that originally contained the two ends of the linear fragments, not the internal regions of the linear fragments, which do not have the biotin moiety.

The length of the primers can be fairly short, for example 6-12 bases, and every primer, or a subset of the primers can carry a ligand irrespective of sequence. In order to prepare suitable randomised biotinylated oligonucleotide, which carry a free 3' hydroxyl and 5' phosphate for polymerase extension and ligation respectively, the biotin can be attached to the internal nucleobases. Such an attachment may be carried out post synthetically, by using a mixture of four amino modified phosphoramidites (for example as available from Glen Research) to prepare an oligonucleotide where a specific location (for example base 3) carries an amino group independent of the sequence context of each random sequence. The pool of oligonucleotides can then be reacted with biotin NHS ester. Alternatively each of the four nucleobase-biotin phosphoramidites can be prepared such that the oligonucleotides are synthesised with the biotin group attached.

The randomised primers may be used to amplify any sequence of interest. Conditions for random primer amplification of target sequences using a nucleic acid polymerase and four nucleotide triphosphates are well known in the art, and kits for random primer amplification are available. If the method is performed using primer amplification rather than mechanical fragmentation of the primary sample, then the products will automatically be blunt ended, so there is no requirement for the enzymatic polishing of fragments. The random amplification process generates duplex fragments of random sizes, so a size selection step may be carried out to select a suitable size of fragments prior to the circularisation. The optionally size selected fragments can then be put through the intramolecular self-ligation circularisation step as described below.

In order to improve the efficiency of subsequent circularisation, the fragment ends can be blunt ended and phosphorylated. The phosphate moiety can be introduced via enzymatic treatment, for example, using polynucleotide kinase. The end repair and phosphorylation may be carried out in a single treatment, or as two consecutive steps. The 5' phosphate can undergo efficient ligation with the 3' hydroxyl of the same strand to form a closed circular duplex. Furthermore the end repair reaction may be carried out in two steps, firstly using unlabelled nucleotides then using nucleotides modified with labels. The two step process helps to selectively produce fragments having modified nucleotides incorporated at the ends of the fragments (rather than fragments having modified nucleotides that are incorporated at locations away from the ends of the fragments). If the DNA fragments are not first blunted using the pre-end repair step, then it is possible that biotin nucleotides can be incorporated at sites some distance away from the terminal ends of the DNA fragments, leading to subsequent selection of biotinylated DNA that does not represent the junction of the DNA fragment's two ligated ends. This can lead to a higher proportion of final paired end sequences being inward facing fragments rather than the desired outward facing fragments, as shown in fig 10. The second end repair process can be carried out using a polymerase with 3'-5' exonuclease activity, such as T4 DNA polymerase or Klenow polymerase in order to exchange the nucleosides at the end of the duplexes.

It is thus advantageous if the biotinylation process is carried out after a first end repair step, and with a low level of biotin dNTP in the reaction mix. The first end repair may be carried out with an unmodified nucleotide mix, and the second end repair with a mix of modified and unmodified nucleotides. In the case of the biotinylated nucleotides, the final concentration of the biotinylated nucleotides may be 2-20% of the nucleotide concentration. Alternatively the end repair may be carried out in a single process with the biotinylated nucleotides added after the non-biotinylated nucleotides up to a level of 2-20% of the final nucleotide concentration. The balance of the exonuclease and polymerase activity of the enzymes in the mixture can be used to exchange the previously repaired bases with their biotinylated counterparts. It may be further advantageous to carry out a third end repair reaction in the absence of biotinylated nucleosides such that the very end bases do not carry biotinylated bases, as this may further improve the efficiency of the self-ligation reaction.

In the step of fragmenting circular constructs, fragmentation by mechanical means (nebulization, sonication and Hydroshear for example) again results in fragments with a heterogeneous mix of blunt and 3'- and 5'-overhanging ends. It is therefore desirable to repair the fragment ends using methods or kits (such as the Lucigen DNA terminator End Repair Kit) known in the art to generate ends that are optimal for insertion, for example, into blunt sites of cloning vectors. In a particular embodiment, the fragment ends of the population of nucleic acids are blunt ended. More particularly, the fragment ends are blunt ended and phosphorylated. The phosphate moiety can be introduced via enzymatic treatment, for example, using polynucleotide kinase.

In a particular embodiment, the target polynucleotide sequences are prepared with single overhanging nucleotides by, for example, activity of certain types of DNA polymerase such as Taq polymerase or Klenow exo minus polymerase which has a nontemplate-dependent terminal transferase activity that adds a single deoxynucleotide, for

example, deoxyadenosine (A) to the 3' ends of, for example, PCR products. Such enzymes can be utilised to add a single nucleotide 'A' to the blunt ended 3' terminus of each strand of the target polynucleotide duplexes. Thus, an 'A' could be added to the 3' terminus of each end repaired duplex strand of the target polynucleotide duplex by reaction with Taq or Klenow exo minus polymerase, whilst the adaptor polynucleotide construct could be a T-construct with a compatible 'T' overhang present on the 3' terminus of each duplex region of the adaptor construct. This end modification also prevents self-ligation of both vector and target such that there is a bias towards formation of the combined ligated adaptor-target sequences.

Paired reads can be obtained on fragments of any length, for example PCR amplicons of 2-10 Kb or DNA clones isolated from bacteria or other biological sources. The targets may be the ends of fosmid molecules of around 40 kB or the ends of Bacterial artifical chromosomes (BAC's) of around 100-200 kB. Such samples may be defined as primary double stranded nucleic acid targets. The ends of targets derived from such sources may be sequenced according to the methods of the invention without fragmentation to obtain reads from the ends of each unfragmented target, or the target may be fragmented. The fragmented targets may be size selected, for example by gel electrophoresis, to obtain a narrow size distribution on the target fragments. Paired reads spaced throughout the sample may be used as a tool for de-novo assembly of a previously unsequenced sample, as well as for resequencing a sample where a reference genome is available. The methods described herein are suitable for use with nucleic acid molecules of any length, obtained from any source, where knowledge of the sequences at either end of the molecules is desired.

A collection of linear double stranded target nucleic acid fragments can be provided for use in a method of the invention. The length may be a PCR fragment of 100 bases or more, or may be a long DNA fragment such as a BAC of 200000 or more bases. The fragments are typically between lkb-50kB, for example PCR fragments of 5-10 kB or fosmids of 40kb. The size distribution depends on the source and quality of the sample. In the case of PCR fragments, the size is controlled by the distance between the two primer sites. In the examples that rely on random fragmentation of a longer primary sample nucleic acid, a size selection step (for example gel electrophoresis) may be performed that defines both the length and distribution of the fragments. Multiple experiments may be performed on the same primary sample involving different fragment lengths in each case. For example the same sample of genomic DNA may be split, treated and size selected to give a population of 2kb, 5kb, 10kb and 20kb fragments. Each separate pool of fragments is suitable for treatment using the same methodology as described herein, which is independent of the length of the fragments.

The double stranded nucleic acid fragments, which may be end repaired as described above, are self ligated to form a circular construct. The use of an adapter sequence is optional, and in the methods of the invention adapter sequences are not used. The absence of adapters means that every single base in the circular construct derives from the primary target, either directly or through amplification of the primary target sequence, there are no artificially synthesised sequences that are not present in the primary target. The ligation reaction can be performed under conditions that favour efficient self ligation of the intramolecular blunt ends over intermolecular ligation in which the blunt ends of two separate fragments are ligated to each other. For example, ligation can be carried out at a low concentration of target fragments to favour intramolecular ligation.

The ligation reaction may be carried out using any suitable ligase, for example T3 or T4 ligase. Ligation with T3 ligase was shown to be an improvement over T4 ligase due to both the increase in diversity and a lowering of insert-insert intramolecular complexes (chimeras). T3 is more efficient than T4 in the highlighted protocol, as shown in Figure 13, which shows the results from sequencing data where T3 and T4 ligase are used in the self-ligation reaction, and indicates that T3 lowers the level of duplicates and intramolecular chimeras compared to T4 under the same ligation conditions using the identical size selected DNA in both ligations.

Upon ligation, for example using a T3 or T4 DNA ligase, the desired constructs are circular nucleic acid fragments. In addition to the circular constructs, there may be a large quantity of non-circular target constructs. It is desirable to select for circular constructs over non-circular constructs. Such selection can be carried out, for example, by selective removal of non-circular targets or selective amplification of circular targets. Selective removal includes embodiments wherein as many of the linear sequences are removed as possible to increase the population of circularized fragments that generate paired read information in subsequent sequencing steps. For example, the level of linear products can be depleted by exonuclease treatment, for example using DNase, such as in the commercially available 'Plasmid Safe' treatment (Epicentre Technologies). Such exonucleases rely on the presence of a terminus of the DNA strand, and therefore only digest linear and not circular constructs. An alternative to removing linear strands is to selectively amplify the circular constructs. Such an amplification reaction may be rolling circle amplification (RCA). The RCA reaction may be carried out using the polymerase Phi29, which has high processivity and strand displacing activity. The priming of the RCA reaction may be carried out using specific nucleic acid primers, or may be carried out using random primers. The primers may be short sequences of 5-10 bases, for example hexamers. The RCA reaction may be carried out using commercially available kits, for example the templiphi kit from Amersham Biosciences (GE Product number 25-6400-10).

It should be noted that the products of RCA are no longer circles, but are long strands of sequences where the circular material is copied multiple times in a linear strand. Each RCA product is thus a long linear string containing

concatomeric copies of the circular sequence. For the methods where the ends of the fragments are labelled prior to rolling circle amplification and the label is not copied into the RCA product the label information is lost upon amplification, as the ends of the original fragments are no longer marked. However the amplified linear strands contain multiple copies of sequences where the ends of the original fragments are directly linked together. If the strands are then fragmented and two regions of the fragments are sequenced, a significant proportion of the sequence reads will contain information deriving from two locations spaced well apart in the original primary sequence from which the fragments were derived.

Fragmentation of the circular constructs, or the products of the circle amplification reaction gives rise to a second population of linear fragments. A percentage of these fragments will span the two ends of the first population of linear target fragments. The percentage of the population which covers the ends of the first population can be increased using methods that select for the nucleoside triphosphates used in the end labelling reaction. For example, the use of biotinylated dNTP's in the end labelling step, means that a proportion of the second linear fragments, namely those deriving from the ends of the first fragment population contain biotin, and fragments deriving from the central region of the first fragment population do not contain biotin. Isolation of the biotinylated sequences from the non biotinylated sequences therefore gives rise to a selection of the ends of the original fragments, thus increasing the proportion of paired end reads.

The isolation of the labelled fragments may be carried out at any point after fragmentation of the circles. Two of the possible alternatives are shown in figures 2b and 2c. In protocol 2b, the biotin selection is carried out after the ligation reaction which attaches the adapters. In protocol 2c, the biotin selection 'pull out' is carried out directly after the fragmentation, before the adapter ligation. Figure 14 shows comparative sequencing data from protocols 2a/b and 2c, and indicates that having the pull out earlier in the process, directly after the fragmentation of the self-ligated circles lowers the level of the incorrect inward 'innie' non ends of the first fragment sequences.

Upon sequencing a library generated using the protocols described herein, data on two fragment types is generated. The inward 'innie' read pairs align in an inward facing orientation, and are the unwanted result of selection fragments which should not carry a biotin modification. The size of the fragments are a reflection of the size of the fragments obtained in the second fragmentation step used to fragment the circles, and may be, for example 400-600 base pairs. The desired read pairs are those that align in an outward facing orientation with a gap size which reflects the size of the inserts obtained from the previously described process of fragmentation followed by self-ligation. For example, read pairs produced from fragmentation and self ligation can have gaps that are around 2000 bp. The percent of inward facing pairs can be taken as a measure of the success of the bead enrichment steps, and in particular embodiments is desired to be as low as possible. Any improvements which lower the level of inward facing read pairs may be considered advantageous to increase the level of the outward 'long insert' sequences.

Once the biotinylated fragments are attached to the beads, then a number of subsequent steps can be carried out on the bead-nucleic acid constructs. For example, the fragments can be end repaired, have single nucleotide tails added and ligated to adapters whilst remaining bound to the beads. The material can then be copied from the beads using the first step of an amplification reaction, which also amplifies the material 'removed from' the beads.

The fragments created may be further treated by end repair and 5' phosphorylation in order to prepare fragments that are able to efficiently undergo ligation. The end repair may be carried out by any suitable DNA polymerase, which may also contain exonuclease activity to make blunt any 3' overhanging strands. In order to prevent adapter-adapter ligation, or fragment-fragment ligation, the ends of the fragments may be treated to generate a 3' overhanging sequence. Exposure of the fragments to an exo-minus polymerase such as Taq polymerase or Klenow exo-minus polymerase and a single species of dNTP gives rise to a single non-template dNTP being added to the 3' end of each blunt ended duplex. If the adapter also carries a single 3' overhanging base complementary to the overhang on the fragments, then the only ends that can undergo effective ligation are adapter-target. The use of Klenow polymerase means that the tailing reaction can be carried out at a lower temperature, which is advantageous to ensure the short fragments that contain a high level of A-T base pairing are not denatured during the treatment and are thus retained in the population of adapter-target-adapter sequences.

These fragments may be treated for sequencing using known methods, for example as described in co-pending applications WO07052006 and US 20070128624. The ends of the linear fragments are ligated to known adapter sequences. The known adapter sequences on the ends of each fragment allow universal amplification, whereby a single pair of primers is used to amplify a plurality of different target sequences by hybridizing to the common adapter sequences that flank the different target sequences. The adapters may contain a region of double stranded sequence to enable ligation to the second population of linear fragments, and may further contain a region of single stranded non-complementary sequence. These adapters may be termed mismatched adapters. Mismatched adaptors be formed by annealing two partially complementary polynucleotide strands so as to provide, when the two strands are annealed, at least one double-stranded region and at least one unmatched region. The adapters may also contain a single overhanging base complementary to the overhanging base on the target fragments.

The 'double-stranded region' of the adaptor is a short double-stranded region, typically comprising 5 or more consecutive base pairs, formed by annealing of the two partially complementary polynucleotide strands. This term simply

refers to a double-stranded region of nucleic acid in which the two strands are annealed and does not imply any particular length or structural conformation.

Generally it is advantageous for the double-stranded region to be as short as possible without loss of function. The term 'function' in this context refers to the ability of the double-stranded region to form a stable duplex under standard reaction conditions for an enzyme-catalysed nucleic acid ligation reaction, which conditions are well known to the skilled practitioner (e.g. incubation at a temperature in the range of from 4°C to 25°C in a ligation buffer appropriate for the enzyme). In other words, function in this context refers to the ability of the two strands forming the adaptor to remain partially annealed during ligation of the adaptor to a target molecule. It is not absolutely necessary for the double-stranded region to be stable under the conditions typically used in the annealing steps of primer extension or PCR reactions.

Since identical adaptors are ligated to both ends of each template molecule, the target sequence in each adaptor-target construct is flanked by complementary sequences derived from the double-stranded region of the adaptors. The longer the double-stranded region, and hence the complementary sequences derived therefrom in the adaptor-target constructs, the greater the possibility that the adaptor-target construct is able to fold back and base-pair to itself in these regions of internal self-complementarity under the annealing conditions used in primer extension and/or PCR. It is, therefore, generally preferred that the double-stranded region is 20 or less, 15 or less, or 10 or less base pairs in length in order to reduce this effect. The stability of the double-stranded region may be increased, and hence its length potentially reduced, by the inclusion of non-natural nucleotides which exhibit stronger base-pairing than standard Watson-Crick base pairs.

The two strands of the adaptor can be 100% complementary in the double-stranded region. It will, however, be appreciated that one or more nucleotide mismatches may be tolerated within the double-stranded region, provided that the two strands are capable of forming a stable duplex under standard ligation conditions.

Adaptors will generally include a double-stranded region forming the 'ligatable' end of the adaptor, i.e., the end that is joined to a target polynucleotide in the ligation reaction. The ligatable end of the adaptor may be blunt or, in other embodiments, short 5' or 3' overhangs of one or more nucleotides may be present to facilitate/promote ligation. The 5' terminal nucleotide at the ligatable end of the adaptor is preferably phosphorylated for example to enable phosphodiester linkage to a 3' hydroxyl group on the target polynucleotide.

The term 'unmatched region' refers to a region of the adaptor wherein the sequences of the two polynucleotide strands forming the adaptor exhibit a degree of non-complementarity such that the two strands are not capable of fully annealing to each other under standard annealing conditions for a primer extension or PCR reaction. The unmatched region(s) may exhibit some degree of annealing under standard reaction conditions for a enzyme-catalysed ligation reaction, provided that the two strands revert to single stranded form under annealing conditions in an amplification reaction.

The adaptor constructs may contain exonuclease resistant modifications such as methylphosphonate or phosphorothioate linkages. Such modifications lower the level of adaptor-dimers present in the library as the two adaptors can not undergo ligation without removal of their non complementary overhangs. The adaptors can be treated with an exonuclease enzyme, prior to the ligation reaction with the target, to ensure that the overhanging ends of the strands can not be removed during the ligation process. Treatment of the adaptors in this manner reduces the formation of the adaptor-dimers at the ligation step.

The mismatched region of the adapter can be hybridised with a primer which can be extended by a polymerase to produce a copy of the adapter-target-adapter sequences. Each strand of each template molecule in the library formed in the primer extension reaction will therefore have the following structure, when viewed as a single strand:

5'-[common sequence I]-[target sequence]-[common sequence II]-3'

wherein 'common sequence I' represents a sequence derived from a primer that hybridises to and copies a first strand of the mismatched adaptor and is common to all template molecules in the library generated in the initial primer extension reaction; 'target' represents a sequence derived from one strand of the target polynucleotide duplex and may be different in different individual template molecules within the library; and 'common sequence II' represents a sequence derived from copying of a second strand of the mismatched adaptor and is also common to all template molecules in the library generated in the initial primer extension reaction.

Since 'common sequence I' and 'common sequence II' are common to all template strands in the library they may include 'universal' primer-binding sequences, enabling all templates in the library to be ultimately amplified in a solid-phase PCR procedure using universal primers.

The common 5' and 3' end sequences denoted 'common sequence I' and 'common sequence II' are not *fully* complementary to each other, meaning that each individual template strand can contain different (and non-complementary) universal primer sequences at its 5' and 3' ends.

It is generally advantageous for complex libraries of templates to be amplified, for example by PCR or isothermal amplification (e.g. whole genome amplification), either in solution or on a solid support, to include regions of 'different'

sequence at their 5' and 3' ends, which are nevertheless common to all template molecules in the library, especially if the amplification products are to be sequenced ultimately. For example, the presence of a common unique sequence at one end only of each template in the library can provide a binding site for a sequencing primer, enabling one strand of each template in the amplified form of the library to be sequenced in a single sequencing reaction using a single type of sequencing primer. The method of the invention may also be applied to the preparation of libraries which are amplified *in vivo*, such as for example bacterial cDNA libraries and the like.

Typically 'common sequence I' and 'common sequence II' consist of no more than 100, or no more than 50, or no more than 40 consecutive nucleotides at the 5' and 3' ends, respectively, of each strand of each template polynucleotide. The precise length of the two sequences may or may not be identical. The nucleotide sequences of 'common sequence I' and 'common sequence II' in the template polynucleotides are determined in part by the sequences of the adaptor strands ligated to the target polynucleotides and in part by the sequence of the primer used in the initial primer extension reaction, and any subsequent rounds of nucleic acid amplification.

In embodiments wherein the initial primer extension product is subjected to further amplification by conventional PCR, then the products of the amplification reaction are double-stranded polynucleotides, one strand of which has the structure:

5'-[common sequence I]-[target sequence]-[common sequence II]-3'

It will be appreciated that 'common sequence II' in the amplification products may differ somewhat to the 'common sequence II' present in the products of the initial primer extension reaction, since the former is determined in part by the sequence of the PCR primer used to prime synthesis of a polynucleotide strand complementary to the initial primer extension product, whereas the latter will be determined solely by copying of the adaptor sequences at the 3' ends of the adaptor-template constructs in the initial primer extension. Nevertheless, since the PCR primer is designed to anneal to a sequence in the initial extension products which is complementary to the 3' adaptor, the two forms of 'common sequence II' will contain identical sequence, at least at the 3' end. Additional sequence may be included at the 5' end of 'common sequence II' in the amplified products, for example by the use of 'tailed' PCR primers, as described in detail below. In other embodiments the common sequences present in the amplification products may actually be shorter than the common sequences included in the adaptors originally ligated to the target.

The precise nucleotide sequences of the common regions of the template molecules in the library are not material to the invention and may be selected by the user. In particular embodiments, the common sequences must at least comprise 'primer-binding' sequences which enable specific annealing of amplification primers when the templates are in use in a solid-phase amplification reaction. The primer-binding sequences are thus determined by the sequence of the primers to be used for solid-phase amplification. The sequence of these primers in turn is advantageously selected to avoid or minimise binding of the primers to the target portions of the templates within the library under conditions used for the amplification reaction, but is otherwise not particularly limited. By way of example, if the target portions of the templates are derived from human genomic DNA, then the sequences of the primers to be used in solid phase amplification can be selected to minimise non-specific binding to any human genomic sequence.

The conditions encountered during the annealing steps of an amplification reaction will be generally known to one skilled in the art, although the precise annealing conditions will vary from reaction to reaction (see Sambrook et al., 2001, Molecular Cloning, A Laboratory Manual, 3rd Ed, Cold Spring Harbor Laboratory Press, Cold Spring Harbor Laboratory Press, NY; Current Protocols, eds Ausubel et *al*.). Typically such conditions may comprise, but are not limited to, (following a denaturing step at a temperature of about 94°C for about one minute) exposure to a temperature in the range of from 40°C to 72°C (preferably 50-68°C) for a period of about 1 minute in standard PCR reaction buffer.

Different annealing conditions may be used for a single primer extension reaction, which is not part of a cycle of a PCR reaction (again see Sambrook et al., 2001, Molecular Cloning, A Laboratory Manual, 3rd Ed, Cold Spring Harbor Laboratory Press, Cold Spring Harbor Laboratory Press, NY; Current Protocols, eds Ausubel et *al*.). Conditions for primer annealing in a single primer extension include, for example, exposure to a temperature in the range of from 30 to 37°C in standard primer extension buffer. It will be appreciated that different enzymes, and hence different reaction buffers, may be used for a single primer extension reaction as opposed to a PCR reaction. There is no requirement to use a thermostable polymerase for a single primer extension reaction.

It is to be understood that the 'unmatched region' is provided by different portions of the same two polynucleotide strands which form the double-stranded region(s). Mismatches in the adaptor construct can take the form of one strand being longer than the other, such that there is a single stranded region on one of the strands, or a sequence selected such that the two strands do not hybridise, and thus form a single stranded region on both strands. The mismatches may also take the form of 'bubbles', wherein both ends of the adaptor construct(s) are capable of hybridising to each other and forming a duplex, but the central region is not. The portion of the strand(s) forming the unmatched region are not annealed under conditions in which other portions of the same two strands are annealed to form one or more double-stranded regions. The unmatched region can be of sufficient length to undergo subsequent hybridisation. It is to be

understood that a single-stranded or single base overhang at the 3' end of a polynucleotide duplex that subsequently undergoes ligation to the target sequences does not necessarily constitute an 'unmatched region' in the context of this invention.

The lower limit on the length of the unmatched region will typically be determined by function, for example the need to provide a suitable sequence for binding of a primer for primer extension, PCR and/or sequencing. Theoretically there is no upper limit on the length of the unmatched region, except that in general it is advantageous to minimise the overall length of the adaptor, for example in order to facilitate separation of unbound adaptors from adaptor-target constructs following the ligation step. Therefore, it is preferred in particular embodiments that the unmatched region should be less than 50, or less than 40, or less than 30, or less than 25 consecutive nucleotides in length.

The precise nucleotide sequence of the adaptors is not material to the invention and may be selected by the user such that the desired sequence elements are ultimately included in the common sequences of the library of templates derived from the adaptors, for example to provide binding sites for particular sets of universal amplification primers and/or sequencing primers. Additional sequence elements may be included, for example to provide binding sites for sequencing primers which will ultimately be used in sequencing of template molecules in the library, or products derived from amplification of the template library, for example on a solid support. The adaptors may further include 'tag' sequences, which can be used to tag or mark template molecules derived from a particular source. The general features and use of such tag sequences is described in applicant's pending application published as WO 05/068656.

Although the precise nucleotide sequence of the adaptor is non-limiting to the invention, in some embodiments the sequences of the individual strands in the unmatched region should be such that neither individual strand exhibits any internal self-complementarity which could lead to self-annealing or formation of hairpin structures, etc. under standard annealing conditions. Self-annealing of a strand in the unmatched region is to be avoided as it may prevent or reduce specific binding of an amplification primer to this strand.

The mismatched adaptors are preferably formed from two strands of DNA, but may include mixtures of natural and non-natural nucleotides (e.g. one or more ribonucleotides) linked by a mixture of phosphodiester and non-phosphodiester backbone linkages. Other non-nucleotide modifications may be included such as, for example, biotin moieties, blocking groups and capture moieties for attachment to a solid surface, as discussed in further detail below.

In order to effectively select any modified fragments contained the labelled end repaired constructs, the selection can be carried out before the amplification of the ligated adapters. The dNTP constructs can carry a variety of ligands to allow for selection, for example biotin, fluorescein or dinitrophenyl (DNP). In the case if biotin labelled fragments, the selection step can be carried out using avidin or streptavidin, which may be immobilised to a solid support, for example microparticles or beads. Similarly, bead-immobilized anti-DNP or anti-fluorescein antibody can be used to select for fragments labelled with DNP or fluorescein nucleotides. The beads can be magnetic in order to aid separation of the solid phase and solution phase components. The biotinylated fragments can be isolated before or after the ligation step, and the immobilised fragments can either be eluted from the solid support, or amplified on the surface of the beads. In this context, the original biotinylated fragments may remain immobilised, and the non biotinylated fragments are generated in solution.

The methods described herein also provide a circular construct with no additional adapter sequences. As the ends of the primary target fragments are ligated to themselves rather than to an intermediate sequence, each and every base of the circular construct derives from the primary target. The sequence of each circular construct is thus entirely unknown, and the circles contain no regions of known sequence. The circular constructs can be prepared from linear fragments with blunt ends in an intramolecular self ligation reaction. The blunt ends of the linear fragments may be generated using a polymerase, and nucleoside triphosphates, which may carry a label such as biotin for the selection of the ends of the linear fragments. Thus the circular constructs may comprise modified (labelled) nucleotides at a selected location in the circle, rather than throughout the whole circle. The selected location may be small, for example less than 10 base pairs out of a circle of greater than 1000 base pairs, and each circle may only carry a single modification, or may carry less than 10 modified nucleotides. The modifications to the circles may comprise, for example, biotin attached through the nucleobases to less than 1% of the nucleobases in the circular fragments.

### Use of the 3' and 5' modified library

The nucleic acid library described herein may be used in methods of sequencing, either as an array of single molecules, as described in WO0006770, or may be amplified prior to sequencing, either on beads in an emulsion, for example as described in WO0406984, or on a planar array.

One suitable format for the sequence analysis of the library is the provision of a plurality of template polynucleotide duplexes immobilised on a solid support in the form of amplified clusters as described in WO9844151 and WO00018957. Each of the duplexes within a particular cluster comprises the same double-stranded target region to be sequenced. The duplexes are each formed from complementary first and second template strands which are linked to the solid support at or near to their 5' ends. Typically, the template polynucleotide duplexes will be provided in the form

of a clustered array. Amplification of the array may be carried out using thermocycling, or isothermally, using changes of reagents to denature the hybridised strands.

WO07010252 also describes a method of reading both the first and second template strands from each cluster, but suffers from the disadvantage that only half the strands in each cluster are sequenced. This diminishes the signal intensity of the sequencing reads. The methodology described herein allows the sequencing of essentially all of the copies of each strand in each cluster, and therefore produces a signal of greater intensity than the previous methodology. This property of the present methodology confers greater sensitivity with respect to signal detection and means that longer reads can be obtained from smaller clusters than the prior art.

When referring to immobilisation or attachment of molecules (e.g. nucleic acids) to a solid support, the terms "immobilised" and "attached" are used interchangeably herein and both terms are intended to encompass direct or indirect, covalent or noncovalent attachment, unless indicated otherwise, either explicitly or by context. In certain embodiments of the invention covalent attachment may be preferred, but generally all that is required is that the molecules (e.g. nucleic acids) remain immobilised or attached to the support under the conditions in which it is intended to use the support, for example in applications requiring nucleic acid amplification and/or sequencing.

Certain embodiments of the invention may make use of solid supports comprised of an inert substrate or matrix (e.g. glass slides, polymer beads etc) which is been "functionalised", for example by application of a layer or coating of an intermediate material comprising reactive groups which permit covalent attachment to biomolecules, such as polynucleotides. Examples of such supports include, but are not limited to, polyacrylamide hydrogels supported on an inert substrate such as glass. In such embodiments, the biomolecules (e.g. polynucleotides) may be directly covalently attached to the intermediate material (e.g. the hydrogel) but the intermediate material may itself be non-covalently attached to the substrate or matrix (e.g. the glass substrate). The term "covalent attachment to a solid support" is to be interpreted accordingly as encompassing this type of arrangement.

As will be apparent to the skilled reader, references herein to a particular nucleic acid sequence may, depending on the context, also refer to nucleic acid molecules which comprise the nucleic acid sequence. Sequencing of a target fragment means that a read of the chronological order of bases is established. The bases do not, however, need to be contiguous, nor does every base on the entire fragment have to be sequenced.

After the amplification to produce colonies on a solid support, the colonies are in the form of hybridised duplexes. In order to hybridise a primer for sequencing, the colonies (clusters) may be treated to remove one of the strands from the surface. If the molecules are immobilised such that one of the two immobilised ends can be cleaved from the surface, upon such cleavage the resulting double stranded DNA, which is now immobilised at only one end of the duplex, can be made single stranded using heat or chemical denaturing conditions to give a single stranded molecule containing a primer hybridisation site. The process of removing all or a portion of one immobilised strand in a 'bridged' double-stranded nucleic acid structure may be referred to herein as 'linearisation'. The single stranded molecule can be sequenced using a first sequencing primer, which can then be removed and a second sequencing primer introduced to allow a second read if the adaptor sequence is present in the library constructs. If the constructs are not linearised, then it is possible to obtain four reads from each duplex, since each strand can be sequenced twice, once from the 3' terminal adaptor sequence, and once from the central adaptor sequence.

Either the first or second strand of the template duplexes can include a cleavage site for linearization of the duplex strands. The cleavage site is a site which allows controlled cleavage of the first or second template strand by chemical, enzymatic or photochemical means. The double stranded polynucleotide is then only immobilised through one end. The polynucleotide is then denatured to leave a single stranded polynucleotide immobilised at the 5'-end. A first sequencing primer can then be hybridised to a single-stranded region of the template and used as the primer for a sequencing reaction, after which it is removed from the template, and a second sequencing primer is hybridised and used for sequencing of a different region of the single stranded template.

Any suitable enzymatic, chemical or photochemical cleavage reaction may be used to cleave. The cleavage reaction may result in removal of a part or the whole of the strand being cleaved. Suitable cleavage means include, for example, restriction enzyme digestion, in which case the cleavage site is an appropriate restriction site for the enzyme which directs cleavage of one or both strands of a duplex template; RNase digestion or chemical cleavage of a bond between a deoxyribonucleotide and a ribonucleotide, in which case the cleavage site may include one or more ribonucleotides; chemical reduction of a disulphide linkage with a reducing agent (e.g. TCEP), in which case the cleavage site may include an appropriate disulphide linkage; chemical cleavage of a diol linkage with periodate, in which case the cleavage site may include a diol linkage; generation of an abasic site and subsequent hydrolysis, etc.

In one embodiment cleavage may occur at a cleavage site in one or both strands of a template polynucleotide duplex which comprises one or more or any combination of non-natural nucleotides, ribonucleotides or a non-nucleotide chemical modifications. Suitable cleavage techniques for use in the method of the invention are described in full in co-pending application WO07010251.

The amplified polynucleotides can then be treated in such a way to allow primer hybridisation. This can be performed either by heating the amplified clusters to denature the duplexes, followed by cooling in the presence of the

first sequencing primer, by a chemical treatment such as sodium hydroxide to denature the duplexes or by a treatment to cleave one or both of the strands of the duplex polynucleotide.

Each polynucleotide sequence on the array contains the same universal primer recognition regions to allow the same primers to be used to sequence every cluster. A first sequencing primer is then hybridised to the first template strand and a sequencing reaction proceeds via successive incorporation of nucleotides to the first sequencing primer, resulting in determination of the sequence of a first tag of the target polynucleotide.

Hybridisation of sequencing primer to the template strand is achieved by contacting the primer and template strand under conditions which promote annealing of primer to template. Such conditions will generally be well known to those skilled in the art of molecular biology.

When the first sequencing reaction is complete, the extended first sequencing primer may be removed from the surface. This can be achieved by heating, or chemical denaturation. If an adaptor region is present in the clusters between the two end regions, a second sequencing primer can then be hybridised to the adaptor region of the template and a sequencing reaction proceeds via successive addition of nucleotides to the second sequencing primer, resulting in determination of the sequence of a second region of the target polynucleotide.

Sequencing can be carried out using any suitable "sequencing-by-synthesis" technique, wherein nucleotides are added successively to a free 3' hydroxyl group, typically provided by annealing of a sequencing primer, resulting in synthesis of a polynucleotide chain in the 5' to 3' direction. In a particular embodiment, the nature of the nucleotide added is determined after each addition.

One particular sequencing method which can be used in the methods of the invention relies on the use of modified nucleotides that can act as reversible chain terminators. Nucleotides for use in the invention are described fully in WO04018497 and US7057026. Once the modified nucleotide has been incorporated into the growing polynucleotide chain complementary to the region of the template being sequenced there is no free 3'-OH group available to direct further sequence extension and therefore the polymerase can not add further nucleotides. Once the nature of the base incorporated into the growing chain has been determined, the 3' block may be removed to allow addition of the next successive nucleotide. By ordering the products derived using these modified nucleotides it is possible to deduce the DNA sequence of the DNA template. Such reactions can be done in a single experiment if each of the modified nucleotides has attached thereto a different label, known to correspond to the particular base, which facilitates discrimination between the bases added at each incorporation step. Alternatively, a separate reaction may be carried out containing each of the modified nucleotides, which are added separately.

The modified nucleotides may carry a label to facilitate their detection. In a particular embodiment, the label is a fluorescent label. Each nucleotide type may carry a different fluorescent label. Fluorescent labels suitable for use in the current invention are described in co-pending PCT application PCT/GB2007/001770. However the detectable label need not be a fluorescent label. Any label can be used which allows the detection of the incorporation of the nucleotide into the DNA sequence.

One method for detecting the fluorescently labelled nucleotides comprises using laser light of a wavelength specific for the labelled nucleotides, or the use of other suitable sources of illumination. The fluorescence from the label on the nucleotide may be detected by a CCD camera or other suitable detection means. An imaging system suitable for determining the fluorescent signal from incorporated nucleotides is described in PCT application number PCT/US2007/07991.

The methods of the invention are not limited to use of the sequencing method outlined above, but can be used in conjunction with essentially any sequencing methodology which relies on successive incorporation of nucleotides into a polynucleotide chain. Suitable techniques include, for example, Pyrosequencing™, FISSEQ (fluorescent in situ sequencing), MPSS (massively parallel signature sequencing) and sequencing by ligation-based methods, for example as described in US6306597.

The target polynucleotide to be sequenced using the method of the invention may be any polynucleotide that it is desired to sequence. The target polynucleotide may be of known, unknown or partially known sequence, such as, for example in re-sequencing applications. Using the template preparation method described in detail below it is possible to prepare arrays of templates starting from essentially any double-stranded target polynucleotide of known, unknown or partially known sequence of any length. With the use of arrays it is possible to sequence multiple targets of the same or different sequence in parallel. A particular application of the pairwise method is in the sequencing of fragments of genomic DNA. The method provides particular advantages in the identification of genome rearrangements, since the two regions of sequence obtained for each target molecule using the method will be known to be linked within a certain distance of each other in the genome, depending on the size of the fragments of the starting target molecule.

Preparation of templates to be sequenced

Suitable templates for sequencing using the method of the invention can be prepared by solid-phase nucleic acid amplification to produce nucleic acid colonies. This can be done using procedures analogous to those described

in WO 98/44151 and WO 00/18957.

For amplification to proceed, a mixture of two amplification primers is immobilised or "grafted" onto the surface of a suitable solid support.

The amplification primers are oligonucleotide molecules having the following structures:

Forward primer: A-L-X-S1
Reverse primer: A-L-X-S2

Wherein A represents a moiety which allows attachment to the solid support, L is an optional linker, or spacer moiety, X is an optional cleavage site and S1 and S2 are polynucleotide sequences which permit amplification of a template nucleic acid molecule comprising the target double-stranded polynucleotide.

The mixture of primers will generally comprise substantially equal amounts the forward and reverse primers.

L represents a linker which may be included but is not strictly necessary. The linker may be a carbon-containing chain such as those of formula $(CH_2)_n$ wherein "n" is from 1 to about 1500, for example less than about 1000, particularly less than 100, e.g. from 2-50, particularly 5-25. However, a variety of other linkers may be employed with the only restriction placed on their structures being that the linkers are stable under conditions under which the polynucleotides are intended to be used subsequently, e.g. conditions used in DNA amplification and sequencing.

Linkers which do not consist of only carbon atoms may also be used. Such linkers include polyethylene glycol (PEG) having a general formula of $(CH_2\text{-}CH_2\text{-}O)_m$, wherein m is from about 1 to 600, particularly less than about 500.

Linkers formed primarily from chains of carbon atoms and from PEG may be modified so as to contain functional groups which interrupt the chains. Examples of such groups include ketones, esters, amines, amides, ethers, thioethers, sulfoxides, sulfones. Separately or in combination with the presence of such functional groups may be employed alkene, alkyne, aromatic or heteroaromatic moieties, or cyclic aliphatic moieties (e.g. cyclohexyl). Cyclohexyl or phenyl rings may, for example, be connected to a PEG or $(CH_2)_n$ chain through their 1- and 4-positions.

As an alternative to the linkers described above, which are primarily based on linear chains of saturated carbon atoms, optionally interrupted with unsaturated carbon atoms or heteroatoms, other linkers may be envisaged which are based on nucleic acids or monosaccharide units (e.g. dextrose). It is also within the scope of this invention to utilise peptides as linkers.

In a further embodiment linker may comprise one or more nucleotides which form part of the amplification primer but which do not participate in any reaction carried out on or with the primer (e.g. a hybridisation or amplification reaction). Such nucleotides may also be referred to herein as "spacer" polynucleotides. Typically from 1 to 20, more particularly from 1 to 15 or from 1 to 10, and more particularly 2, 3, 4, 5, 6, 7, 8, 9 or 10 spacer nucleotides may be included. Most particularly the primer will include 10 spacer nucleotides. PolyT spacers may be used, although other nucleotides and combinations thereof can also be used. In one particular embodiment the primer may include 10T spacer nucleotides.

The one or more spacer nucleotides function to space the portion of the primer required to hybridise to a target and direct amplification, away from the site of attachment to the solid support (i.e. S1 or S2). The inclusion of spacer nucleotides at the 5' end can markedly improve the performance of hybridisation of complementary polynucleotides to region S1 or S2. In a particular embodiment the polynucleotide will include 10T spacer nucleotides and a 5' phosphorothioate group for attachment to the solid support, although other attachment moieties may be used.

Sequences S1 and S2 in the forward and reverse primers are polynucleotide sequences which, in combination, direct amplification of a template by solid-phase bridging amplification reaction. The template to be amplified includes (when viewed as a single strand) at the 3' end a sequence capable of hybridising to sequence S1 in the forward primers and at the 5' end a sequence the complement of which is capable of hybridising to sequence S2 the reverse primer.

The precise nature of sequences S1 and S2 in the forward and reverse primer oligonucleotides is typically dependent on the nature of the template it is intended to amplify. S1 and S2 are capable of hybridising to cognate sequences on complementary strands of the template to be amplified. The term "hybridisation" encompasses sequence-specific binding between primer and template. Binding of a primer to its cognate sequence in the template can occur under typical conditions used for primer-template annealing in standard PCR. Typically hybridisation conditions are 5xSSC at 40°C, following an initial denaturation step. It is not essential for hybridisation that sequences S1 and S2 be exactly complementary to their cognate sequences in the template to be amplified.

S1 and S2 may be of different or identical sequence and will typically be around 20-30 nucleotides in length. The primers can include natural and non-natural DNA bases, also ribonucleotides or any combination thereof, and may also include non-natural backbone linkages such as disulphides or phosphorothioates.

Cleavage site X may fall within sequence S1 or S2, or if the linker L is itself a polynucleotide cleavage they may form part of linker region L. In other embodiments the cleavage site may be formed at the junction of sequences L and S1 or L and S2, or at the junction between moiety A and linker L (if present) or between moiety A and sequence S1 or S2 (if L not present).

Moiety A may be any chemical moiety which permits immobilisation of an oligonucleotide primer on a solid support. The surface of the solid support may itself be functionalised to permit attachment of the primers. Any suitable covalent or noncovalent attachment means may be used, of which many are known in the art.

By way of example, biotinylated albumins (BSA) can form a stable attachment of biotin groups by physisorption of the protein onto surfaces. Covalent modification can also be performed using silanes, which have been used to attach molecules to a solid support, usually a glass slide. By way of example, a mixture of tetraethoxysilane and triethoxy-bromoacetamidopropylsilane (e.g. in a ratio of 1:100) can be used to prepare functionalised glass slides which permit attachment of molecules nucleic acids including a thiophosphate or phosphorothioate functionality. Biotin molecules can be attached to surfaces using appropriately reactive species such as biotin-PEG-succinimidyl ester which reacts with an amino surface. A mixture of amplification primers may then be brought into contact with the functionalised solid support.

In alternative embodiments functionalised polyacrylamide hydrogels may be used to attach primers wherein moiety A is a sulfur-containing nucleophilic groups are used. Examples of appropriate sulfur nucleophile-containing polynucleotides are disclosed in Zhao et al (Nucleic Acids Research, 2001, 29(4), 955-959) and Pirrung et al (Langmuir, 2000, 16, 2185-2191) and include, for example, simple thiols, thiophosphates and thiophosphoramidates. Particular hydrogels are those formed from a mixture of

(i) a first comonomer which is acrylamide, methacrylamide, hydroxyethyl methacrylate or N-vinyl pyrrolidinone; and
(ii) a second comonomer which is a functionalised acrylamide or acrylate of formula (I):

$$H_2C=C(H)-C(=O)-A-B-C \qquad (I);$$

or a methacrylate or methacrylamide of formula (II): or

$$H_2C=C(CH_3)-C(=O)-A-B-C- \qquad (II)$$

(wherein:

A is NR or O, wherein R is hydrogen or an optionally substituted saturated hydrocarbyl group comprising 1 to 5 carbon atoms;
-B- is an optionally substituted alkylene biradical of formula $-(CH_2)_n-$ wherein n is an integer from 1 to 50; and wherein n = 2 or more, one or more optionally substituted ethylene biradicals $-CH_2CH_2-$ of said alkylene biradical may be independently replaced by ethenylene and ethynylene moieties; and wherein n=1 or more, one or more methylene biradicals $-CH_2-$ may be replaced independently with an optionally substituted mono- or polycyclic hydrocarbon biradical comprising from 4 to 50 carbon atoms, or a corresponding heteromonocyclic or heteropolycyclic biradical wherein at least 1 $CH_2$ or $CH_2$ is substituted by an oxygen sulfur or nitrogen atom or an NH group; and
C is a group for reaction with a compound to bind the compound covalently to the hydrogel) to form a polymerised product. A particular hydrogel is formed by co-polymerisation of acrylamide and N-(5- bromoacetamidylpentyl)acrylamide (BRAPA).

The term "solid support", as used herein, refers to the material to which the polynucleotides molecules are attached. Suitable solid supports are available commercially, and will be apparent to the skilled person. The supports can be manufactured from materials such as glass, ceramics, silica and silicon. Supports with a gold surface may also be used. The supports usually comprise a flat (planar) surface, or at least a structure in which the polynucleotides to be interrogated are in approximately the same plane. Alternatively, the solid support can be non-planar, e.g., a microbead. Any suitable size may be used. For example, the supports might be on the order of 1-10 cm in each direction.

For the grafting reaction to proceed a mixture of the amplification primers is applied to a (suitable functionalised) solid support under conditions which permit reaction between moiety A and the support. The result of the grafting reaction is a substantially even distribution of the primers over the solid support.

In certain embodiments the template to be amplified may be grafted onto the solid support together with the amplification primers in a single grafting reaction. This can be achieved by adding template molecules including moiety A at the 5' end to the mixture of primers to form a primer-template mixture. This mixture is then grafted onto the solid support in a single step. Amplification may then proceed using the immobilised template and primers in a reaction analogous to that described in WO 00/18957. The first step in such a reaction can be hybridisation between surface-bound templates and surface-bound amplification primers.

If the mixture of primers only is grafted onto the solid support and the template to be amplified is present in free solution, the amplification reaction may proceed substantially as described in WO 98/44151. Briefly, following attachment of the primers the solid support is contacted with the template to be amplified under conditions which permit hybridisation

between the template and the immobilised primers. The template is usually added in free solution under suitable hybridisation conditions, which will be apparent to the skilled reader. Typically hybridisation conditions are, for example, 5xSSC at 40°C, following an initial denaturation step. Solid-phase amplification can then proceed, the first step of the amplification being a primer extension step in which nucleotides are added to the 3' end of the immobilised primer hybridised to the template to produce a fully extended complementary strand. This complementary strand will thus include at its 3' end a sequence which is capable of binding to the second primer molecule immobilised on the solid support. Further rounds of extension, denaturation and hybridisation to immobilised primer lead to the formation of clusters or colonies of template molecules bound to the solid support.

Sequences S1 and S2 in the amplification primers may be specific for a particular target nucleic acid that it is desired to amplify, but in other embodiments sequences S1 and S2 may be "universal" primer sequences which enable amplification of any target nucleic acid of known or unknown sequence which has been modified to enable amplification with the universal primers.

Suitable templates to be amplified with universal primers may be prepared by the methods described herein. The target molecules themselves may be any double-stranded molecules it is desired to sequence (e.g. random fragments of human genomic DNA). The target sequences are treated to both remove the central region whilst keep the two ends in communication with each other, and to attach known sequence regions onto the ends of each target fragment. The known ends come from the vector sequence attached to each target fragment, and enable amplification of these molecules on a solid support to form clusters using forward and reverse primers having the general structure described above, wherein sequences S1 and S2 are universal primer sequences. The resultant construct comprising a target nucleic acid sequence flanked by known ends may be referred to herein as a "template nucleic acid construct".

The known ends of the templates contain sequences which permit nucleic acid amplification using the amplification primer molecules immobilised on the solid support. These sequences in the adaptors may be referred to herein as "primer binding sequences". In order to act as a template for nucleic acid amplification, a single strand of the template construct can contain a sequence which is complementary to sequence S1 in the forward amplification primers (such that the forward primer molecule can bind and prime synthesis of a complementary strand) and a sequence which corresponds to sequence S2 in the reverse amplification primer molecules (such that the reverse primer molecule can bind to the complementary strand). The sequences in the adaptors which permit hybridisation to primer molecules will typically be around 20-30 nucleotides in length, although the invention is not limited to sequences of this length.

The precise identity of sequences S1 and S2 in the amplification primers, and hence the cognate sequences in the adaptors, can be any of a variety of possibilities as long as the primer molecules are able to interact with the amplification sequences in order to direct bridging amplification. The criteria for design of primers are generally well known to those of ordinary skill in the art.

Solid-phase amplification by either the method analogous to that of WO 98/44151 or that of WO 00/18957 will result in production of an array of colonies of "bridged" amplification products. Both strands of the amplification product will be immobilised on the solid support at or near the 5' end, this attachment being derived from the original attachment of the amplification primers. Typically the amplification products within each colony will be derived from amplification of a single target molecule.

The utility of the sequencing method of the invention is not limited to sequencing of templates produced by an amplification reaction. The method may be applied to sequencing of any template immobilised on a support by any other means amenable to repeated cycles of hybridisation and sequencing.

In order to generate paired read information from each cluster, it is necessary to generate a read from each strand of the immobilised cluster. Methods for generating paired reads from each cluster are fully detailed in co-pending applications US 11/973,321, WO07010252, PCT/GB2007/000447, US60/850210 and US60/898910. Some representative methods of obtaining two reads from each cluster are shown in figures 3-6.

The first and second regions for sequence determination are at both ends of complementary strands of the double-stranded polynucleotide template, which are referred to herein respectively as first and second template strands.

The starting point for particular embodiments of the methods of the invention is the provision of a plurality of template polynucleotide duplexes immobilised on a solid support. Each of the duplexes within a particular cluster comprises the same double-stranded target region to be sequenced. The duplexes are each formed from complementary first and second template strands which are linked to the solid support at or near to their 5' ends. Typically, the template polynucleotide duplexes will be provided in the form of a clustered array.

When referring to immobilisation or attachment of molecules (e.g. nucleic acids) to a solid support, the terms "immobilised" and "attached" are used interchangeably herein and both terms are intended to encompass direct or indirect, covalent or noncovalent attachment, unless indicated otherwise, either explicitly or by context. In certain embodiments of the invention covalent attachment may be preferred, but generally all that is required is that the molecules (e.g. nucleic acids) remain immobilised or attached to the support under the conditions in which it is intended to use the support, for example in applications requiring nucleic acid amplification and/or sequencing.

Sequence data can be obtained from both ends of the immobilised duplex by a method wherein the duplex is

treated to free a 3'-hydroxyl moiety that can be used an extension primer. The extension primer can then be used to read the first sequence from one strand of the template. After the first read, the strand can be extended to fully copy all the bases up to the end of the first strand. This second copy remains attached to the surface at the 5'-end. If the first strand is removed from the surface, the sequence of the second strand can be read. This gives a sequence read from both ends of the original fragment.

Alternatively, sequence data can be obtained from both ends of a template duplex by obtaining a sequence read from one strand of the template, copying the strand using immobilised primers, releasing the first strand and sequencing the second, copied strand. The first sequencing primer can be immobilised, or in solution.

Methods of generating a free 3'-hydroxyl in only one strand of a duplex immobilised at both ends include either treatment with a nicking enzyme, or chemical treatment to remove a nucleotide. Suitable nicking enzymes are well known in the art, and would preferably cut at a site that is 3'-remote to their binding site to avoid having to sequence bases that derive from the known nicked site. The nicking enzyme typically cuts only one of the immobilised strands, at the end closest to the surface. See Figure 3 for schematic of paired end sequencing methodology utilising a nicking enzyme. Examples of suitable restriction enzymes would include Nt.BstNBI and Nt.AlwI, which have no bases of defined sequence beyond the released 3'-hydroxyl.

Methods of removing nucleotides from the duplex involve the design of the template such that the base immediately adjacent to the unknown target region can be removed to make an abasic site. An "abasic site" is defined as a nucleotide position in a polynucleotide chain from which the base component has been removed. Abasic sites can occur naturally in DNA under physiological conditions by hydrolysis of nucleotide residues, but may also be formed chemically under artificial conditions or by the action of enzymes. Once formed, abasic sites may be cleaved (e.g. by treatment with an endonuclease or other single-stranded cleaving enzyme, exposure to heat or alkali), providing a means for site-specific cleavage of a polynucleotide strand.

In a preferred but non-limiting embodiment an abasic site may be created at a pre-determined position on one strand of a template polynucleotide duplex and then cleaved by first incorporating deoxyuridine (U) at a pre-determined cleavage site in one strand of the template polynucleotide duplex. This can be achieved, for example, by including U in one of the primers used for preparation of the template polynucleotide duplex by solid-phase amplification. The enzyme uracil DNA glycosylase (UDG) may then be used to remove the uracil base, generating an abasic site on one strand. The polynucleotide strand including the abasic site may then be cleaved at the abasic site by treatment with endonuclease (e.g EndoIV endonuclease, AP lyase, FPG glycosylase/AP lyase, EndoVIII glycosylase/AP lyase), heat or alkali. In a particular embodiment, the USER reagent available from New Englad Biolabs (M5505S) is used for the creation of a single nucleotide gap at a uracil base in a duplex strand. Treatment with endonuclease enzymes gives rise to a 3'-phosphate moiety at the cleavage site, which can be removed with a suitable phosphatase such as alkaline phosphatase. See Figure 4 for a schematic of a paired-end sequencing reaction that utilizes USER linearization resynthesis.

Abasic sites may also be generated at non-natural/modified deoxyribonucleotides other than deoxyuridine and cleaved in an analogous manner by treatment with endonuclease, heat or alkali. For example, 8-oxo-guanine can be converted to an abasic site by exposure to FPG glycosylase. Deoxyinosine can be converted to an abasic site by exposure to AlkA glycosylase. The abasic sites thus generated may then be cleaved, typically by treatment with a suitable endonuclease (e.g. EndoIV, AP lyase). If the non-natural/modified nucleotide is to be incorporated into an amplification primer for use in solid-phase amplification, then the non-natural/modified nucleotide should be capable of being copied by the polymerase used for the amplification reaction.

In one embodiment, the molecules to be cleaved may be exposed to a mixture containing the appropriate glycosylase and one or more suitable endonucleases. In such mixtures the glycosylase and the endonuclease will typically be present in an activity ratio of at least about 2:1.

This method of cleavage has particular advantages in relation to the creation of templates for nucleic acid sequencing. In particular, cleavage of an abasic site generated by treatment with a reagent such as USER automatically releases a free 3' phosphate group on the cleaved strand which after phosphatase treatment can provide an initiation point for sequencing a region of the complementary strand. Moreover, if the initial double-stranded nucleic acid contains only one cleavable (e.g. uracil) base on one strand then a single "nick" can be generated at a unique position in this strand of the duplex. Since the cleavage reaction requires a residue, e.g. deoxyuridine, which does not occur naturally in DNA, but is otherwise independent of sequence context, if only one non-natural base is included there is no possibility of glycosylase-mediated cleavage occurring elsewhere at unwanted positions in the duplex. In contrast, were the double-stranded nucleic acid to be cleaved with a "nicking" endonuclease that recognises a specific sequence, there is a possibility that the enzyme may create nicks at "other" sites in the duplex (in addition to the desired cleavage site) if these possess the correct recognition sequence. This could present a problem if nicks are created in the strand it is intended to copy, rather than the strand that will be fully or partially removed, and is a particular risk if the target portion of the double-stranded nucleic acid molecule is of unknown sequence. These limitations may be overcome by preparing a sample with the recognition site for two different nicking enzymes. If both preparations are processed separately using the method, the regions of the genome cleaved by one nicking enzyme should be represented in the other preparation.

The fact that there is no requirement for the non-natural (e.g. uracil) residue to be located in a detailed sequence context in order to provide a site for cleavage using this approach is itself advantageous. In particular, if the cleavage site is to be incorporated into an amplification primer to be used in the production of a clustered array by solid-phase amplification, it is only necessary to replace one natural nucleotide (e.g. T) in the primer with a non-natural nucleotide (e.g. U) in order to enable cleavage. There is no need to engineer the primer to include a restriction enzyme recognition sequence of several nucleotides in length. Oligonucleotide primers including U nucleotides, and other non-natural nucleotides, such as those listed above, can easily be prepared using conventional techniques and apparatus for chemical synthesis of oligonucleotides.

Another advantage gained by cleavage of abasic sites in a double-stranded molecule generated by action of UDG on uracil is that the first base incorporated in a "sequencing-by-synthesis" reaction initiating at the free 3' hydroxyl group formed by cleavage at such a site will always be T. Hence, if the template polynucleotide duplex forms part of a clustered array comprised of many such molecules, all of which are cleaved in this manner to produce sequencing templates, then the first base universally incorporated across the whole array will be T. This can provide a sequence-independent assay for individual cluster intensity at the start of a sequencing "run".

In particular cases, it may be advantageous to perform a blocking treatment with a dideoxynucleotide triphosphate and a polymerase and/or terminal transferase. After a solid phase amplification, there remain on the surface a large number of unused amplification primers, in addition to the free 3'-ends of each of the template strands. Treatment with such a blocked nucleotide ensures these free 3'-OH functional groups are unavailable for extension during any subsequent polymerase steps. In methods where 3'-hydroxyl groups are created with chemical treatment or restriction endonucleases, it is advantageous to block any residual 3'-hydroxyl groups before the desired 3'-hydroxyl groups are created. In the case of treatment with the USER reagent, as a phosphate group is released, the blocking step can be performed either before or after the USER treatment, as the phosphate group will act as a protecting group to prevent blocking of the desired 3'-hydroxyl moieties. The phosphate group can be removed after the blocking step has been performed.

The act of restriction enzyme treatment or abasic site generation and cleavage results in a free 5'-end on the strand that is no longer immobilised to the surface. This strand can be completely removed from the surface by treatment with a 5'-3' exonuclease, such as lambda or T7 exonuclease. Such a treatment means that the template strand is single stranded, and available for subsequent copying. If the polymerase that is used to extend the 3'-hydroxyl group has a strand displacing activity, then the 5'-3' exonuclease treatment may not be necessary.

In the first embodiment of the invention, it is advantageous to extend the free 3'-hydroxyl primer with a plurality of bases complementary to the template prior to initiating sequencing. This both raises the melting temperature of the immobilised duplex, and helps prevent the template strand from re-hybridising to other immobilised primers during sequencing, which gives rises to phasing problems within the cluster. The ligation step is carried out after the phosphatase step has removed the phosphate group from the immobilised primer. Addition of 20-30 bases of sequence can be performed by a ligation reaction with a 5'-phosphate modified primer hydridised adjacent to the free 3'-hydroxyl. A ligase, such as T4 DNA ligase can be used to seal the gap. In the case of USER treatment which removes the U nucleotide, the 5'-base of the primer will be T that replaces the excised U. For the hybridisation step to be carried out efficiently, the 5'-non immobilised strand can be removed by 5'-3' exonucleolysis treatment as described above. Such immobilised, extended primers with a free 3'-hydroxyl are described as 5'-anchored, or immobilised primers, and generation of such extended primers is only one part of the steps involved in treating the plurality of double stranded template polynucleotides such that the first template strand is hybridised to a primer that is immobilised on the solid support at its 5'-end.

The free 3'-hydroxyl group on the 5'-anchored primer can be used to initiate rounds of sequencing to determine the sequence of the bases in the hybridised template. Sequencing can be carried out using any suitable "sequencing-by-synthesis" technique, wherein nucleotides are added successively to the free 3' hydroxyl group, resulting in synthesis of a polynucleotide chain in the 5' to 3' direction. The nature of the nucleotide added is preferably determined after each addition.

The use of USER according to the present invention produces a linearised first template strand and a lawn of primers left as abasic sites with phosphate moieties. Without further phosphatase or endonuclease treatment, the immobilised primer lawn is not suitable for polymerase extension. A sequencing primer in solution can be used to initiate a sequencing read for the first template strand, as shown in figure 5. If the phosphate groups (and abasic sites) are removed from the lawn of primers, then the first strands, once the first extended sequencing primer has been removed, can be hybridised to the primer lawn and subjected to one or more cycles of extension/denaturation and re-hybridisation. This re-generates the template duplexes where both strands are immobilised. The first strand can be removed by a suitable orthogonal linearisation treatment step, such as diol cleavage or removal of an 8-oxo-G residue, and after denaturation of the first template strand, a second sequencing primer can be hybridised to the second template strand, and the second template strand sequenced. This orthogonal linearisation strategy allows reads from both ends of the template.

The sequencing reaction can be carried out using the immobilised primers. The immobilised sequencing primer

can be denatured, and the first template strand used to re-initiate further rounds of extension or amplification as above. Again this re-generates the template duplexes where both strands are immobilised. The first strand can be removed by a suitable orthogonal linearisation treatment step, such as diol cleavage or removal of an 8-oxo-G residue, and after denaturation of the first template strand, a second sequencing primer can be hybridised to the second template strand, and the second template strand sequenced. This orthogonal linearisation strategy also allows reads from both ends of the template.

Both of the methods detailed above allow for cluster repopulation between the first and second reads. This is beneficial to increase the level of signal obtained for the second read of the cluster. Any method that results in both amplification primers being retained on the surface during the first sequencing read is within the scope of the invention, although it is advantageous if the primers can be treated, as in the case of USER, to make the 3'-hydroxyl unavailable for primer extension during the sequencing reaction. An alternative method of blocking the unextended grafting primers would be to use a nucleoside comprising an unextendable 3' group. Treating the surface with such a nucleotide and terminal transferase would block the surface to further extension. After the first sequencing run, the nucleotides could be deprotected to allow further cycles of copying the template strand. If the nucleotides carry a dideoxy modification, this can be removed using an exonuclease, or a polymerase with exonuclease activity. Thus the clusters could be made with two grafted primers as described, the unused primers blocked during the first sequencing reaction, and then deblocked to allow further copying of the template strands.

A portion of the amplification primers may be attached to the surface with a modification blocking the 3'hydroxyl from extension in the amplification cycles, as shown in figure 6. This in effect means that the surface is treated with three amplification primers rather than the usual two. The second and third amplification primers will be of identical sequence, but the third one will not be susceptible to the conditions used to remove the second primer during the linearisation process, and will contain a 3'- blocking moiety. The blocking moiety may take the form of a chemical block, such as an azidomethyl group that can be removed with a phosphine reagent, an enzymatically removable group such as a phosphate group that can be removed with a phosphatase, or it may be in the form of a nucleoside group that can be removed using 3'-5' exonucleolysis. Such nucleoside modifications include abasic sites, that can be removed as described, or 2', 3' dideoxy nucleotides that can be removed by a polymerase with exonuclease activity. Treatment of the surface after the first sequencing run is completed to deblock the primers and thus allow the remaining first strand to hybridise to the deprotected primers and recopy the already sequenced strands. A second sequencing read can be obtained by removing the first strand, hybridising a sequencing primer and reading the newly synthesised second strand.

The two reads generated by the methods described herein may come from two regions of the primary target separated by a few hundred basepairs, or may come from two regions of the primary target separated by many thousands of basepairs. If the fragments are not selected, then the ratio of end fragments to central fragments will be defined by the sizes of the respective fragments. For example, if a primary nucleic acid sequence is fragmented to an average size of 2000 basepairs, which is self-ligated to form circles, which are then fragmented to an average size of 200 base pairs, then 10% of the fragments derived from the circle will span the ends of the 2000 basepairs linear fragments. If the fragments are initially 5000 base pairs on average, then the ratio of ends/central fragments will be reduced to 4%. These long range reads are very useful for annotating the fragments and for de-novo assembly. Using methods described herein for selecting the ends of the fragments, it is possible to generate a library where at least 50% of the fragments span the two ends of a fragment greater than 1kb in length. Selection using biotinylation of the ends of the fragments followed by streptavidin pull out may give to rise to a library where >90%, or even >99% of the sequence reads are spaced out over distances greater than 1kb. Data shown in figure 8 shows that it is possible to obtain a high level of reads that derive from the ends of fragments longer than 2kb. However, there may also be some residual fragments that only give two reads separated by the size of the sample used to make clusters, which can be seen in the spikes on the left hand side of the traces for the libraries. These residual shorter reads are to be expected where no selection of the end regions is employed. In the case where end selection is used, the residual shorter fragments derive either from linear strands that are not digested when the circular constructs are treated, or derive from non labelled (biotinylated) fragments of the circles that are carried through the selection step.

The invention will be further understood with reference to the following experimental examples:

## Examples

### Example 1: Library construction

Libraries were made, using purified human BAC DNA (140kb human chromosome 6 insert cloned into a pTARBAC vector). The DNA was first fragmented by nebulization. The DNA was end repaired using biotinylated dNTPs so that the ends of the DNA were blunt-ended, 5' phosphorylated and 3' biotinylated. The DNA was sized selected on an agarose gel and the purified DNA ligated, to promote self-ligation, to form circular DNA. Any linear DNA was removed from the ligation reaction product using Plasmid Safe DNase. The circular DNA was prepared for ligation to forked

adaptors by: fragmentation of the DNA by nebulization, end repair of the DNA ends with natural dNTPs to make them blunt-ended and 5' phosphorylated, then the addition of a single 'A' nucleotide onto the 3' ends of the DNA fragments. The ligation reaction was performed with the prepared fragmented DNA and adaptors preformed by annealing 'Oligo A' and 'Oligo B' (sequences given below). The ligation reaction product was purified on streptavidin beads, to isolate fragments labelled with biotin. The captured DNA was amplified from the bead by PCR to selectively amplify ligated products that contained genomic DNA with adaptor at both ends of the fragments. Finally the PCR was gel purified to select the library fragment sizes.

**Materials and Methods**

1) Nebulization

Materials:

- 0.08 μg/μl Human BAC DNA (140k human chromosome 6 insert cloned into a pTARBAC vector)
- Nebulization Buffer (53.1 ml glycerol, 42.1 ml water, 3.7 ml 1 M Tris HCl pH 7.5, 1.1 ml 0.5 M EDTA)
- Nebulizers (Invitrogen, K7025-05)
- PCR purification kit columns (Qiagen, 28104)

Procedure:

Mixed 50 μl (4 μg) of BAC DNA with 700 μl of nebulization buffer. The chilled DNA solution was fragmented in a nebulizer on ice for 20 seconds under 10 pounds per square inch (psi) of pressure. This was repeated once (total of 8 μg of BAC nebulized) and the two nebulizations pooled. The recovered, pooled volume was purified with a Qiagen PCR purification kit column and eluted in 30 μl of EB.

2) End-repair

Materials:

- Nebulized DNA (from 1.)
- Water
- T4 DNA ligase buffer with 10mM ATP (10x) (NEB, B0202S)
- Biotin-11-dATP (1mM) (Perkin Elmer, NEL540001EA)
- Biotin-11-dUTP (1mM) (Perkin Elmer, NEL539001EA)
- Biotin-11-dGTP (1mM) (Perkin Elmer, NEL541)
- Biotin-11-dCTP (3mM) (Perkin Elmer, NEL538B001EA)
- T4 DNA Polymerase (3U/μl) (NEB, M0203L)
- *E. coli* DNA Pol I large fragment (Klenow) (5U/μl) (NEB, M0210S)
- T4 polynucleotide kinase (10U/μl) (NEB, M0201L)
- PCR purification kit columns (Qiagen, 28104)

Procedure:

| | |
|---|---|
| Nebulized DNA | 30 μl |
| Water | 36 μl |
| T4 DNA ligase buffer with 10mM ATP | 12 μl |
| Biotin-11-dATP | 9 μl |
| Biotin-11-dUTP | 9 μl |
| Biotin-11-dGTP | 9 μl |
| Biotin-11-dCTP | 1.8 μl |

(continued)

| | |
|---|---|
| T4 DNA pol | 6 μl |
| Klenow DNA pol | 1.2 μl |
| T4 PNK | 6 μl |
| | 120 μl total |

The reaction was incubated for 30 mins at 20 °C. The DNA was purified on a Qiagen column, eluting in 30 μl EB.

3) Gel purification

Materials:

- End repaired DNA (from 2.)
- Agarose (Biorad, 161-3101)
- TAE (50x)
- Distilled water
- Ethidium bromide (Sigma, E1510)
- Loading buffer (4x) (50 mM Tris pH8, 40 mM EDTA, 40% w/v sucrose)
- 1kb Plus DNA ladder (Invitrogen, 10787-026)
- Gel trays and tank. Electrophoresis unit
- Dark reader transilluminator (Clare Chemical Research, D195M)
- MinElute kit column (Qiagen, 28004)

Procedure:

The entire sample from the end repair reaction was loaded into one lane of a 1% TAE agarose gel containing ethidium bromide and run at 120V for 75 mins. The gel was then viewed on a 'White-light' box and fragments of 3-4kb were excised. The DNA from each gel slice was purified with a minelute column, eluting in 15μl EB.

4) Ligation

Materials:

- Gel purified DNA (from 3.)
- Quick ligase buffer (2x) (NEB, B2200S)
- Quick Ligase (1U/μl) (NEB, M2200L)
- PCR purification kit columns (Qiagen, 28104)
- Water

Procedure:

| | |
|---|---|
| Gel purified DNA | 15 μl |
| Quick ligase buffer | 50 μl |
| Water | 25 μl |
| Quick Ligase | 10 μl |
| | 100 μl total |

The reaction was incubated for 15 min at 20 °C, then the DNA purified on a Qiagen column, eluting in 30 μl EB.

5) Plasmid Safe

Materials:

- Ligated DNA (from 4.)
- Plasmid Safe buffer (10x) (Epicentre, E3105K)
- ATP (25mM) (Epicentre, E3105K)
- Plasmid Safe DNase (10U/ul) (Epicentre, E3105K), diluted 1/5 to 2U/ul in 1X buffer
- Water
- PCR purification kit columns (Qiagen, 28104)

Procedure:

| | |
|---|---|
| Ligated DNA | 30 μl |
| Plasmid safe buffer | 4 μl |
| ATP | 1.7 μl |
| 1/5 diluted plasmid safe DNase | 2 μl |
| Water | 4.3 μl |
| | 42 μl |

The reaction was incubated for 20 mins at 37 °C and then the enzyme was heat inactivated for 30 mins at 70 °C. The DNA was purified on a Qiagen column, eluting in 50 μl EB.

6) Nebulization

Materials:

- Plasmid safe DNase treated DNA (from 5.)
- Nebulization Buffer (53.1 ml glycerol, 42.1 ml water, 3.7 ml 1 M Tris HCl pH 7.5, 1.1 ml 0.5 M EDTA)
- Nebulizers (Invitrogen, K7025-05)
- PCR purification kit columns (Qiagen, 28104)

Procedure:

Mixed 50 μl plasmid safe DNase treated DNA with 700 μl of nebulization buffer. Chilled DNA solution was fragmented in a nebulizer on ice for 6 minutes under 32 pounds per square inch (psi) of pressure. The recovered volume was purified with a Qiagen PCR purification kit column and eluted in 30 μl of EB.

7) End-repair

Materials:

- Nebulized DNA (from 6.)
- Water
- T4 DNA ligase buffer with 10mM ATP (10x) (NEB, B0202S)
- dNTPs mix (10mM each) (NEB, N0447S)
- T4 DNA Polymerase (3U/μl) (NEB, M0203L)
- *E. coli* DNA Pol I large fragment (Klenow) (5U/μl) (NEB, M0210S)
- T4 polynucleotide kinase (10U/μl) (NEB, M0201L)

- PCR purification kit columns (Qiagen, 28104)

Procedure:

| | |
|---|---|
| Nebulized DNA | 30 μl |
| Water | 45 μl |
| T4 DNA ligase buffer with 10 mM ATP | 10 μl |
| dNTPs | 4 μl |
| T4 DNA pol | 5 μl |
| Klenow DNA pol | 1 μl |
| T4 PNK | 5 μl |
| | 100 μl total |

The reaction was incubated for 30 mins at 20 °C. The DNA was purified on a Qiagen column, eluting in 30 μl EB.

8) A-tailing Reaction

Materials:

- End repaired DNA (from 7.)
- Water
- NEB buffer 2 (10x) (NEB, B7002S)
- dATP (1mM) (Amersham-Pharmacia, 272050)
- Klenow fragment (3' to 5' exo minus) (5U/μl) (NEB, M0212B)
- Hot block or PCR machine
- MinElute PCR purification kit column (Qiagen, 28004)

Procedure:

| | |
|---|---|
| End repaired DNA | 30 μl |
| Water | 2 μl |
| NEB buffer 2 | 5 μl |
| dATP | 10 μl |
| Klenow fragment (3' to 5' exo minus) | 3 μl |
| | 50 μl total |

The reaction was incubated for 30 min at 37 °C, then the DNA purified on a Qiagen MinElute column, eluting in 10 μl EB.

9) Annealed Adaptors

Materials:

- Oligo A: 5' ACACTCTTTCCCTACACGACGCTCTTCCGATCxT (x = phosphorothioate bond)
- Oligo B: 5'Phosphate GATCGGAAGAGCGGTTCAGCAGGAATGCCGAG
- 50mM Tris/50mM NaCl pH7.0
- PCR machine

Procedure:

The oligos were mixed together to a final concentration of 15 μM each, in 10 mM Tris/10 mM NaCl pH 7.0. The adaptor strands were annealed in a PCR machine programmed as follows:

Ramp at 0.5 °C/sec to 97.5 °C
Hold at 97.5 °C for 150 sec

Then a step of 97.5 °C for 2 sec with a temperature drop of 0.1 °C/cycle for 775 cycles.

10) Ligation

Materials:

- A-tailed DNA (from 8.)
- Quick ligase buffer (2x) (NEB, B2200S)
- Water
- Annealed adaptor (15 μM) (from 9.), diluted 1/40 to 0.375 μM in water
- Quick Ligase (1U/μl) (NEB, M2200L)
- PCR purification kit columns (Qiagen, 28104)

Procedure:

| | |
|---|---|
| A-tailed genomic DNA | 10 μl |
| Quick ligase buffer | 25 μl |
| Water | 8.8 μl |
| 1/40 diluted annealed adaptor | 1.2 μl |
| Quick Ligase | 5 μl |
| | 50 μl total |

The reaction was incubated for 15 min at 20 °C, then the DNA purified on a Qiagen column, eluting in 30 μl EB.

11) Purification on Streptavidin Beads

Materials:

- Ligated DNA (from 10.)
- Streptavidin M280 magnetic beads (Dynal, 112.05)
- B&W buffer (2x) (10 mM Tris-HCl pH 7.5, 1 mM EDTA, 2 M NaCl)
- B&W buffer + 0.1% TWEEN® (1x) (5 mM Tris-HCl pH 7.5, 0.5 mM EDTA, 1 M NaCl, 0.1% TWEEN®)
- EB buffer (Qiagen)

Procedure:

20 μl of resuspended streptavidin magnetic beads were aliquoted into a microfuge tube. The tube was placed in a magnet for 1 min. The supernatant was removed with a pipette while the tube remained in the magnet. The beads were washed twice in 50 μl of 2x B&W buffer, by pipetting up and down. The magnet was used to separate the beads from the wash supernatant.

The washed beads were resuspended in 30 μl of 2X B&W buffer. 30 μl of ligated DNA from step 10 was incubated with the beads for 15 mins at room temperature. The beads were resuspended every 2 mins by gentle mixing. The supernatant was removed and the beads were washed four times in 200 μl of 1x B&W buffer + 0.1% TWEEN®followed

by two washes in 200 μl of Qiagen EB buffer. The final wash solution was removed from the beads.

**12) Exonuclease I Treatment of PCR Primers**

Materials:

- Exonuclease I (E. coli) (20 U/μl) (NEB, M0293S)
- Exonuclease I Reaction Buffer (10x) (NEB, M0293S)
- Water
- DNA Primers with a phosphorothioate at the n-1 position
- P6 Bio-Rad columns (Bio-Rad, 732-6221)

Procedure:

DNA Primers with a phosphorothioate at the n-1 position (5 x 85 μl of each Primer (approx 25 μM)) were aliquoted into microfuge tubes. 10 μl of 10 x Exonuclease I Reaction Buffer and 5 μl of Exonuclease I was added to each tube. Each Eppendorf tube was placed in a rack and stored in an oven set at 37 °C for 16 hours. After 16 hr, the tubes were placed on a hotblock set at 80 °C for 2 minutes. Then the solutions from the eppendorf tubes were passed through P6 Bio Rad columns and spun in a centrifuge at 2000 rpm for 2 minutes. An extra 20 μl of $H_20$ was added and the columns respun. The filtered solutions were placed into a speedvac and evaporated until each was at 20 μl, and the fractions combined. The pooled fractions were injected into a reverse phase HPLC system, and the main peak was collected. The collected fractions were evaporated to dryness in a speedvac, 50 μl of water was added and the fraction was subjected again to evaporation to dryness. The resulting pellets were dissolved in 50 μl of water, pooled and the UV measurement taken to determine the concentration of the oligonucleotide.

**13) PCR**

Materials:

- Washed streptavidin beads (from 11.)
- Water
- Phusion master mix (2x) (NEB, F-531L)
- Exonuclease treated PCR primer 1 (25uM): 5' AATGATACGGCGACCACCGAGATCTACACTCTTTCCCTACACGACGCTCTTCCGATCxT 3', where x= phosphorothioate bond (from 12.)
- Exonuclease treated PCR primer 2 (25uM): 5' CAAGCAGAAGACGGCATACGAGATCGGTCTCGGCATTCCTGCTGAACCGCTCTTCCGATCxT, where x= phosphorothioate bond (from 12.)
- PCR machine
- PCR purification kit columns (Qiagen, #28104)

Procedure:

The following PCR mix was added to the washed streptavidin beads

| | |
|---|---|
| Phusion mastermix | 25 μl |
| PCR primer 1 | 1 μl |
| PCR primer 2 | 1 μl |
| Water | 23 μl |
| | 50 μl total |

Thermocycling was carried out in a PCR machine under the following conditions:

- 30 secs @ 98 °C
- [10 sec@ 98 °C, 30 sec @ 65 °C, 30 sec @ 72 °C] 18 cycles

- 5 min @ 72 °C
- Hold @ 4 °C

PCR products were purified on a Qiagen column, eluting in 30 μl EB.

14) Gel Purification

Materials:

- PCR (from 13.)
- Agarose (Biorad, 161-3107)
- TAE (50x)
- Distilled water
- Ethidium bromide (Sigma, E1510)
- Loading buffer (4x) (50 mM Tris pH8, 40 mM EDTA, 40% w/v sucrose)
- Low molecular weight ladder (NEB, N3233L)
- Gel trays and tank. Electrophoresis unit
- Dark reader transilluminator (Clare Chemical Research, D195M)
- MinElute kit column (Qiagen, 28004)

Procedure:

The entire sample from the PCR was loaded into one lane of a 2% TAE agarose gel containing ethidium bromide and run at 120V for 60 min. The gel was then viewed on a 'White-light' box and fragments from 300bp to 400bp excised and purified with a minelute column, eluting in 15 μl EB.

**Validation of Libraries by Conventional Sanger Sequencing**

4 μl of the libraries was cloned into a plasmid vector (Zero Blunt TOPO PCR cloning kit, Invitrogen #K2800-20) and plated out on agar, according to the manufacturer's instructions. Colonies were picked, mini-prepped and the cloned fragments sequenced by conventional Sanger sequencing.

Results are shown in figure 7. The table shows the position of the two paired regions on the BAC and the distance between them. Of the thirteen clones sequenced, 10 clones contained two paired regions which were 3-4kb apart, as expected. One clone had two paired regions that were a larger distance apart. Two other clones contained single regions.

Preparation and sequencing of clusters

The following experimental details describe an exemplary complete exposition of embodiments of the invention as described above. Preparation and sequencing of clusters are described in copending patents WO06064199 and WO07010251, whose protocols are included herein by reference in their entirety.

Cluster preparation using the template library

Example 2): Acrylamide coating of glass chips

The solid supports used are typically 8-channel glass chips such as those provided by Silex Microsystems (Sweden). However, the experimental conditions and procedures are readily applicable to other solid supports.

Chips were washed as follows: neat Decon for 30 minutes, MilliQ $H_2O$ for 30 minutes, NaOH 1N for 15 minutes, MilliQ $H_2O$ for 30 minutes, HCl 0.1N for 15 minutes, MilliQ $H_2O$ for 30 minutes.

Polymer solution preparation:

For 10 ml of 2% polymerisation mix.

- 10 ml of 2% solution of acrylamide in MilliQ $H_2O$

- 165 μl of a 100mg/ml N-(5-bromoacetamidylpentyl) acrylamide (BRAPA) solution in DMF (23.5 mg in 235μl DMF)
- 11.5 μl of TEMED
- 100 μl of a 50 mg/ml solution of potassium persulfate in milliQ $H_2O$ (20mg in 400μl $H_2O$)

The 10 ml solution of acrylamide was first degassed with argon for 15 minutes. The solutions of BRAPA, TEMED and potassium persulfate were successively added to the acrylamide solution. The mixture was then quickly vortexed and immediately used. Polymerization was then carried out for 1h 30 minutes at RT. Afterwards the channels were washed with MilliQ $H_2O$ for 30 minutes and filled with 0.1 M potassium phosphate buffer for storage until required.

Example 3) Synthesis of *N*-(5-bromoacetamidylpentyl) acrylamide (BRAPA) (1)

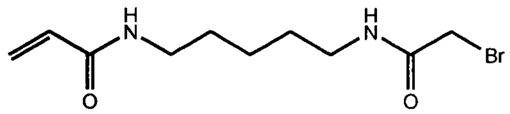

(**1**)

*N*-Boc-1,5-diaminopentane toluene sulfonic acid was obtained from Novabiochem. The bromoacetyl chloride and acryloyl chloride were obtained from Fluka. All other reagents were Aldrich products.

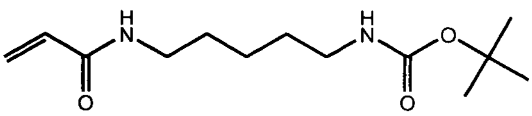

(**2**)

To a stirred suspension of *N*-Boc-1,5-diaminopentane toluene sulfonic acid (5.2 g, 13.88 mmol) and triethylamine (4.83 ml, 2.5 eq) in THF (120 ml) at 0°C was added acryloyl chloride (1.13 ml, 1 eq) through a pressure equalized dropping funnel over a one hour period. The reaction mixture was then stirred at room temperature and the progress of the reaction checked by TLC (petroleum ether:ethyl acetate 1:1). After two hours, the salts formed during the reaction were filtered off and the filtrate evaporated to dryness. The residue was purified by flash chromatography (neat petroleum ether followed by a gradient of ethyl acetate up to 60%) to yield 2.56 g (9.98 mmol, 71 %) of product 2 as a beige solid. $^1H$ NMR (400 MHz, $d_6$-DMSO) : 1.20-1.22 (m, 2H, $CH_2$), 1.29-1.43 (m, 13H, tBu, $2xCH_2$), 2.86 (q, 2H, *J* = 6.8 Hz and 12.9 Hz, $CH_2$), 3.07 (q, 2H, *J* = 6.8 Hz and 12.9 Hz, $CH_2$), 5.53 (dd, 1H, *J* = 2.3 Hz and 10.1 Hz, CH), 6.05 (dd, 1H, *J* = 2.3 Hz and 17.2 Hz, CH), 6.20 (dd, 1H, *J* = 10.1 Hz and 17.2 Hz, CH), 6.77 (t, 1H, *J* = 5.3 Hz, NH), 8.04 (bs, 1H, NH). Mass (electrospray+) calculated for $C_{13}H_{24}N_2O_3$ 256, found 279 (256+$Na^+$).

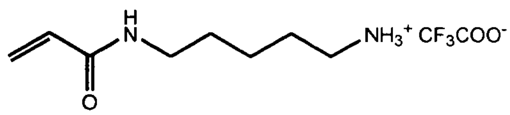

(**3**)

Product 2 (2.56g, 10 mmol) was dissolved in trifluoroacetic acid:dichloromethane (1:9, 100 ml) and stirred at room temperature. The progress of the reaction was monitored by TLC (dichloromethane:methanol 9:1). On completion, the reaction mixture was evaporated to dryness, the residue co-evaporated three times with toluene and then purified by flash chromatography (neat dichloromethane followed by a gradient of methanol up to 20%). Product **3** was obtained as a white powder (2.43 g, 9 mmol, 90%). $^1H$ NMR (400 MHz, $D_2O$) : 1.29-1.40 (m, 2H, $CH_2$), 1.52 (quint., 2H, *J* = 7.1 Hz, $CH_2$), 1.61 (quint., 2H, J = 7.7 Hz, $CH_2$), 2.92 (t, 2H, *J* = 7.6 Hz, $CH_2$), 3.21 (t, 2H, *J* = 6.8 Hz, $CH_2$), 5.68 (dd, 1H, *J* = 1.5 Hz and 10.1 Hz, CH), 6.10 (dd, 1H, J = 1.5 Hz and 17.2 Hz, CH), 6.20 (dd, 1H, *J* = 10.1 Hz and 17.2 Hz, CH). Mass (electrospray+) calculated for $C_8H_{16}N_2O$ 156, found 179 (156+$Na^+$).

To a suspension of product **3** (6.12 g, 22.64 mmol) and triethylamine (6.94 ml, 2.2 eq) in THF (120 ml) was added bromoacetyl chloride (2.07 ml, 1.1eq), through a pressure equalized dropping funnel, over a one hour period and at -60°C (cardice and isopropanol bath in a Dewar). The reaction mixture was then stirred at room temperature overnight and the completion of the reaction was checked by TLC (dichloromethane:methanol 9:1) the following day. The salts formed during the reaction were filtered off and the reaction mixture evaporated to dryness. The residue was purified by chromatography (neat dichloromethane followed by a gradient of methanol up to 5%). 3.2 g (11.55 mmol, 51 %) of the product 1 (BRAPA) were obtained as a white powder. A further recrystallization performed in petroleum ether:ethyl acetate gave 3g of the product **1.** $^1H$ NMR (400 MHz, $d_6$-DMSO) : 1.21-1.30 (m, 2H, $CH_2$), 1.34-1.48 (m, 4H, $2xCH_2$),

3.02-3.12 (m, 4H, 2x$CH_2$), 3.81 (s, 2H, $CH_2$), 5.56 (d, 1H, *J* = 9.85 Hz, CH), 6.07 (d, 1H, *J* = 16.9 Hz, CH), 6.20 (dd, 1H, *J* = 10.1 Hz and 16.9 Hz, CH), 8.07 (bs, 1H, NH), 8.27 (bs, 1H, NH). Mass (electrospray+) calculated for $C_{10}H_{17}BrN_2O_2$ 276 or 278, found 279 (278+$H^+$), 299 (276+$Na^+$).

Example 4) Grafting primers onto surface of SFA coated chip

An SFA coated chip is placed onto a modified MJ-Research thermocycler and attached to a peristaltic pump. Grafting mix consisting of 0.5 μM of a forward primer and 0.5 μM of a reverse primer in 10 mM phosphate buffer (pH 7.0) is pumped into the channels of the chip at a flow rate of 60 μl/minutes for 75 s at 20 °C. The thermocycler is then heated to 51.6 °C, and the chip is incubated at this temperature for 1 hour. During this time, the grafting mix undergoes 18 cycles of pumping: grafting mix is pumped in at 15 μl/minutes for 20 s, then the solution is pumped back and forth (5 s forward at 15 μl/minutes, then 5 s backward at 15 μl/minutes) for 180 s. After 18 cycles of pumping, the chip is washed by pumping in 5xSSC/5mM EDTA at 15 μl/minutes for 300 s at 51.6 °C. The thermocycler is then cooled to 20 °C.

The primers are typically 5'-phosphorothioate oligonucleotides incorporating any specific sequences or modifications required for cleavage. Their sequences and suppliers vary according to the experiment for which they are to be used, and in this case were complementary to the 5'-ends of the template duplex. The DNA sequence used in this process was the pool of the two libraries, which have ends complementary to the grafted primers. The library mix was denatured using sodium hydroxide treatment followed by snap dilution as described.

For some of the experiments detailed, the amplified clusters contained a diol linkage in one of the grafted primers. Diol linkages can be introduced by including a suitable linkage into one of the primers used for solid-phase amplification. Synthesis of the diol phosphoramidite is described in Example 4 below. Products containing such diol linkages can be cleaved using periodate and propanolamine as described, and the resulting single stranded polynucleotides hybridised as described.

The grafted primers contain a sequence of T bases at the 5'-end to act as a spacer group to aid linearisation and hybridization. The sequences of the three primers grafted to the chip are as follows:

Oligo A: 5'-PS-TTTTTTTTTTAATGATACGGCGACCACCGA**U**CTACAC-3' where U = 2-deoxyuridine;

Oligo B: 5'-PS-TTTTTTTTTTCAAGCAGAAGACGGCATACGA**Goxo**AT-3', where Goxo = 8-oxoguanine).

Example 5) Preparation of clusters by isothermal amplification:

Step 1: Hybridisation and amplification

The DNA sequence used in the amplification process is the mixture of the two libraries prepared in Example 1, which have ends complementary to the grafted primers. The duplex DNA (1 nM) is denatured using 0.1 M sodium hydroxide treatment followed by snap dilution to the desired 0.2-2 pM 'working concentration' in 'hybridization buffer' (5 x SSC / 0.1% TWEEN®).

Surface amplification was carried out by isothermal amplification using a commercially available Solexa/Illumina cluster station as described in PCT/US/2007/014649. The cluster station is essentially a hotplate and a fluidics system for controlled delivery of reagents to a flow cell.

The single stranded template (denatured as indicated above) is hybridised to the grafted primers immediately prior to the amplification reaction, which thus begins with an initial primer extension step rather than template denaturation. The hybridization procedure begins with a heating step in a stringent buffer to ensure complete denaturation prior to hybridisation. After the hybridization, which occurs during a 20 minutes slow cooling step, the flowcell was washed for 5 minutes with a wash buffer (0.3 x SSC / 0.1% TWEEN®).

During template hybridization and first extension, a number of solutions/buffers are typically employed, e.g., a solution comprising the DNA samples, a hybridization buffer (5 x SSC / 0.1% TWEEN®), a wash buffer (0.3 x SSC / 0.1% TWEEN®), a 2M sodium hydroxide solution, a cluster buffer (200 mM Tris, 100 mM Ammonium Sulfate, 20 mM Magnesium sulfate, 1% Triton, 1.3% DMSO, pH 8.8); an amplification additive (5 M betaine), DNA polymerase, and 10 mM dNTP mix.

To prepare the hybridization mixes, a 0.2 ml strip sample tube and the hybridization buffer are pre-chilled. Using 1.7 ml Eppendorf tube(s), the DNA template(s) are then diluted to 1nM in buffer EB (Qiagen). 1 μL of 2 M NaOH is added to 19 μL of template, vortexed briefly and incubated for 5 minutes at room temperature to denature the DNA template into single strands.

The denatured DNA is diluted to working concentration (0.2-2 pM) in pre-chilled hybridization buffer (e.g. for 1 mL of 1pM Hybridization mix, 1 μL of denatured DNA is diluted into 1 mL of pre-chilled hybridization buffer). The volume required depends on the number of channels used - at least 120 μL of hybridization mix per channel is optionally used.

Thus, 1 mL of hybridization mix is enough for 8 channels. The samples are vortexed briefly, spun down and aliquoted into the pre-chilled 0.2 ml strip tubes (with no bubbles in the bottom of the tubes) and used immediately.

To prepare the Amplification pre-mix (of volume enough for the first extension and 35 cycles of isothermal amplification), 35 mL of $H_20$ (MilliQ®), 7 mL of Cluster buffer (200 mM Tris, 100 mM Ammonium Sulfate, 20 mM Magnesium sulfate, 1% Triton, 1.3% DMSO, pH 8.8), and 28 mL of Amplification additive (5 M betaine solution) are mixed to achieve a final volume of 70 mL.

To prepare the first extension Taq mix, 780 μL of Amplification pre-mix, 16 μL of 10 mM dNTPs, and 4 μL of *Taq* DNA polymerase are mixed together for a final volume of 800 μl.

A typical amplification process is detailed in the following table (Table 1), detailing the flow volumes per channel, controlled automatically by the computer component of the invention.

**Table 1. Template hybridization and first extension.**

| **Step** Step | **Description** | **T (°C)** | **Time (sec)** | **Flow rate (μl/min)** | **Pumped V (μl)** |
|---|---|---|---|---|---|
| 1 | Pump Hybridization pre-mix | 20 | 120 | 60 | 120 |
| 2 | Pump Hybridization mix | 96 | 300 | 15 | 75 |
| 3 | Remove bubbles | 96 | 6 | 100 | 10 |
| 4 | Stop flow and hold T | 96 | 30 | static | 0 |
| 5 | Slow cooling | 96-40 | 1120 | static | 0 |
| 6 | Pump wash buffer | 40 | 300 | 15 | 75 |
| 7 | Pump amplification pre-mix | 40 | 280 | 15 | 70 |
| 8 | Pump amplification mix | 40 | 95 | 60 | 95 |
| 9 | First Extension | 74 | 90 | static | 0 |
| 10 | cool to room temperature | 20 | 0 | static | 0 |

**Isothermal amplification at 60 °C using formamide as denaturant**

The copied DNA can be isothermally amplified into clusters at 60 °C using formamide as a denaturant. The isothermal amplification (including both temperature control and reagent control) is overseen by the computer component. Table 2 gives outlines of exemplary script controls. After the isothermal amplification, and optional washing step occur, the nucleic acid of the clusters is ready to be linearized (*see below*).

**Table 2. Isothermal amplification**

| **Step** | **Description** | **T (°C)** | **Time (sec)** | **Flow rate (μl/min)** | **Pumped V (μl)** |
|---|---|---|---|---|---|
| (1) This sequence 35 times | Pump Formamide | 60 | 56 | 30 | 28 |
| | Pump Amplification pre-mix | 60 | 56 | 30 | 28 |
| | Pump Bst mix | 60 | 72 | 30 | 36 |
| 2 | Pump wash buffer | 60 | 280 | 30 | 140 |
| 3 | Pump Storage Buffer | 20 | 380 | 15 | 95 |

Wash buffer = 0.3 x SSC / 0.1% TWEEN®

Amplification pre mix = 2 M betaine, 20 mM Tris, 10 mM Ammonium Sulfate, 2 mM Magnesium sulfate, 0.1% Triton, 1.3% DMSO, pH 8.8

Bst mix = 2 M betaine, 20 mM Tris, 10 mM Ammonium Sulfate, 2 mM Magnesium sulfate, 0.1% Triton, 1.3% DMSO, pH 8.8 plus 200 μM dNTPs and 80 units/mL of Bst polymerase (NEB Product ref M0275L) Storage Buffer = 5X SSC.

Example 6) Preparation of clusters for first sequencing read

The preparation for read one was performed on the Illumina cluster station. All volumes used in the protocol were 95 μl per lane unless otherwise stated. Linearisation of A-type surface immobilised oligonucleotides was achieved by incubation with USER enzyme mix (cocktail of Uracil DNA Glycosylase and Endonuclease VIII, NEB #M5505, 10 U/ml, 10 mM KC1, 20 mM Tris pH 8.8, 10 mM $(HN_4)_2SO_4$, 2 mM $MgSO_4$, 0.1% Triton X-100, 37 °C, 30 minutes). All exposed 3'-OH termini of DNA, either from the extended template or unextended surface oligonucleotides were blocked by dideoxy chain termination using a cocktail of terminal transferase (0.25 U/μl) and a modified polymerase (SBS polymerase as described below) (0.015 mg/ml, 100 μM ddNTP, 50 mM Tris, 50 mM NaCl, 6 mM $MgSO_4$, 1 mM EDTA, 0.05 % TWEEN® 20). Blocking was achieved in a two stage protocol, initial incubation at 37 °C for 30 minutes followed by a ramping to 60 °C and incubating the flowcell for a further 15 minutes). Linearised and blocked clusters were washed with 0.3X SSC and storage buffer prior to denaturation with 0.IN NaOH. Denatured clusters were neutralised with TE buffer (10 mM Tris pH 8.0, 1 mM EDTA) and washed with storage buffer. The read 1 specific sequencing primer (5'-ACACTCTTTCCCTACACGACGCTCTTCCGATC -3', 0.5 μM in hybridisation buffer) was annealed to the clusters by incubation at 60 °C for 15 minutes, followed by a 0.3X SSC wash at 40 °C (ramp rate 1 °C / sec). The flowcell was finally flushed with storage buffer (at 20 °C). Processed flowcells were transferred to the Illumina Genome Analyser for sequencing read 1.

Example 7) Sequencing from the target fragment

Sequencing of the clusters from the above illustrative protocol was carried out using modified nucleotides prepared as described in International patent application WO 2004/018493, and labeled with four spectrally distinct fluorophores, as described in PCT application number PCT/GB2007/001770. Sequencing of clusters is described in more detail in patent WO06064199.

A mutant 9°N polymerase enzyme (an exo- variant including the triple mutation L408Y/Y409A/P410V and C223S) (SBS polymerase) was used for the nucleotide incorporation steps.

All processes were conducted as described in the Illumina Genome Analyser operating manual. The flowcell was mounted to the analyser, primed with sequencing reagents: position #1 = incorporation mix (1 μM dNTP mix, 0.015 μg/ml SBS polymerase, 50 mM Tris pH 9.0, 50 mM NaCl, 6 mM $MgSO_4$, 1 mM EDTA, 0.05 % TWEEN® 20); position # 2 = spare (MilliQ® water only); position # 3 = scan mix (100 mM Tris pH 7.0, 50 mM sodium ascorbate); position #4 = High salt wash (5x SSC, 0.05 % TWEEN® 20); position # 5 = incorporation buffer (50 mM Tris pH 9.0, 50 mM NaCl, 1 mM EDTA, 0.05 % TWEEN® 20); position #6 = cleavage mix (100 mM TCEP, 100 mM Tris pH 9.0, 100 mM NaCl, 50 mM sodium ascorbate, 0.05% TWEEN® 20); position #7 = cleavage buffer (100 mM Tris pH 9.0, 100 mM NaCl, 0.05% TWEEN® 20); position #8 = spare (single reads) or connected to PE module outlet (paired read experiments). Flowcells were sequenced using standard sequencing recipes for either 27- or 37-cycle experiments. Data was analysed using the standard analysis pipeline.
Incorporation: Prime with Incorporation buffer, 125 μL/channel; 60 μL/minutes, Heat to 60°C
Treat with Incorporation mix, 75 μL/channel; 60 μL/minutes.
Wait for a total of 15 minutes in addition to pumping fresh Incorporation mix, 25 μL/channel; 60 μL/minutes, every 4 minutes. Cool to 20 °C.
Wash with Incorporation buffer, 75 μL/channel; 60 μL/minutes.
Wash with 5XSSC/0.05% TWEEN® 20, 75 μL/channel; 60 μL/minutes
Prime with imaging buffer, 100 μL/channel; 60 μL/minutes
Scan in 4 colors at RT.

Cleavage: Prime with Cleavage buffer (0.1M Tris pH 7.4, 0.1M NaCl and 0.05% TWEEN® 20), 125 μL/channel; 60 μL/minutes. Heat to 60 °C.
Treat the clusters with Cleavage mix (100 mM TCEP in Cleavage buffer), 75 μL/channel; 60 μL/minutes.
Wait for a total of 15 minutes in addition to pumping fresh cleavage mix, 25 μL/channel; 60 μL/minutes, every 4 minutes. Cool to 20 °C.
Wash with Enzymology buffer.
Wash with 5XSSC/0.05% TWEEN® 20.

Repeat the process of Incorporation and Cleavage for as many cycles as required.

Incorporated nucleotides were detected using the Illumina genome analyzer, a Total Internal Reflection based fluorescent CCD imaging apparatus described in "Systems and Devices for Sequence by Synthesis Analysis," USSN 60/788,248, filed March 31, 2006 and corresponding PCT application PCT/US07/07991 filed March 30 2007.

Example 8) Preparation of the clusters for the second read

Following the successful completion sequencing of read 1 on the Genome Analyser, flowcells remained mounted and were prepared for read 2 *in situ*, using the Illumina Paired End module. Temperature control was achieved by using the Genome Analyser peltier. All flow rates were 60 μl/min and 75 μl per lane unless otherwise stated. Clusters were denatured with 0.1 M NaOH to remove the extended sequencing primer from read 1. Clusters were 3'-dephosphorylated using T4 polynucleotide kinase (Invitrogen #18004-010, 200 U/ml, 50 mM imidazole pH 6.4, 12 mM $MgCl_2$, 70 μM ADP, 1 mM 2-mercaptoethanol, 37 °C, 30 minutes), prior to resynthesis of the A-strand achieved using 15 cycles of 60 °C isothermal amplification (same reagents and conditions as described for cluster creation except conducted at 30 μl/min). Clusters were washed before and after resynthesis with 0.3X SSC (150 μl and 245 μl respectively). Linearisation of the B-strand of the re-synthesised clusters was achieved by the excision of 8-oxoguanine from the B-type oligo using Fpg (formamidopyrimidine DNA glycosylase, NEB #M0240, 80 U/ml, 10 mM Bis Tris propane pH 7.0, 10 mM MgCl2, 1mM dithiothreitol, 37 °C, 30 minutes). Blocking was performed as described for read 1 using the same blocking cocktail. Linearised and blocked clusters were denatured prior to hybridisation of the read 2 specific sequencing primer (5'-CGGTCTCGGCATTCCTGCTGAACCGCTCTTCCGATC -3', 0.5 μM in hybridisation buffer) as described for read 1. Read 2 of the processed flowcells was subsequently sequenced on the Illumina Genome Analyser.

Data generated from the two reads is shown in figure 8. CT840 and 841 refer to two different libraries prepared with different size fragments selected from the polyacrylamide gel in Example 1, step 3, fragmented to different sizes for cluster growth according to example 1, step 14. CT840 consisted of circularised fragments of 2-2.5 kb, and library fragments of 400-600 bases, whereas CT841 consisted of circularised fragments of 3-4 kb, and library fragments of 300-400 base pairs. As can be seen from the data, the majority of fragments gave paired ends reads of approximately the size of the circular fragments, showing effective selection of the ends of the original sample fragments. A low level of fragments showing two reads separated by only the size of the linear fragments was present in the library, as expected from the conventional sequencing data which gave 2/13 clones that came from either non-circular fragments that were not digested, or from non biotinylated fragments of the circular fragments that were not removed.

The standard control sample generated only sequences separated by the length of the fragments used to make the clusters, as expected.

**Example 3 'Biotin End Repair Nucleotide Exchange Reaction'**

Introduction

This protocol describes a method to incorporate limited amounts of biotinylated nucleotides in close proximity to the end of DNA fragments. It has been observed that biotin molecules located close to the end of DNA fragments can inhibit the subsequent ligation of the fragments. By limiting the amount of biotin that is incorporated into the molecules it is possible to optimize the conditions so as to generate more ligatable product that still contains biotinylated nucleotides.

The reaction takes place in two steps, 1) a Pre-Biotin End Repair Reaction, with only natural nucleotides present, which generates blunt ended molecules, and 2) a Biotin dNTP End Repair Nucleotide Exchange reaction, in which natural nucleotides are exchanged with Biotin nucleotides in a end repair reaction in the presence of ~4% Biotin nucleotides and 96% natural nucleotides.

1)_Pre-Biotin End-Repair Reaction

Materials:

- Nebulized or fragmented DNA
- Water
- T4 DNA ligase buffer with 10mM ATP (10x) (NEB, B0202S)
- dNTPs mix (10 mM each) (NEB, N0447S)
- T4 DNA Polymerase (3U/μl) (NEB, M0203L)
- E. coli DNA Pol I large fragment (Klenow) (5U/μl) (NEB, M0210S)
- T4 polynucleotide kinase (10U/μl) (NEB, M0201L)
- PCR purification kit columns (Qiagen, 28104)

Procedure:

| | |
|---|---|
| Nebulized DNA (10 μg) | 50 μl |
| Water | 25 μl |
| T4 DNA ligase buffer with 10mM ATP | 10 μl |
| dNTPs | 4 μl |
| T4 DNA pol | 5 μl |
| Klenow DNA pol | 1 ul |
| T4 PNK | 5 ul |
| | 100 μl total |

The reaction was incubated for 30 mins at 20°C. The DNA was purified on a Qiagen column, eluting in 30 μl EB.

2)_Biotin End-repair Nucleotide Exchange Reaction.

Materials:

- Blunt, Pre-End Repaired DNA
- Water
- T4 DNA ligase buffer with 10 mM ATP (10x) (NEB, B0202S)
- 4% Mix Biotin-dNTP (1 mM)
- T4 DNA Polymerase (3U/μl) (NEB, M0203L)
- E. coli DNA Pol I large fragment (Klenow) (5U/μl) (NEB, M0210S)
- T4 polynucleotide kinase (10U/μl) (NEB, M0201L)
- PCR purification kit columns (Qiagen, 28104)

Procedure:

| | |
|---|---|
| Nebulized DNA | 30 μl |
| Water | 39 μl |
| T4 DNA ligase buffer with 10 mM ATP | 10 μl |
| 4% Biotin dNTP Mix | 10 μl |
| T4 DNA pol | 5 μl |
| Klenow DNA pol | 1 μl |
| T4 PNK | 5 μl |
| | 100 μl total |

The reaction was incubated for 30 mins at 20 °C. The DNA was purified on a Qiagen column, eluting in 30 μl EB. The DNA is now ligation ready.

**Example 4 Biotin End Repair Nucleotide Exchange Reaction Optimization**

Introduction

Using a blunt 2686 bp test DNA fragment (representing an end repaired molecule) it is possible to incorporate limited amounts of biotin dNTPs into the DNA fragment and subsequently ligate this molecule to itself. It can be seen that by limiting the amount of biotin nucleotides present during the nucleotide exchange reaction, the amount of biotin incorporated is reduced. Furthermore results show that a reduced level of biotinylation increases the efficiency of subsequent intramolecular ligation of biotinylated fragments (figure 12).

Method

A blunt ended 2686 bp fragment representing a pre-biotin end repaired molecule was incubated at 20°C for 30 minutes in a biotin end repair nucleotide exchange reaction in the presence of varying proportions of biotin and natural nucleotides ranging from 50% biotin to 0% biotin, at a final total concentration of 100 μM dNTPs. The end repair exchange reactions were then purified and ligated overnight. The subsequent ligations were then run on an agarose gel with or without Streptavidin to determine how much of the linear blunt end fragment circularized and how much of it contained biotin molecules. The gel is shown in figure 12, where it can clearly be seen that reducing the percentage of Biotin dNTPs in end repair exchange reaction increases the efficiency of the ligation, as measured by the amount of circular product generated. If lower amounts of biotin are used, less biotinylated circles are formed, as the circular product does not gel shift in the presence of Streptavidin. With the end repair exchange reaction carried out on blunt ended or previously end repaired fragments, the optimum condition for the formation of biotinylated circles is between 2%-7.5% Biotin dNTPs in combination with natural dNTPs.

**Example 5 Protocol using T3 ligase for circularisation:**

10 μg of genomic DNA were fragmented to lengths approximately equal to the gap size required for the paired sequencing reads. DNA can be fragmented by a number of techniques including: sonication, nebulisation, HydroShear, chemical cleavage and enzymatic digestion. Example of a hydroshear protocol which was developed for obtaining DNA fragments between 2 and 5 kb in size is below, although modifications to the shearing parameters allows higher fragment sizes.

1) HydroShear Procedure:

Sample Preparation

Diluted 10 μg of genomic DNA to -150 ng/μl in water, to give a final volume of approximately 70 μl per sample preparation. Incubated at 37 °C for 30 minutes, and immediately before shearing spun at 18,000 g on a bench-top centrifuge for 15-30 minutes. Transferred the DNA solution to a clean tube, leaving behind any pellet that may have formed during the centrifugation. The sample was loaded into the hydroshear device and sheared following the manufacturer's directions and ejected into a clean tube.

2) End Repair

Materials:

- DNA from above
- End Repair Buffer 10X (NEB, B0202)
- UltraPureTM Water
- Natural dNTP Mix 10 mM each (NEB, N0447)
- T4 DNA Polymerase 3 U/μl (NEB, M0203)
- T4 Polynucleotide Kinase 10 U/μl (NEB, M0201)
- Klenow DNA Polymerase 5 U/μl (NEB, M0210)
- QIAquick PCR Purification Kit (Qiagen, 28104)

Procedure:

Prepared the following reaction mix in a 0.2 ml PCR tube:

| | |
|---|---|
| DNA from section 1 | 70 μl |
| End Repair Buffer | 10 μl |
| UltraPure™ Water | 5 μl |
| Natural dNTP Mix | 4 μl |

(continued)

| | |
|---|---|
| T4 DNA Polymerase | 5 μl |
| T4 Polynucleotide Kinase | 5 μl |
| Klenow DNA Polymerase | 1 μl |
| | 100 μl |

Incubated for 30 mins at 20 °C in a PCR machine. Purified on one QIAquick column using the QIAquick PCR Purification Kit and protocol, eluted in 50 μl of EB.

3) Biotin Label

Materials:

- DNA from section 2
- End Repair Buffer 10X (NEB, B0202)
- UltraPureTM Water
- Biotin dNTP Mix (1 mM final dNTP, 4 % Biotin-11-dNTPs, 96 % natural dNTPs (Perkin Elmer, NEL540001EA, NEL539001EA , NEL541 and NEL538B001EA, NEB, N0447))
- T4 DNA Polymerase 3 U/μl (NEB, M0203)
- T4 Polynucleotide Kinase 10 U/μl (NEB, M0201)
- Klenow DNA Polymerase 5 U/μl (NEB, M0210)
- QIAquick PCR Purification Kit (Qiagen, 28104)

Procedure:

Prepared the following reaction mix in a 0.2 ml PCR tube:

| | |
|---|---|
| DNA from section 2 | 50 μl |
| End Repair Buffer | 10 μl |
| UltraPureTM Water | 19 μl |
| Biotin dNTP Mix | 10 μl |
| T4 DNA Polymerase | 5 μl |
| T4 Polynucleotide Kinase | 5 μl |
| Klenow DNA Polymerase | 1 μl |
| | 100 μl |

Incubated for 30 mins at 20 °C in a PCR machine. Purified on one QIAquick column using the QIAquick PCR Purification Kit and protocol, eluted in 30 μl of EB.

4) Size Selection

The material was size selected on a 1% agarose gel for 2 hours, and the material with the desired size compared to a marker lane was excised using a clean scalpel. The DNA was purified from the agarose slices using a QIAquick Gel Extraction Kit according to the manufacturer's instructions. Eluted in 50 μl EB.

5) Circularize DNA

Materials:

- DNA from section 4 (200 ng, quantified on Agilent Bioanalyser)
- Circularization Buffer 10X (NEB, B0202)
- UltraPure™ Water
- T3 Ligase 3000 U/μl (Enzymatics, L601)

Procedure:

Prepared the following reaction mix in a 0.2 ml PCR tube:

| | |
|---|---|
| DNA from section 4 | 200 ng in 50 μl |
| Circularization Buffer | 10 μl |
| Circularization Ligase | 6.7 μl |
| UltraPure™ Water | 33.3 μl (to final reaction vol. of 100 μl) |

Incubated at 16 °C for 16 hours (overnight).

6) Digest Linear DNA

Materials:

- DNA from section 5
- DNA Exonuclease 10 U/μl (Epicentre, E3105K)
- EDTA (0.5 M)

Procedure:

Added 1 μl of DNA exonuclease to 100 μl of DNA from section 5. Incubated at 37 °oC for 20 mins followed by 70 °C for 30 mins. Added 4 μl of EDTA to exonuclease treated sample.

7) Fragmentation of circularised DNA by nebulisation.

Materials:

- DNA from section 6
- Nebulizers (Illumina, 1000182)
- PVC tubing (Intersurgical, 1174-003)
- Compressed air of at least 32 psi
- QIAquick PCR Purification Kit (Qiagen, 28104)

Procedure:

Added DNA from section 6 to the nebulizer. Added 700 μl of Nebulization buffer to the DNA and mixed well. The nebuliser was chilled on ice and compressed air was delivered at 32 psi for 6 mins. The contents of the nebuliser were collected and purified on one QIAquick column using the QIAquick PCR Purification Kit and protocol, eluting in 50 μl of EB.

**Example 6 Protocol 2c bead pull out after fragmentation:**

The nucleic acid material was treated according to example 5, steps 1-7 to obtain nebulised fragments. The fragments were bound to streptavidin beads as below:

Binding and washing of Biotinylated DNA

Materials:

- DNA from step 6, example 1
- Dynabeads® M-280 Streptavidin Magnetic Beads (Invitrogen, 112-05D)
- Magnet for microfuge tubes (Invitrogen, CS15000)
- Bead Binding Buffer (10 mM Tris-HCl pH 7.4 , 1 mM EDTA, 2 M NaCl)
- Bead Wash Buffer (5 mM Tris-HCl pH 7.4 , 0.5 mM EDTA, 1 M NaCl, 0.1 % TWEEN®)
- EB (from QIAquick PCR Purification Kit (Qiagen, 28104))

Procedure:

Transfered 20 μl of resuspended beads into a 1.5 ml microfuge tube. Placed the tube in the magnet for 1 min. Removed the supernatant with a pipette while the tube remained in the magnet. Washed the beads in 50 μl of Bead Binding Buffer, by carefully pipetting up and down. Used the magnet to remove the supernatant from the beads. Repeated the wash once. Resuspended the beads in 50 μl of Bead Binding Buffer. Added the DNA from step 6, example 1 (50 μl) and incubated for 15 mins at 20 °C. Resuspended the beads every 2 mins by gentle mixing. Removed the supernatant and wash the beads four times in 200 μl of Bead Wash Buffer. Resuspended the beads in each wash solution by pipetting up and down five times. Removed the supernatant and wash the beads twice in 200 μl of EB (Qiagen). Resuspended the beads in each wash solution by pipetting up and down five times. Removed the final wash solution from the beads and resuspend the beads in 50 μl of EB.

Step 2: End Repair

Materials:

- Washed beads from above
- End Repair Buffer 10X (NEB, B0202)
- UltraPureTM Water
- Natural dNTP Mix 10 mM each (NEB, N0447)
- T4 DNA Polymerase 3 U/μl (NEB, M0203)
- T4 Polynucleotide Kinase 10 U/μl (NEB, M0201)
- Klenow DNA Polymerase 5 U/μl (NEB, M0210)
- Magnet for microfuge tubes (Invitrogen, CS15000)
- Bead Wash Buffer (5 mM Tris-HCl pH 7.4 , 0.5 mM EDTA, 1 M NaCl, 0.1 % TWEEN®)
- EB (from QIAquick PCR Purification Kit (Qiagen, 28104))

Procedure:

Prepared the following reaction mix in a 0.2 ml PCR tube:

| | |
|---|---|
| Beads from above | 50 μl |
| End Repair Buffer | 10 μl |
| UltraPure™ Water | 25 μl |
| Natural dNTP Mix | 4 μl |
| T4 DNA Polymerase | 5 μl |
| T4 Polynucleotide Kinase | 5 μl |
| Klenow DNA Polymerase | 1 μl |
| | 100 μl |

Resuspended the beads in the end repair mix by pipetting up and down gently. Incubated for 30 mins at 20 °C in a PCR machine.

Transferred the bead mix to a 1.5 ml microfuge tube. Removed the supernatant using the magnet and washed the beads four times in 200 μl of Bead Wash Buffer. Resuspended the beads in each wash solution by pipetting up and down five times. Removed the supernatant and washed the beads twice in 200 μl of EB (Qiagen). Resuspended the beads in each wash solution by pipetting up and down five times. Removed the final wash solution from the beads and resuspended the beads in 32 μl of EB.

Step 3: A-Tail

Materials:

- Washed beads from above
- A-Tailing Buffer 10X (NEB, B7002)
- 1 mM dATP (Amersham-Pharmacia, 272050)
- A-Tailing Enzyme (Klenow exo minus) 5 U/μl (NEB, M0212)
- Magnet for microfuge tubes (Invitrogen, CS15000)
- Bead Wash Buffer (5 mM Tris-HCl pH 7.4, 0.5 mM EDTA, 1 M NaCl, 0.1 % TWEEN®)
- EB (from QIAquick PCR Purification Kit (Qiagen, 28104))

Procedure:

Prepared the following reaction mix in a 0.2 ml PCR tube:

| | |
|---|---|
| Beads from section 9 | 32 μl |
| A-Tailing Buffer | 5 μl |
| 1 mM dATP | 10 μl |
| A-Tailing Enzyme | 3 μl |
| | 50 μl |

Resuspended the beads are well in the A-tailing mix by pipetting up and down gently. Incubated for 30 mins at 37 °C. Transferred the bead mix to a 1.5 ml microfuge tube. Removed the supernatant using the magnet and washed the beads four times in 200 μl of Bead Wash Buffer. Resuspend the beads in each wash solution by pipetting up and down five times. Removed the supernatant and washed the beads twice in 200 μl of EB (Qiagen). Resuspended the beads in each wash solution by pipetting up and down five times. Removed the final wash solution from the beads and resuspended the beads in 19 μl of EB.

Step 4: Adapter Ligation

Materials:

- Washed beads from above
- Adapter Ligation Buffer 2X (NEB, B2200)
- PE Adapter Oligo Mix

Oligo A: 5' ACACTCTTTCCCTACACGACGCTCTTCCGATCxT (x = phosphorothioate bond)

Oligo B: 5'Phosphate GATCGGAAGAGCGGTTCAGCAGGAATGCCGAG

The oligos were mixed together to a final concentration of 15 μM each, in 10 mM Tris/10 mM NaCl pH 7.0. The adaptor strands were annealed in a PCR machine programmed as follows:

Ramp at 0.5 °C/sec to 97.5 °C
Hold at 97.5 °C for 150 sec

Then a step of 97.5 °C for 2 sec with a temperature drop of 0.1 °C/cycle for 775 cycles.

- Adapter Ligase U/µl (NEB, M2200)
- Magnet for microfuge tubes (Invitrogen, CS15000)
- Bead Wash Buffer (5 mM Tris-HCl pH 7.4, 0.5 mM EDTA, 1 M NaCl, 0.1 % TWEEN®)
- EB (from QIAquick PCR Purification Kit (Qiagen, 28104))

Procedure:

Prepared the following reaction mix in a 0.2 ml PCR tube:

| | |
|---|---|
| Beads from above | 19 µl |
| Adapter Ligation Buffer | 25 µl |
| PE Adapter Oligo Mix | 1 µl |
| Adapter Ligase | 5 µl |
| | 50 µl |

Resuspended the beads in the ligation mix by pipetting up and down gently. Incubated for 15 mins at 20 °C in a PCR machine. Transferred the bead mix to a 1.5 ml microfuge tube. Removed the supernatant using the magnet and washed the beads four times in 200 µl of Bead Wash Buffer. Resuspended the beads in each wash solution by pipetting up and down five times. Remove the supernatant and washed the beads twice in 200 µl of EB (Qiagen). Resuspended the beads in each wash solution by pipetting up and down five times. Removed the final wash solution from the beads and resuspended the beads immediately in 50 µl of PCR mix (see below).

Step 5 PCR

Materials:

- Washed beads from above
- Phusion DNA Polymerase 2X (NEB, F-531)
- UltraPureTM Water
- PCR Primer PE 1.0, 25 µM
  (Exonuclease treated 5' AATGATACGGCGACCACCGAGATCTACACTCTTTCCCTACACGACGCTCTTCCGATCxT 3', where x= phosphorothioate bond)
- PCR Primer PE 2.0
  (Exonuclease treated 5' CAAGCAGAAGACGGCATACGAGATCGGTCTCGGCATTCCTGCTGAACCGCTCTTCCGATCxT, where x= phosphorothioate bond)
- Magnet for microfuge tubes (Invitrogen, CS15000)

Procedure:

Prepared the following reaction mix in a 0.2 ml PCR tube:

| | |
|---|---|
| Phusion DNA Polymerase | 25 µl |
| UltraPureTM Water | 23 µl |

(continued)

| | |
|---|---|
| PCR Primer PE 1.0 | 1 μl |
| PCR Primer PE 2.0 | 1 μl |
| | 50 μl |

Resuspended the beads in 50 μl of the PCR mix. Carried out thermocycling in a PCR machine under the following conditions:

30 secs @ 98 °C
[10 sec@ 98 °C, 30 sec @ 65 °C, 30 sec @ 72 °C] 18 cycles
5 mins @ 72 °C
Hold @ 4 °C

Removed the PCR supernatant from the beads using the magnet and ran the PCR supernatant on an agarose gel (see step 14, example 1).

This library is suitable for cluster sequencing as described above. Sequencing data from this library is shown in figure 14, where the level of inward facing fragments was shown to be lower than a library made using the protocol detailed in example 1 above.

While the foregoing invention has been described in some detail for purposes of clarity and understanding, it will be clear to one skilled in the art from a reading of this disclosure that various changes in form and detail can be made without departing from the true scope of the invention. For example, all the techniques and apparatus described above may be used in various combinations.

## Claims

1. A method for producing a library of 3' and 5' modified nucleic acids suitable for paired end sequencing comprising;

    a) fragmenting a primary double stranded nucleic acid target sequence to produce a first population of linear nucleic acid target fragments with blunt ends, wherein the ends of the linear fragments are labelled;
    b) self-ligating the blunt ends of the fragments to form circular constructs without using an adapter of known sequence;
    c) treating the fragments of step b) to reduce the ratio of linear fragments to circular fragments, wherein treatment to reduce the ratio of linear to circular fragments comprises exonuclease treatment;
    d) fragmenting the circular constructs to produce a second population of linear fragments, a percentage of which will span the two ends of the first population of linear target fragments;
    e) selecting the linear fragments in the second population that span the two ends of the first population of linear fragments, wherein said selecting comprises isolating labelled linear fragments from unlabelled fragments by binding a ligand to the label in the linear fragments, and removing the unbound unlabelled fragments; and
    f) ligating adapter sequences to the 3' and 5' ends of the second population of linear fragments to produce a library of 3' and 5' modified nucleic acids suitable for paired end sequencing.

2. The method according to claim 1, wherein in step a) said blunt ended fragments are generated by shearing and enzymatically end repairing the primary sequence with a nucleic acid polymerase and nucleotides.

3. The method according to claim 1, wherein in step a) said blunt ended fragments are generated by random primer amplification of the primary sequence.

4. The method according to claim 3, wherein the random primers used in the amplification carry a ligand.

5. The method according to claim 4, wherein the ligand is biotin.

6. The method according to claim 2, wherein the nucleotide carries a modification allowing selection of the end repaired fragments.

7. The method according to claim 6, wherein the modification is biotin.

**8.** The method according to claim 1, wherein the exonuclease treatment is performed by DNase.

**9.** The method according to claim 1, wherein the library of 3' and 5' modified fragments is amplified in solution using amplification primers complementary to the adapter sequences.

**10.** The method according to claim 9, wherein the amplification primers are complementary to the adapter sequences and further comprise a single stranded sequence of nucleotides that extends beyond the 5' end of the adapter sequence.

**11.** The method according to claim 5 or 7, wherein the biotinylated sequences are isolated from non biotinylated sequences using avidin or streptavidin beads.

**12.** A nucleic acid construct for paired end sequencing comprising a circular nucleic acid molecule containing no adapter regions of known sequence and prepared by circularisation of a linear fragment of a primary double stranded nucleic acid target sequence containing no adapter regions of known sequence, wherein the ends of the linear fragment are labelled prior to circularisation, wherein the absence of adapter regions of known sequence in the circular nucleic acid molecule means that there are no artificially synthesized sequences present in the circular nucleic acid molecule that are not present in the primary target sequence of the nucleic acid molecule.

**13.** The nucleic acid construct according to claim 12, wherein the labels are biotin.

**14.** The nucleic acid construct according to claim 13, wherein the biotinylated nucleotides comprise less than 1 % of the nucleotides in the circular construct.

**Patentansprüche**

**1.** Verfahren zur Herstellung einer Bibliothek von 3'- und 5'-modifizierten Nukleinsäuren, die für Sequenzierung mit gepaarten Enden geeignet sind, das umfasst, dass

a) eine primäre doppelsträngige Nukleinsäure-Zielsequenz fragmentiert wird, um eine erste Population von linearen Nukleinsäure-Zielfragmenten mit stumpfen Enden zu erzeugen, wobei die Enden der linearen Fragmente markiert sind;
b) die stumpfen Enden der Fragmente selbstligiert werden, um ringförmige Konstrukte zu bilden, ohne einen Adapter mit bekannter Sequenz zu verwenden;
c) die Fragmente von Schritt b) behandelt werden, um das Verhältnis von linearen Fragmenten zu ringförmigen Fragmenten zu reduzieren, wobei die Behandlung, um das Verhältnis von linearen zu ringförmigen Fragmenten zu reduzieren, eine Exonukleasebehandlung umfasst;
d) die ringförmigen Konstrukte fragmentiert werden, um eine zweite Population von linearen Fragmenten zu erzeugen, wobei ein Prozentsatz von diesen die beiden Enden der ersten Population von linearen Zielfragmenten überspannt;
e) die linearen Fragmente in der zweiten Population ausgewählt werden, die die beiden Enden der ersten Population von linearen Fragmenten überspannen, wobei das Auswählen das Isolieren von markierten linearen Fragmenten aus unmarkierten Fragmenten durch Binden eines Liganden an die Markierung in den linearen Fragmenten, und das Entfernen der ungebundenen unmarkierten Fragmente umfasst; und
f) Adaptersequenzen an die 3'- und 5'-Enden der zweiten Population von linearen Fragmenten ligiert werden, um eine Bibliothek von 3'- und 5'-modifizierten Nukleinsäuren zu erzeugen, die für eine Sequenzierung mit gepaarten Enden geeignet sind.

**2.** Verfahren nach Anspruch 1, wobei in Schritt a) die stumpfendigen Fragmente durch Scherung und enzymatische Endreparatur der Primärsequenz mit einer Nukleinsäure-Polymerase und Nukleotiden erzeugt werden.

**3.** Verfahren nach Anspruch 1, wobei in Schritt a) die stumpfendigen Fragmente durch Zufallsprimeramplifikation der Primärsequenz erzeugt werden.

**4.** Verfahren nach Anspruch 3, wobei die bei der Amplifikation verwendeten Zufallsprimer einen Liganden tragen.

**5.** Verfahren nach Anspruch 4, wobei der Ligand Biotin ist.

6. Verfahren nach Anspruch 2, wobei das Nukleotid eine Modifikation trägt, die das Auswählen der endreparierten Fragmente erlaubt.

7. Verfahren nach Anspruch 6, wobei die Modifikation Biotin ist.

8. Verfahren nach Anspruch 1, wobei die Exonukleasebehandlung durch DNase erfolgt.

9. Verfahren nach Anspruch 1, wobei die Bibliothek von 3'- und 5'-modifizierten Fragmenten unter Verwendung von Amplifikationsprimern, die zu den Adaptersequenzen komplementär sind, in Lösung amplifiziert wird.

10. Verfahren nach Anspruch 9, wobei die Amplifikationsprimer zu den Adaptersequenzen komplementär sind und ferner eine einzelsträngige Sequenz von Nukleotiden aufweisen, die sich über das 5'-Ende der Adaptersequenz hinaus erstreckt.

11. Verfahren nach Anspruch 5 oder 7, wobei die biotinylierten Sequenzen aus nicht biotinylierten Sequenzen unter Verwendung von Avidin- oder Streptavidin-Kügelchen isoliert werden.

12. Nukleinsäurekonstrukt für eine Sequenzierung mit gepaarten Enden, das ein ringförmiges Nukleinsäuremolekül aufweist, das keine Adapterregionen bekannter Sequenzen enthält und durch Zirkularisierung eines linearen Fragments einer primären doppelsträngigen Nukleinsäure-Zielsequenz, die keine Adapterregionen bekannter Sequenz enthält, hergestellt ist, wobei die Enden des linearen Fragments vor der Zirkularisierung markiert sind, wobei Abwesenheit von Adapterregionen bekannter Sequenz im ringförmigem Nukleinsäuremolekül bedeutet, dass keine künstlich synthetisierten Sequenzen im ringförmigen Nukleinsäuremolekül vorhanden sind, die in der primären Zielsequenz des Nukleinsäuremoleküls nicht vorhanden sind.

13. Nukleinsäurekonstrukt nach Anspruch 12, wobei die Markierungen Biotin sind.

14. Nukleinsäurekonstrukt nach Anspruch 13, wobei die biotinylierten Nukleotide weniger als 1% der Nukleotide im ringförmigen Konstrukt ausmachen.

## Revendications

1. Procédé de production d'une banque ou bibliothèque d'acides nucléiques modifiés en 3' et 5' adaptée pour le séquençage d'extrémités appariées comprenant ;

   a) la fragmentation d'une séquence cible d'acide nucléique double brin primaire pour produire une première population de fragments cibles d'acide nucléique linéaire avec des extrémités franches, dans lequel les extrémités des fragments linéaires sont marquées ;
   b) l'auto-ligature des extrémités franches des fragments pour former des constructions circulaires sans utiliser un adaptateur de séquence connue ;
   c) le traitement des fragments de l'étape b) pour réduire le rapport des fragments linéaires aux fragments circulaires, où le traitement pour réduire le rapport des fragments linéaires à circulaires comprend un traitement par exonucléase ;
   d) la fragmentation des constructions circulaires pour produire une deuxième population de fragments linéaires, dont un pourcentage couvre les deux extrémités de la première population de fragments cibles linéaires ;
   e) le choix des fragments linéaires dans la deuxième population qui couvrent les deux extrémités de la première population de fragments linéaires, où ladite sélection comprend l'isolement de fragments linéaires marqués à partir de fragments non marqués par liaison d'un ligand au marqueur dans les fragments linéaires, et élimination des fragments non marqués, non liés ; et
   f) la ligature de séquences adaptatrices aux extrémités 3' et 5' de la deuxième population de fragments linéaires pour produire une banque d'acides nucléiques modifiés en 3' et 5' adaptée pour le séquençage d'extrémités appariées.

2. Procédé selon la revendication 1, dans lequel, dans l'étape a), lesdits fragments à extrémités franches sont générés par cisaillement et réparation enzymatique d'extrémité de la séquence primaire avec une acide nucléique polymérase et des nucléotides.

3. Procédé selon la revendication 1, dans lequel, dans l'étape a), lesdits fragments à extrémités franches sont générés par amplification d'amorce aléatoire de la séquence primaire.

4. Procédé selon la revendication 3, dans lequel les amorces aléatoires utilisées dans l'amplification comportent un ligand.

5. Procédé selon la revendication 4, dans lequel le ligand est la biotine.

6. Procédé selon la revendication 2, dans lequel le nucléotide comporte une modification permettant la sélection des fragments à extrémités réparées.

7. Procédé selon la revendication 6, dans lequel la modification est la biotine.

8. Procédé selon la revendication 1, dans lequel le traitement par exonucléase est effectué par DNase.

9. Procédé selon la revendication 1, dans lequel la banque ou bibliothèque de fragments modifiés en 3' et 5' est amplifiée en solution en utilisant des amorces d'amplification complémentaires des séquences adaptatrices.

10. Procédé selon la revendication 9, dans lequel les amorces d'amplification sont complémentaires des séquences adaptatrices et comprennent en outre une séquence de nucléotides simple brin qui s'étend au-delà de l'extrémité 5' de la séquence adaptatrice.

11. Procédé selon la revendication 5 ou 7, dans lequel les séquences biotinylées sont isolées à partir de séquences non biotinylées en utilisant des billes d'avidine ou de streptavidine.

12. Construction d'acide nucléique pour le séquençage d'extrémités appariées comprenant une molécule d'acide nucléique circulaire ne contenant pas de régions adaptatrices de séquence connue et préparées par circularisation d'un fragment linéaire d'une séquence cible d'acide nucléique double brin primaire ne contenant pas de régions adaptatrices de séquence connue, où les extrémités du fragment linéaire sont marqués avant circularisation, où l'absence de régions adaptatrices de séquence connue dans la molécule d'acide nucléique circulaire signifie qu'il n'y a pas de séquences artificiellement synthétisées présentes dans la molécule d'acide nucléique circulaire qui ne sont pas présentes dans la séquence cible primaire de la molécule d'acide nucléique.

13. Construction d'acide nucléique selon la revendication 12, dans laquelle les marqueurs sont la biotine.

14. Construction d'acide nucléique selon la revendication 13, dans laquelle les nucléotides biotinylés comprennent moins de 1 % des nucléotides dans la construction circulaire.

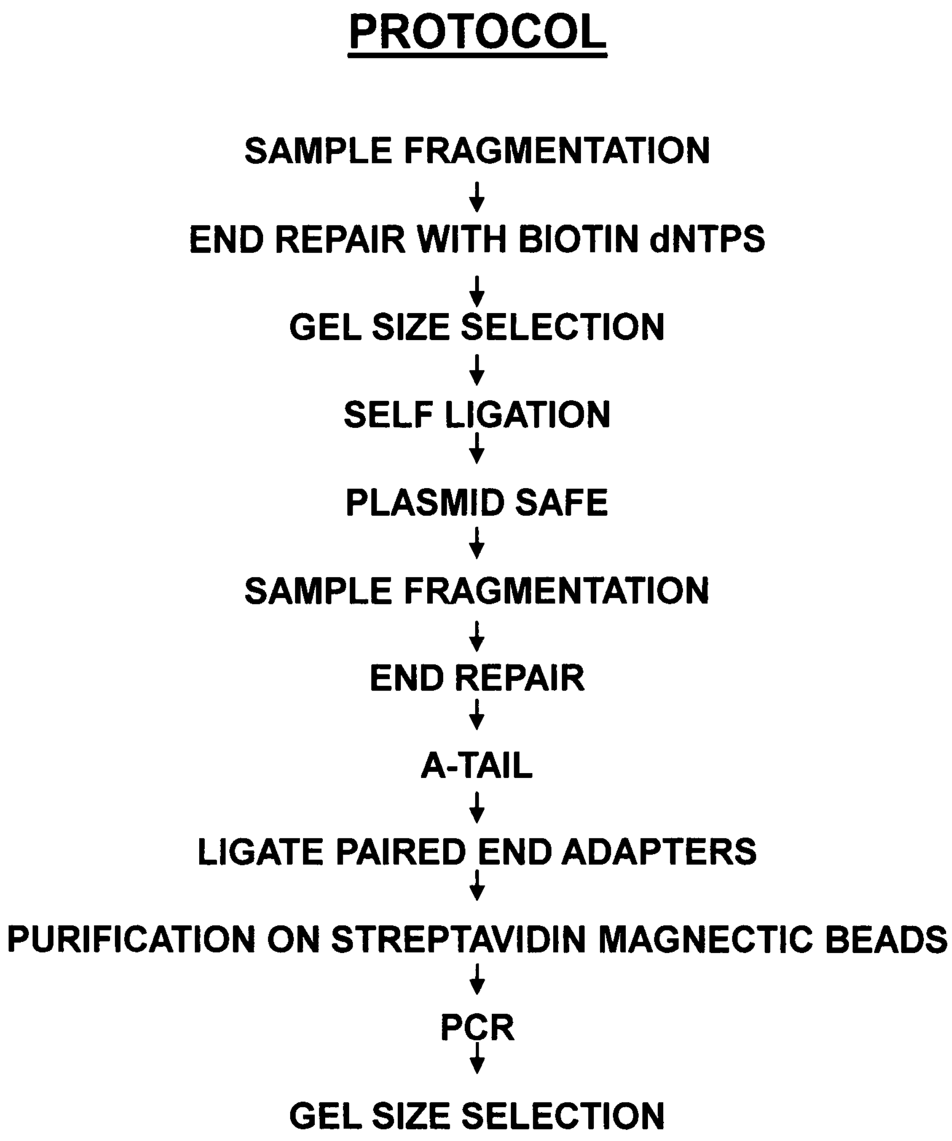
PROTOCOL
SAMPLE FRAGMENTATION
END REPAIR WITH BIOTIN dNTPS
GEL SIZE SELECTION
SELF LIGATION
PLASMID SAFE
SAMPLE FRAGMENTATION
END REPAIR
A-TAIL
LIGATE PAIRED END ADAPTERS
PURIFICATION ON STREPTAVIDIN MAGNECTIC BEADS
PCR
GEL SIZE SELECTION

Fig 1

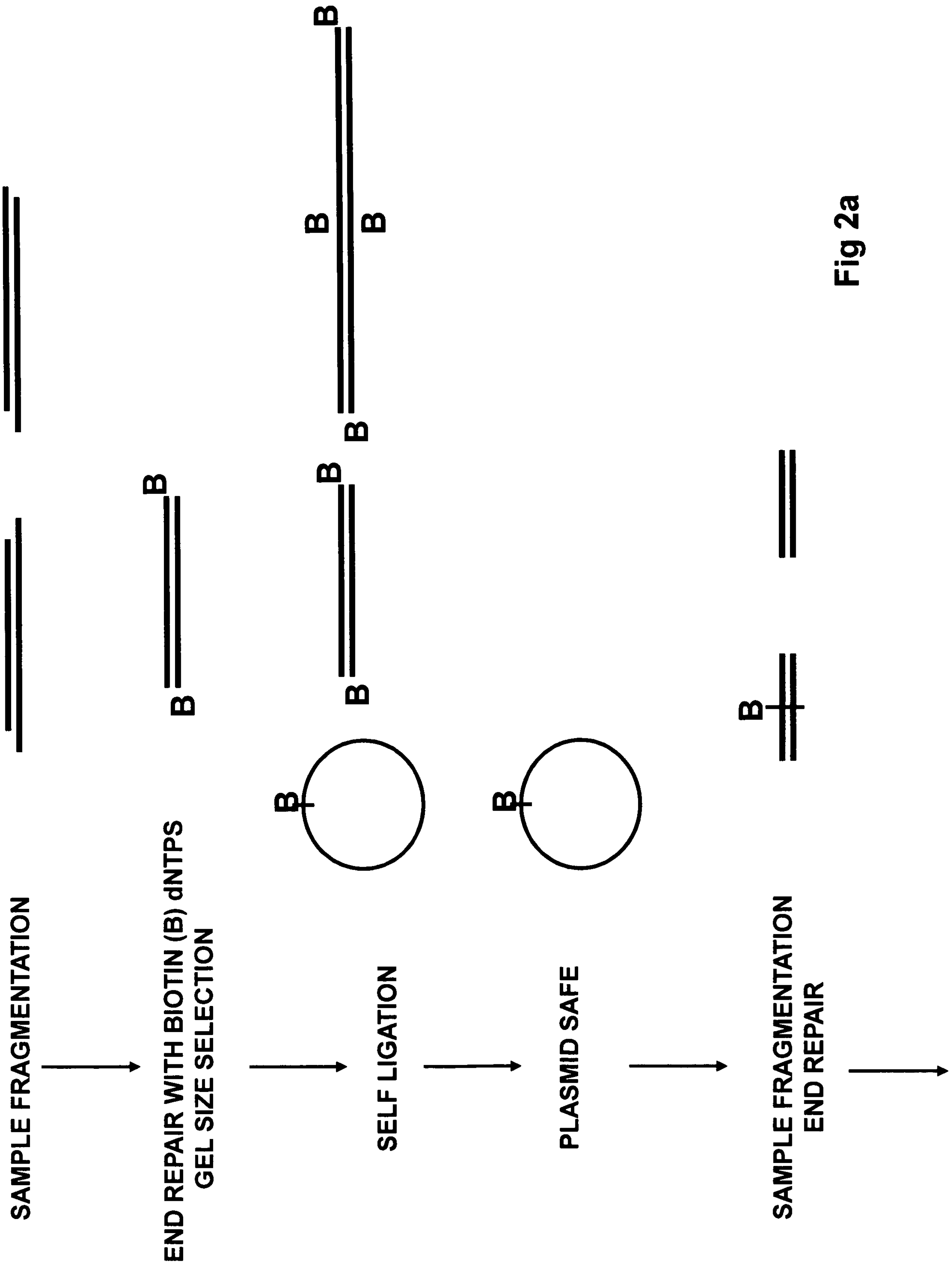
SAMPLE FRAGMENTATION
END REPAIR WITH BIOTIN (B) dNTPS
GEL SIZE SELECTION
B
B
SELF LIGATION
B
B
B
B
B
B
PLASMID SAFE
B
SAMPLE FRAGMENTATION
END REPAIR
B

Fig 2a

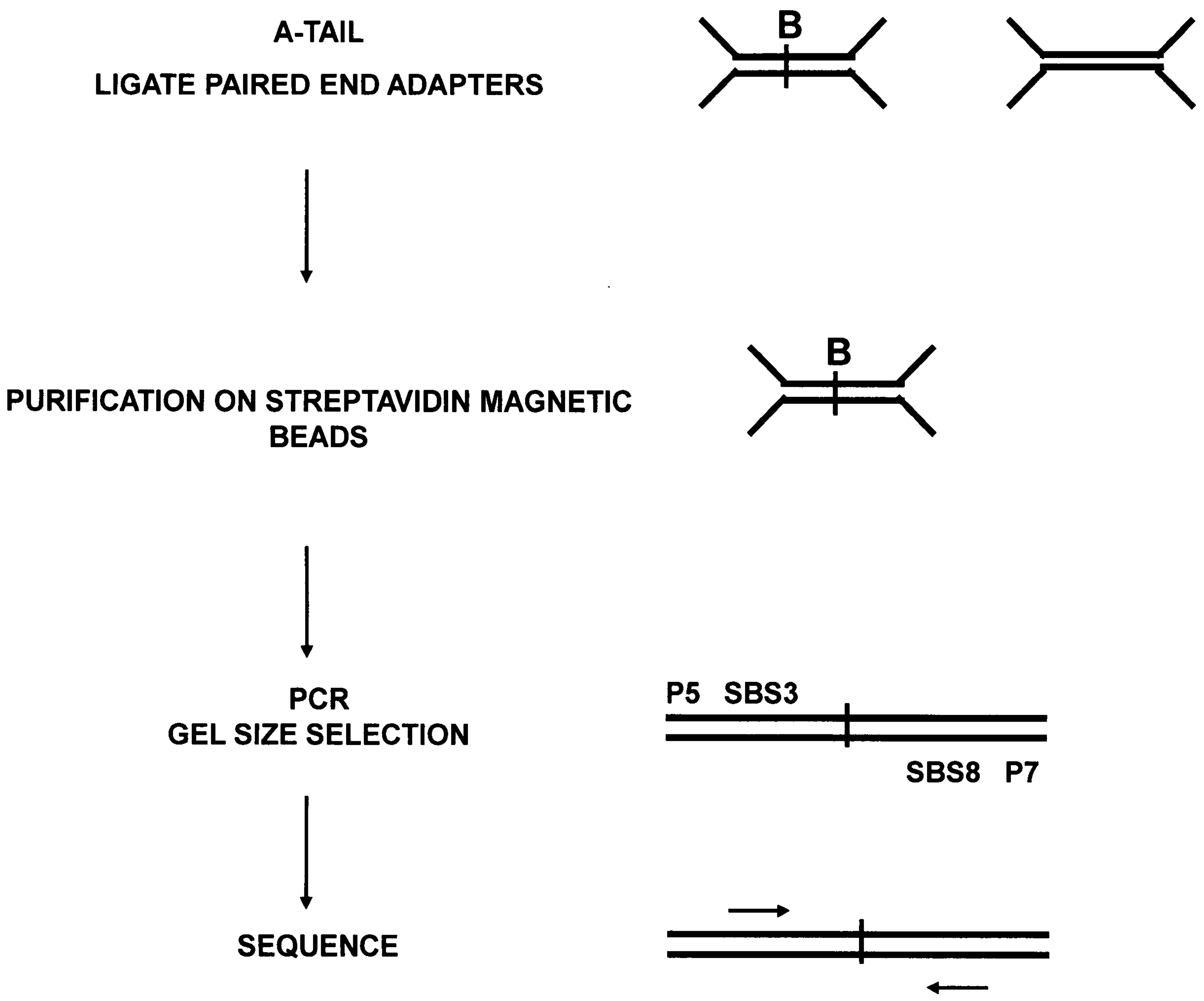
A-TAIL
LIGATE PAIRED END ADAPTERS
B
PURIFICATION ON STREPTAVIDIN MAGNETIC BEADS
B
PCR
GEL SIZE SELECTION
P5
SBS3
SBS8
P7
SEQUENCE

Fig 2b

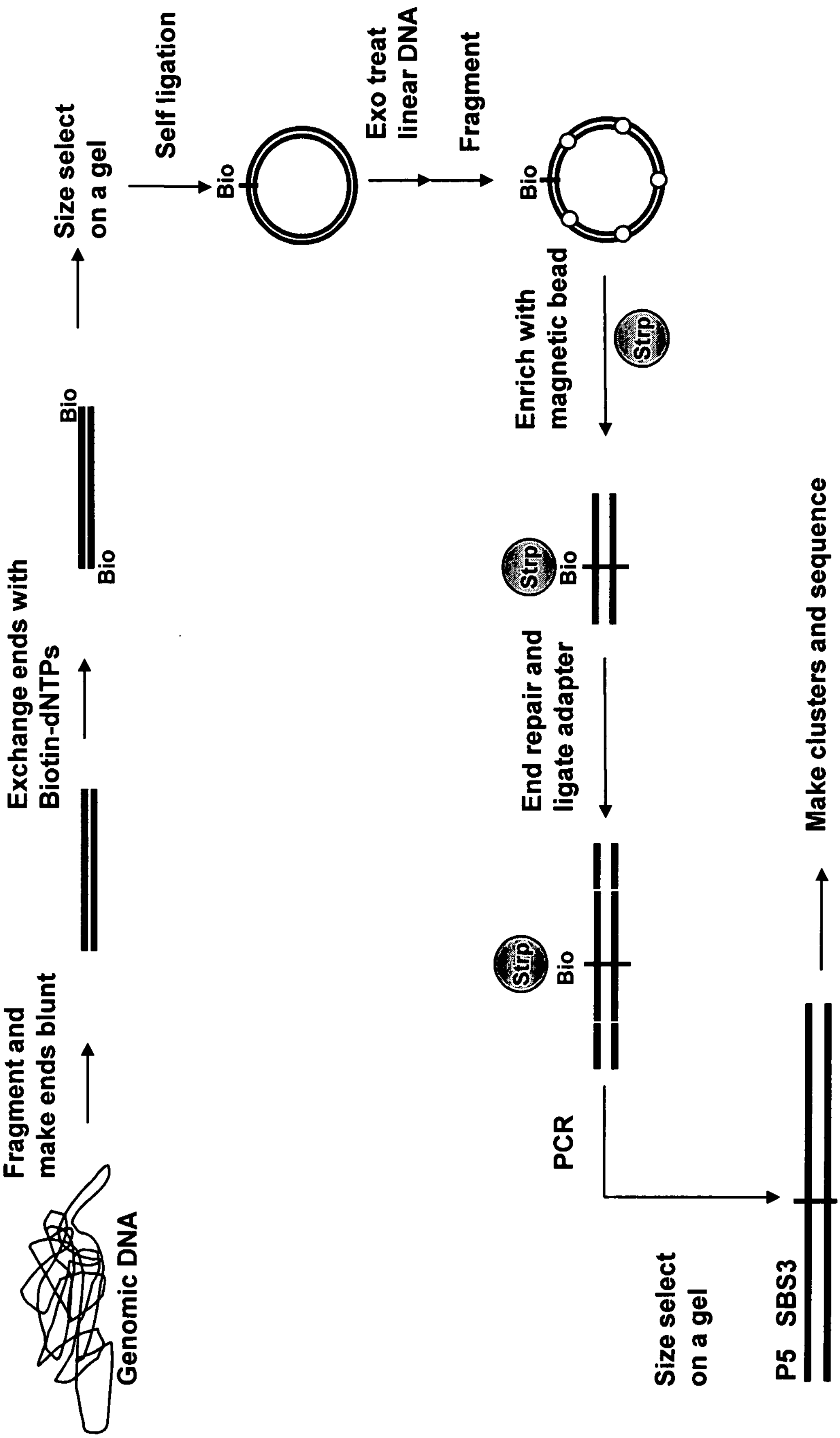
Genomic DNA
Fragment and
make ends blunt
Exchange ends with
Biotin-dNTPs
Bio
Bio
Size select
on a gel
Self ligation
Bio
Exo treat
linear DNA
Fragment
Bio
Enrich with
magnetic bead
Strp
Strp
Bio
End repair and
ligate adapter
Strp
Bio
PCR
Size select
on a gel
P5 SBS3
SBS8 P7
Make clusters and sequence

Fig 2c

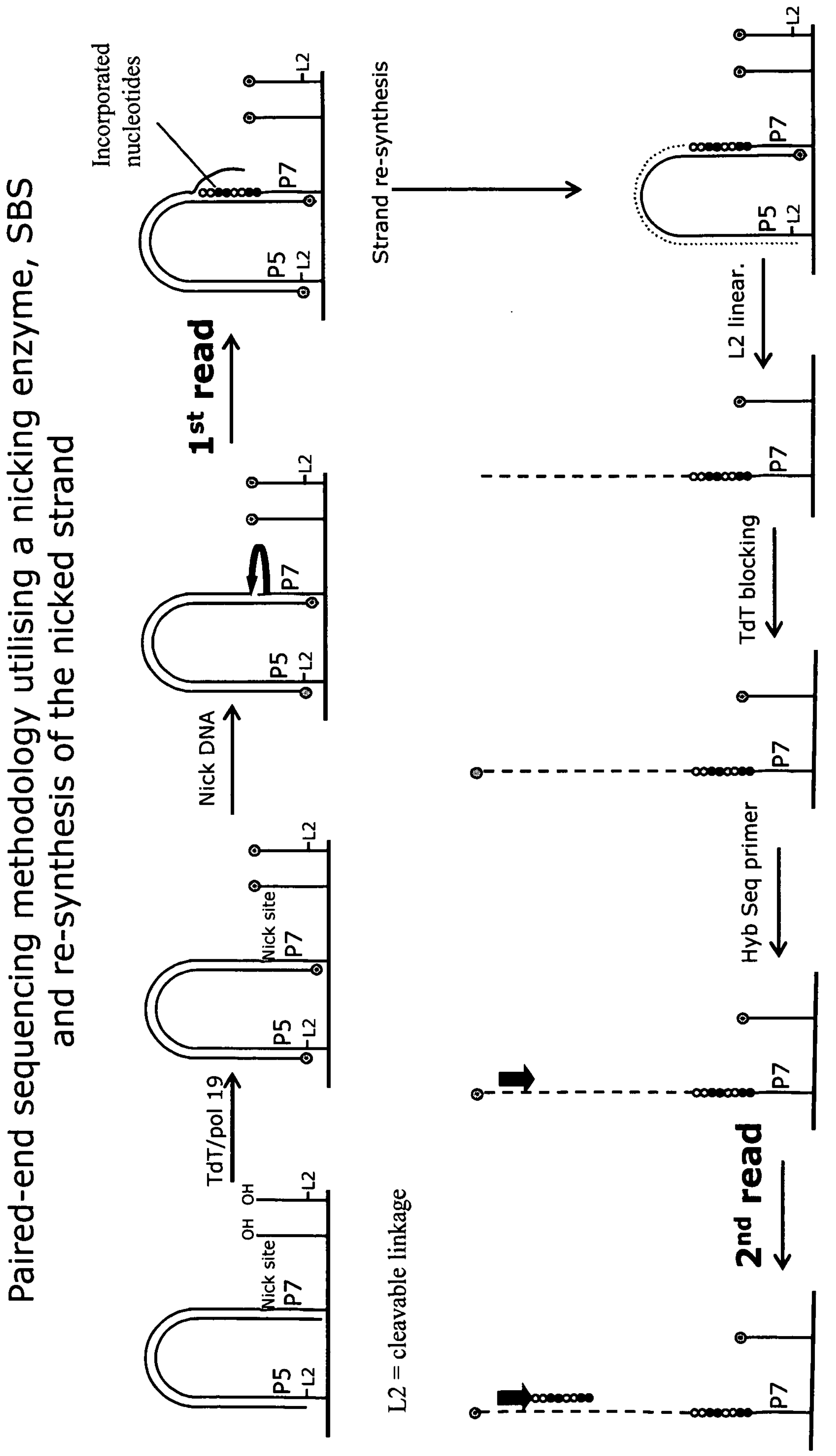
Paired-end sequencing methodology utilising a nicking enzyme, SBS
and re-synthesis of the nicked strand
P5
L2
P7
Nick site
OH
OH
TdT/pol 19
Nick DNA
1st read
Incorporated
nucleotides
L2 = cleavable linkage
Strand re-synthesis
L2 linear.
TdT blocking
Hyb Seq primer
2nd read

Figure 3

Paired-end sequencing: USER linearisation resynthesis method

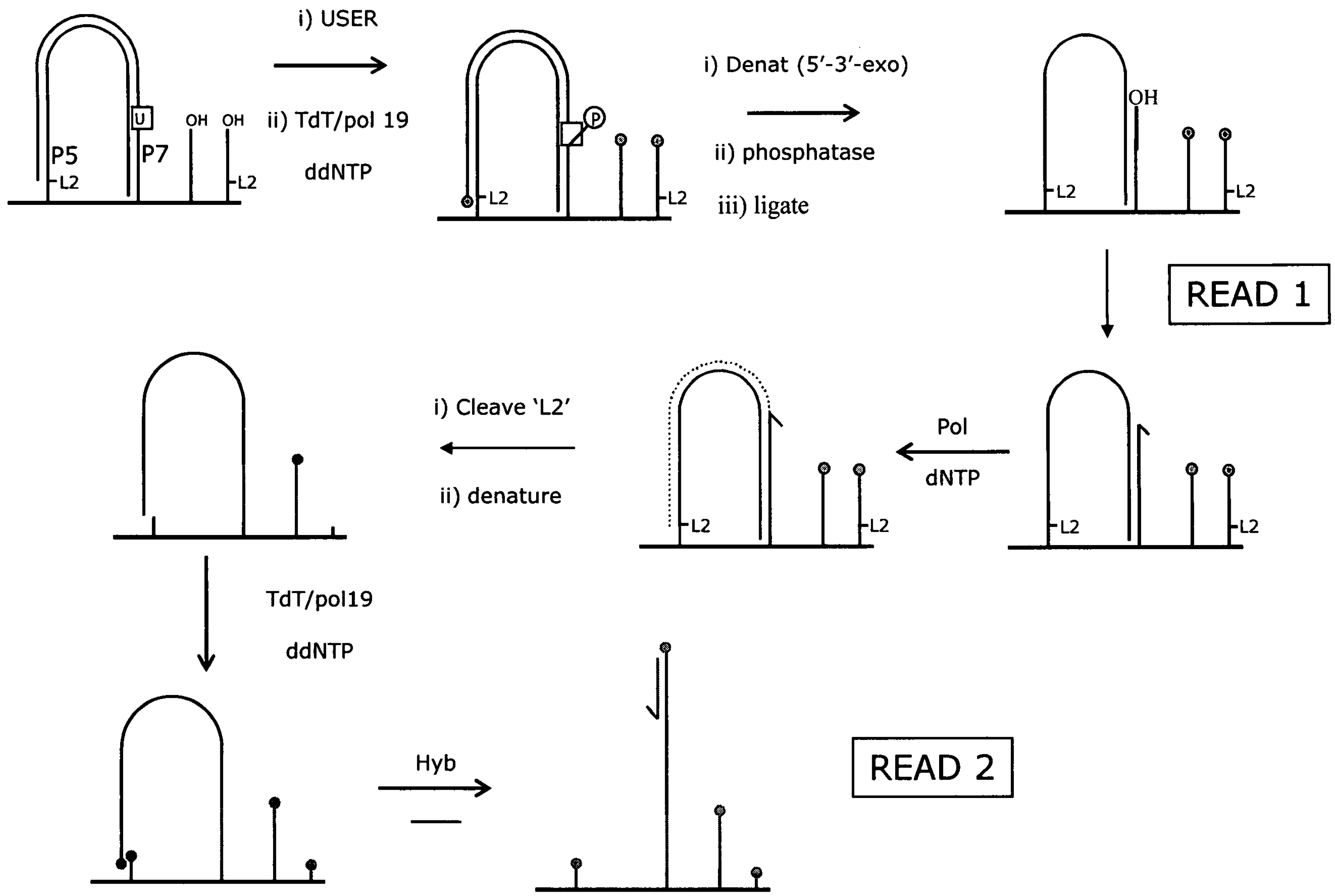


Figure 4

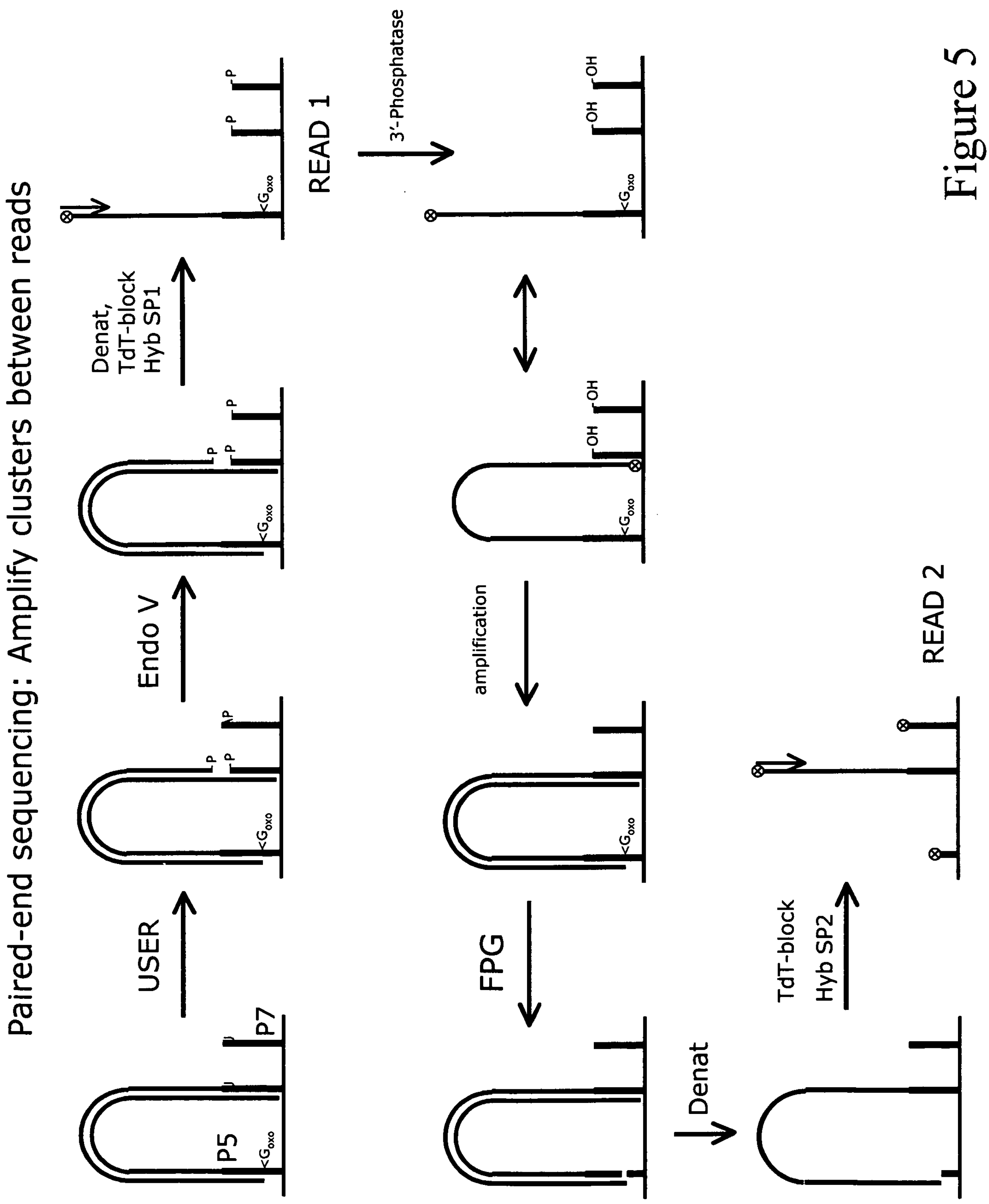
Paired-end sequencing: Amplify clusters between reads
P5
P7
USER
Endo V
Denat,
TdT-block
Hyb SP1
READ 1
3'-Phosphatase
FPG
amplification
Denat
TdT-block
Hyb SP2
READ 2

Figure 5

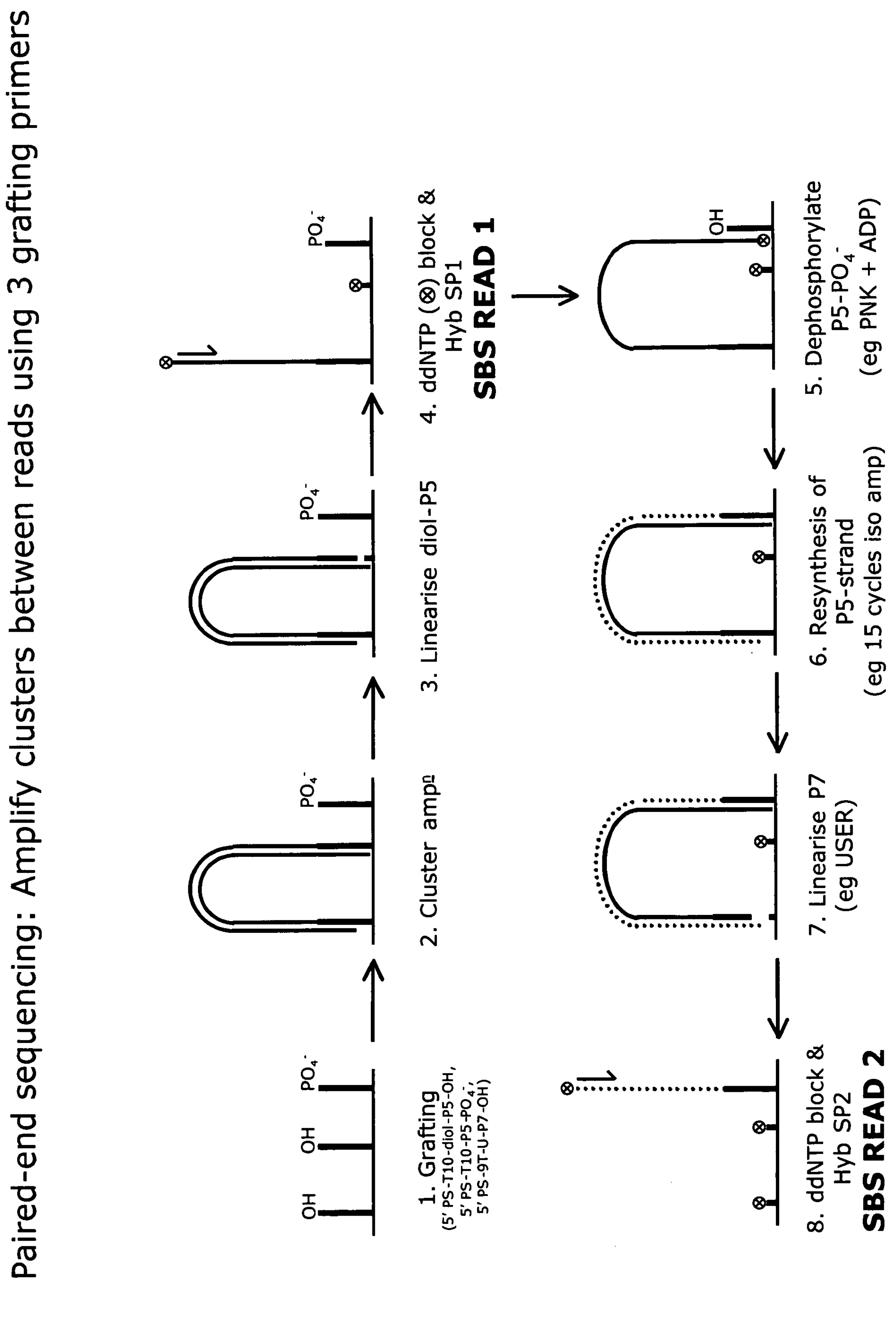
Paired-end sequencing: Amplify clusters between reads using 3 grafting primers
OH
OH
PO4-
1. Grafting
(5' PS-T10-diol-P5-OH,
5' PS-T10-P5-PO4-,
5' PS-9T-U-P7-OH)
PO4-
2. Cluster ampn
PO4-
3. Linearise diol-P5
PO4-
4. ddNTP (⊗) block &
Hyb SP1
SBS READ 1
OH
5. Dephosphorylate
P5-PO4-
(eg PNK + ADP)
6. Resynthesis of
P5-strand
(eg 15 cycles iso amp)
7. Linearise P7
(eg USER)
8. ddNTP block &
Hyb SP2
SBS READ 2

Figure 6

## Sequencing data

## 3-4kb BAC, 200-300bp inserts

Fig 7

| Region 1 | Region 2 | Distance between regions (bp) |
|---|---|---|
| 46070-46087 (18bp) | 49068-49312 (245bp) | 3242 |
| 53044-53138 (95bp) | 55954-56141 (188bp) | 3097 |
| 59057-59341 (285bp) | 90527-90534 (8bp) | 31477 * |
| 76645-76813 (169bp) | 79911-79994 (84bp) | 3349 |
| 78827-79043 (217bp) | 81981-82034 (54bp) | 3207 |
| 107729-107770 (42bp) | 110942-111056 (115bp) | 3327 |
| 116599-116685 (87bp) | 119798-119958 (161bp) | 3359 |
| 120651-120673 (23bp) | 123823-124011 (189bp) | 3360 |
| 121758-121797 (40bp) | 125263-125448 (186bp) | 3690 |
| 128462-128514 (53bp) | 131624-131763 (140bp) | 3301 |
| 153281-153392 (112bp) | 156901-157016 (116bp) | 3735 |

**Single regions**

71902-72178 (277bp) /

Human chromosome 2 (228bp)

10/13 correct

1/13 incorrect spacing between 2 regions chimera?

2/13 single region inefficient plasmid safe/binding of non-biotin to beads

# SBS - Gap Sizes of Paired Reads

**CT840**

**2-2.5kb fragments, 400-600bp library**

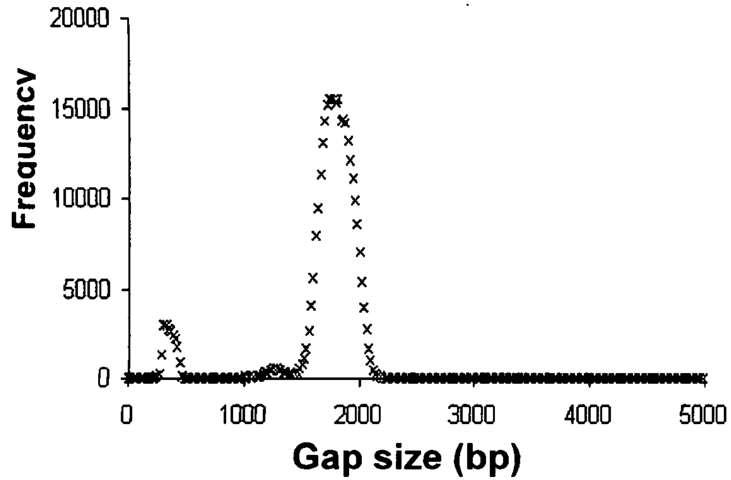


**CT841**

**3-4kb fragments, 300-400bp library**

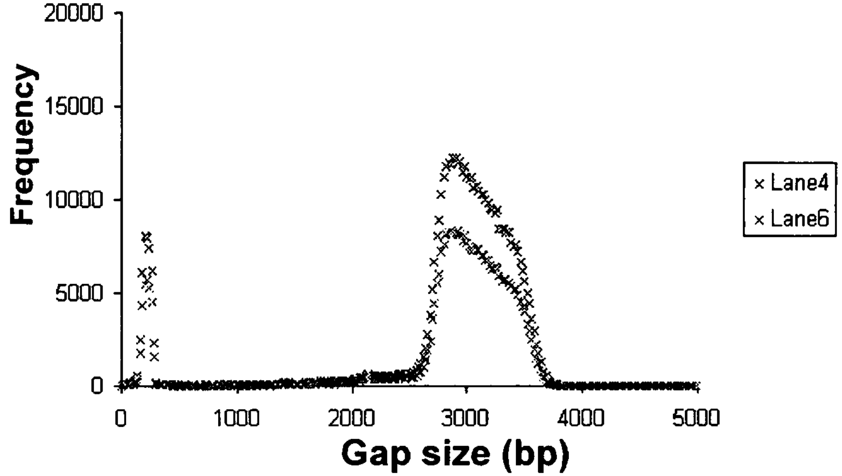


**BAC Control**

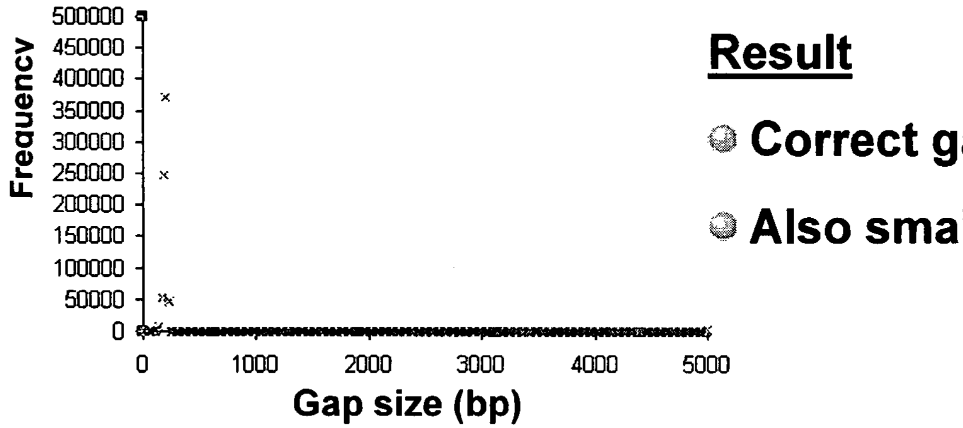


**Result**

- **Correct gap sizes for CT840 and 841**
- **Also smaller gap size, similar to BAC control**

Fig 8

# PE sample prep method using biotinylated random primers

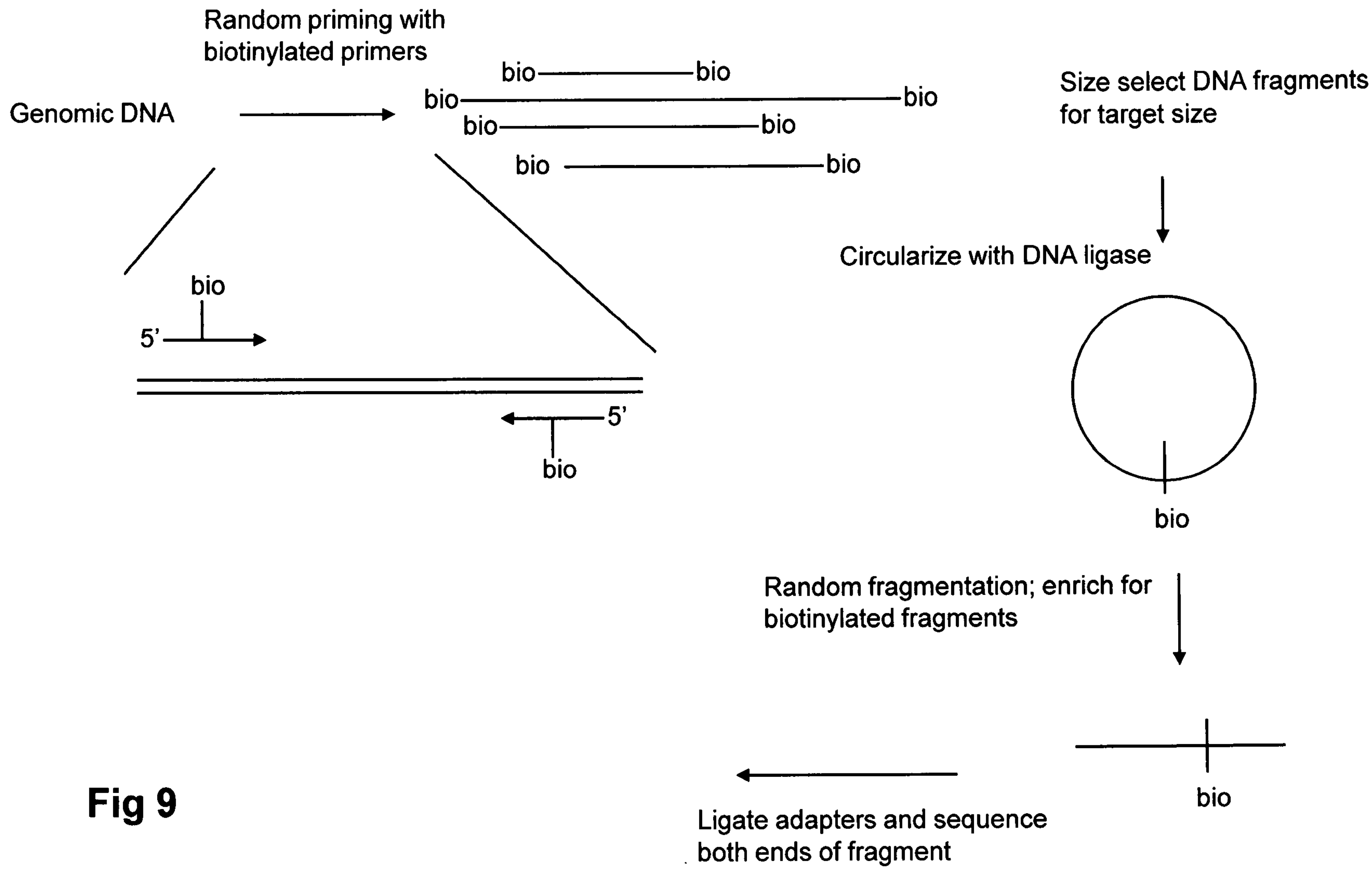


Fig 9

# Pre End Repair

Gap size distributions for two large insert libraries, one created using a pre-end repair step and then a 5% biotin nucleotide reaction, the second create with 5% biotin end repair without the pre-end repair step.

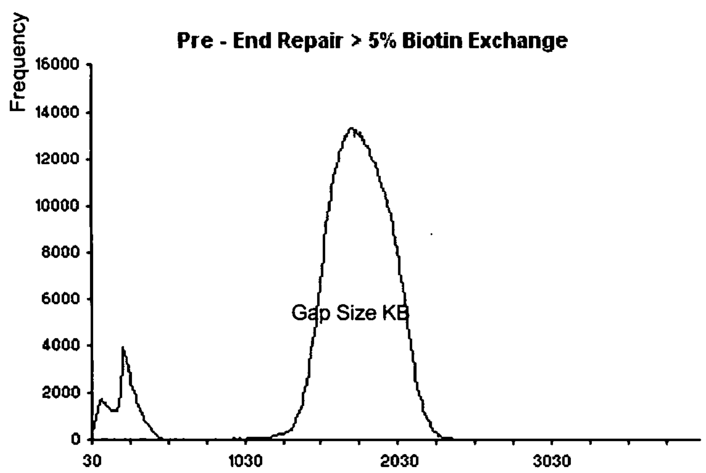


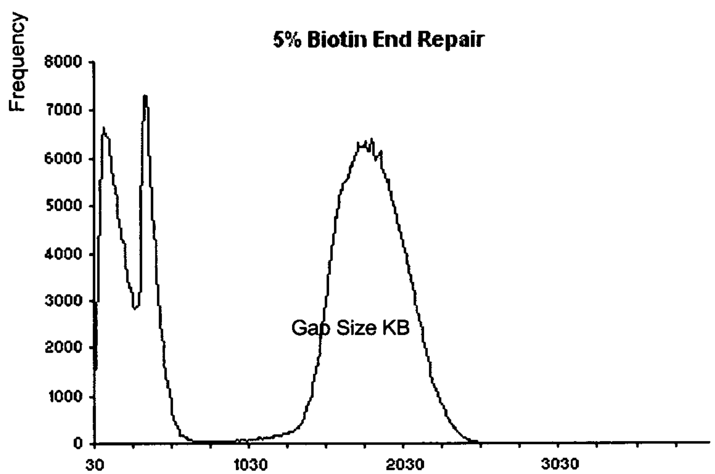


| | |
|---|---|
| 89.18 % | Outie Fragments |
| 8.32 % | Innie Fragments |
| 0.21 % | 100K Duplicates |

| | |
|---|---|
| 60.25 % | Outie Fragments |
| 35.12 % | Innie Fragments |
| 0.1 % | 100K Duplicates |

Fig 10

# Titration of biotin concentration in large insert libraries

The data below shows the effect of varying Biotin concentration used in the exchange reaction on the final library diversity.
The percentage of duplicated sequences found in 100,000 paired sequences (100K duplicates) is used as a measure of the library's diversity. The lower the duplicates the more diverse the library population.

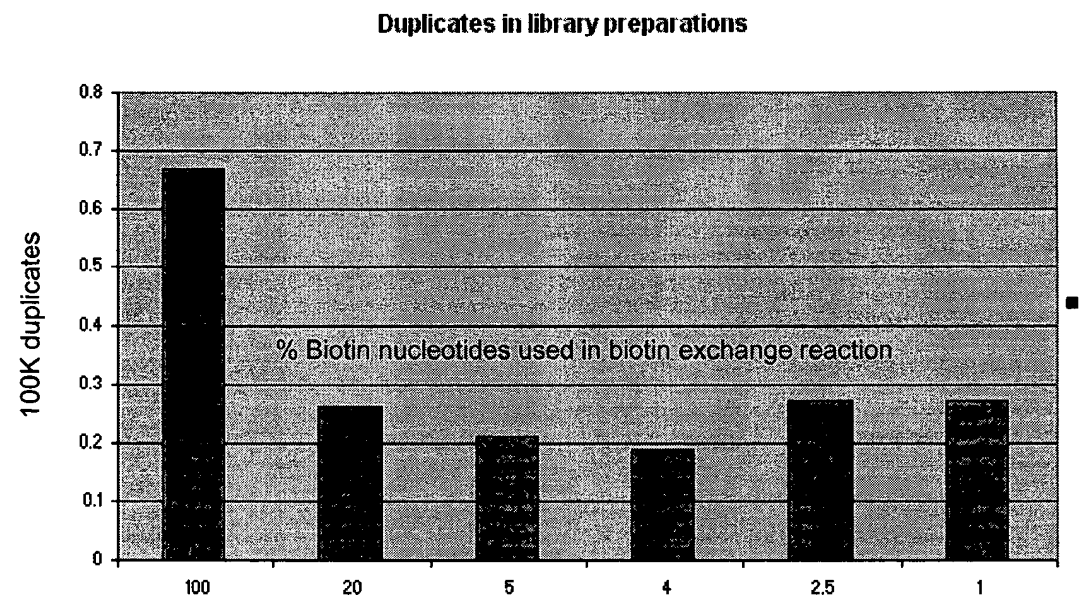


Fig 11

# Biotin Inhibition of Self-Ligation

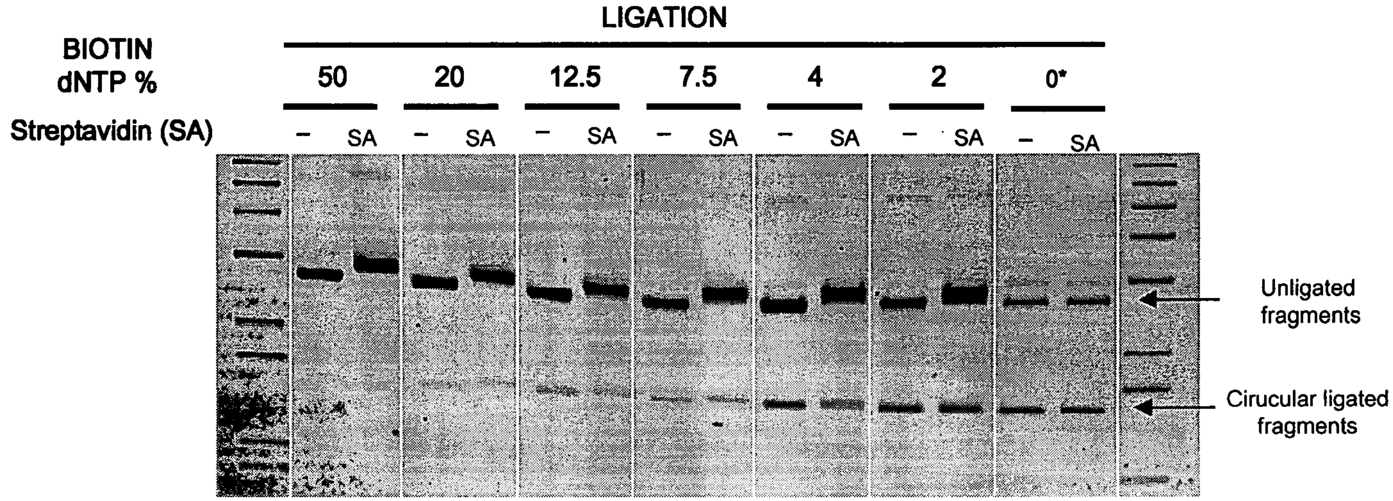


| % of Fragment | | | | | | | | | | | | | | |
|---|---|---|---|---|---|---|---|---|---|---|---|---|---|---|
| LINEAR | 98 | 95 | 96 | 93 | 93 | 90 | 89 | 87 | 83 | 82 | 70 | 73 | 40 | 41 |
| CIRCULAR | 1 | 1 | 3 | 3 | 5 | 5 | 9 | 9 | 13 | 15 | 26 | 22 | 54 | 54 |

Fig 12

## HydroShear 2.3kb - T3 Ligase vs. T4 Ligase.

BC21-9. Hydroshear 2.3 kb T3 Ligase 6.7ul.

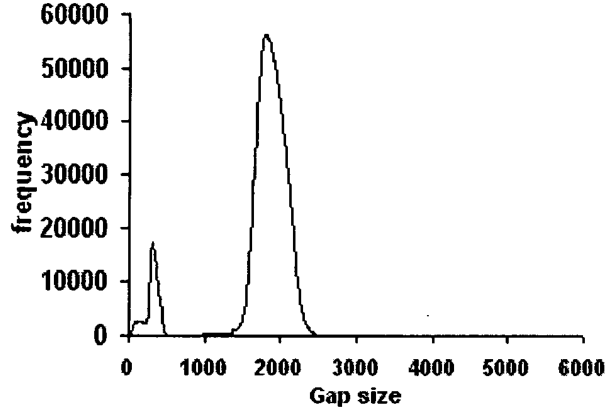


| Library | CT1790 |
|---|---|
| Median | 0 |
| %Var | - |
| %Innies | 8.91 |
| %Outies | 89.10 |
| % 100K dups | 0.23 |
| Diversity | 39,734,028 |
| %Chimeras | 2.1 |

BC21-10. Hydroshear 2.3 kb T4 Ligase. 6.7ul

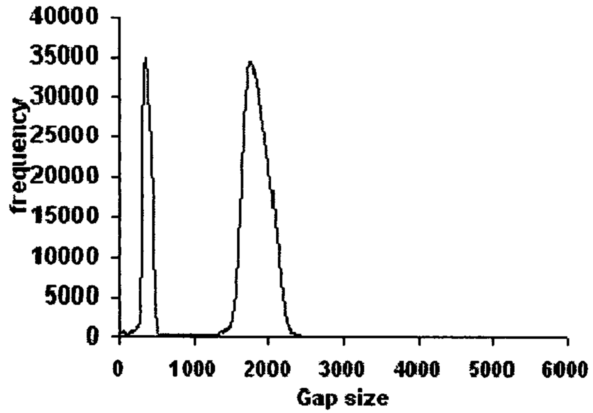


| Library | CT1791 |
|---|---|
| Median | 0 |
| %Var | - |
| %Innies | 21.44 |
| %Outies | 61.91 |
| % 100K dups | 3.07 |
| Diversity | 2,558,274 |
| %Chimeras | 17.4 |

Fig 13

Protocol 2b

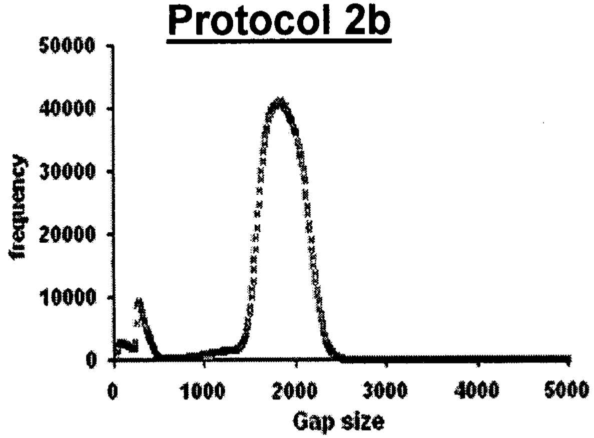


Protocol 2c

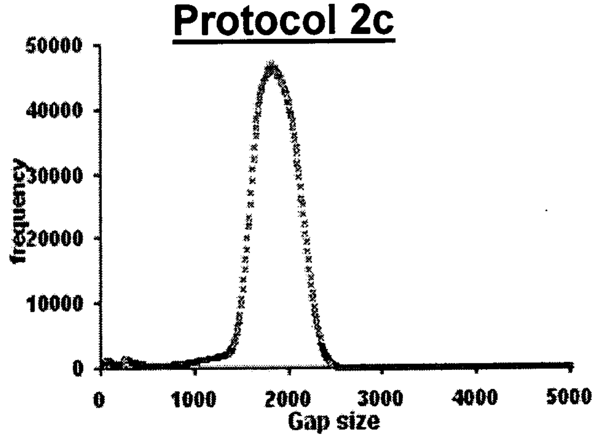


| | Protocol 2b | | Protocol 2c | |
|---|---|---|---|---|
| Median gap size | 1860bp | | 1864bp | |
| Gap size variance | 12.1% | | 12.3% | |
| Chimeras | 2.4% | | 2.5% | |
| Inward fragments | | 6.68% | | 1.75% |
| Outward fragments | | 92.10% | | 97.02% |
| Duplicates removed (100K) | 0.10% | | 0.04% | |
| Diversity | 113,827,127 fragments | | 231,195,441 fragments | |

- Protocol 2c
  - decreased % inward
  - increased % outward
  - Increased diversity

Fig 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 96877007 P
- US 60990104 B
- US 61046203 B
- US 2006292611 A1
- WO 07057652 A
- US 20070141604 A
- WO 07052006 A
- US 20070128624 A
- WO 05068656 A
- WO 0006770 A
- WO 0406984 A
- WO 9844151 A
- WO 00018957 A
- WO 07010252 A
- WO 07010251 A
- WO 04018497 A
- US 7057026 B
- GB 2007001770 W
- US 200707991 W
- US 6306597 B
- WO 0018957 A
- US 11973321 B
- GB 2007000447 W
- US 60850210 B
- US 60898910 B
- WO 06064199 A
- US 2007014649 W
- WO 2004018493 A
- US SN60788248 P
- US 0707991 W


### Non-patent literature cited in the description


- **SAMBROOK ; RUSSELL.** Molecular Cloning, A Laboratory Manual
- **SAMBROOK et al.** Molecular Cloning, A Laboratory Manual. Cold Spring Harbor Laboratory Press, 2001
- Current Protocols
- **ZHAO et al.** *Nucleic Acids Research,* 2001, vol. 29 (4), 955-959
- **PIRRUNG et al.** *Langmuir,* 2000, vol. 16, 2185-2191